# EUROPEAN PATENT APPLICATION

(11) **EP 1 471 064 A1**
(43) Date of publication of application: **27.10.2004**
(21) Application number: 03701037.8
(22) Date of filing: 09.01.2003
(51) Int. Cl.: C07D 311/18, C07D 405/10, C07D 405/12, C07D 413/10, C07D 417/10, A61K 31/352, A61K 31/41, A61K 31/4245, A61K 31/433, A61K 31/4709, A61K 31/4725, A61K 31/496, A61P 3/06, A61P 9/00, A61P 9/10, A61P 25/28, A61P 43/00

(54) **COUMARIN DERIVATIVES, PROCESS FOR THEIR PRODUCTION AND USE THEREOF**

(30) Priority: 11.01.2002 JP 2002004359
(71) Applicant: Takeda Chemical Industries, Ltd., Osaka-shi, Osaka 541-0045 (JP)
(72) Inventor: TERASHITA, Zen-ichi, Nishinomiya-shi,Hyogo (JP); NAKAMURA, Masahira, Kashiba-shi, Nara 639-0242 (JP); MARUI, Shogo, Kobe-shi, Hyogo 651-1233 (JP); OGINO, Masaki, Nishinomiya-shi, Hyogo 662-0838 (JP)
(74) Representative: Rickard, Timothy Mark Adrian
(86) International application number: PCT/JP2003/000112
(87) International publication number: WO 2003/059900

(57) **Abstract**

Compounds represented by the general formula [I]: 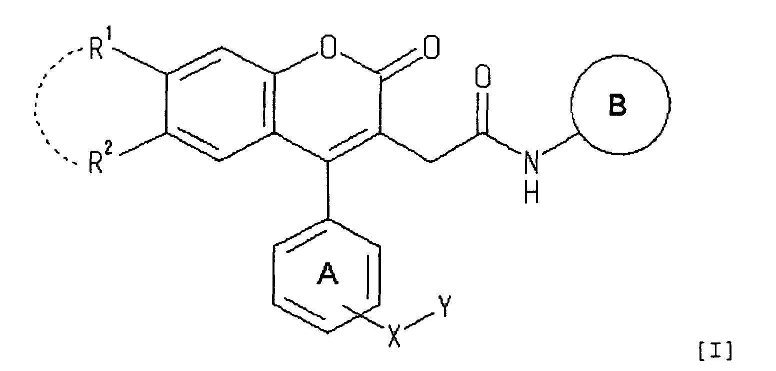 wherein R¹ and R² are each hydrogen, halogen, an optionally substituted linear hydrocarbon group, or hydroxyl which may be substituted with an optionally substituted liner hydrocarbon group, or R¹ and R² together with the carbon atoms adjacent thereto may form an optionally substituted cyclic hydrocarbon or a dihydrofuran ring which may have an oxo group; ring A is a benzene ring which may be further substituted; ring B is an aromatic ring which may be substituted; X is a bond or a spacer whose main chain has 1 to 6 atoms; Y is carboxyl which may be esterified, carbamoyl which may be substituted, cyano, or an optionally substituted heterocyclic group bearing a hydrogen atom capable of being deprotonated, or salts thereof, which are useful as lipid-rich plaque regressing agents and/or ACAT inhibitors.

## Description

### Technical Field

The present invention relates to coumarin derivatives having lipid-rich plaque regressing activity and/or ACAT inhibitory activity which are useful for preventing or treating acute coronary syndrome such as acute myocardial infarction and unstable angina, peripheral artery occlusion, hyperlipemia, cerebral infarction, cerebral apoplexy, arteriosclerosis, Alzheimer's disease, or the like, or preventing or treating restenosis after PTCA or after stent placement.

### Background Art

As an agent for reducing the level of blood cholesterol which causes arteriosclerosis, an agent which inhibits absorption of bile acid by capturing it such as cholestyramine and cholestipol (US Patent 4027009), an agent which inhibits absorption of cholesterol via an intestinal tract by inhibiting an acyl coenzyme A cholesterol acyl transferase (ACAT) such as melinamide and a cholesterol synthesis inhibitor, especially an agent which inhibits 3-hydroxy-3-methylgultaryl coenzyme A (HMG-CoA) reductase such as lovastatin (US Patent 4231938), simvastatin (US Patent 4444784) and pravastatin (US Patent 4346227) are employed in pharmaceuticals.

However, an HMG-CoA reductase inhibitor may cause a problem associated with side effects due to its inhibitory effect not only on cholesterol biosynthesis but also on synthesis of a biologically essential component such as ubiquinone, dolichol and heme A.

Acute coronary syndrome (for example, unstable angina, acute myocardial infarction and ischemic sudden death) is caused by destruction of a coronary artery plaque (atheroma) followed by formation of a thrombus and the resultant plugging of the lumen of a coronary artery. Peripheral artery occlusion is caused by destruction of an artery plaque (atheroma) followed by formation of a thrombus and the resultant plugging of the lumen of a peripheral artery. These diseases are related closely to the characteristics of a plaque, and a lipid-rich plaque formed by deposition of a macrophage retaining lipids such as cholesterol extensively onto the inner wall of a blood vessel is believed to cause acute coronary syndrome and peripheral artery occlusion. A lipid-rich plaque formed at carotid artery or intracerebral vessel is believed to cause cerebral apoplexy or cerebral infarction.

Accordingly, regression and removal of a lipid-rich plaque are very important for preventing or treating acute coronary syndrome such as acute myocardial infarction and unstable angina as well as peripheral artery occlusion, cerebral apoplexy, or cerebral infarction. Also since a lipid-rich plaque is observed in a human whose blood cholesterol level is not high and a lipid-rich plaque once formed is difficult to be removed, an agent capable of regressing such a lipid-rich plaque efficiently has been desired. Since a lipid-rich plaque is observed in a human whose blood cholesterol level is not high, inhibiting ACAT to reduce intestinal absorption of cholesterol is not considered to be sufficient for regressing and removing a lipid-rich plaque.

In view of the aforementioned circumstances, the present inventors studied intensively and, as a result, found coumarin derivatives having lipid-rich plaque regressing activity or ACAT inhibitory activity (International Publication WO02/06264).

In addition, recently, possibility of prevention or treatment of Alzheimer's disease with ACAT inhibition has been suggested (L.Puglielli et al., Nature Cell Biology, 2001, vol.3, p.905-912).

### Object of the Invention

The present invention provides coumarin derivatives having lipid-rich plaque regressing activity or ACAT inhibitory activity useful in preventing or treating acute coronary syndrome such as acute myocardial infarction and unstable angina as well as peripheral artery occlusion, cerebral apoplexy, or cerebral infarction. The present invention also provides coumarin derivatives which are migrated readily into a blood vessel or tissue to act directly on a macrophage in which lipids such as cholesterol are retained extensively, whereby exerting a direct regressing effect on an arteriosclerotic lesion.

In addition, since coumarin derivatives provided in the present invention have ACAT inhibitory activity, it is thought that they have activity of suppressing secretion of very low-density lipoprotein from liver, activity of suppressing absorption of cholesterol via small intestin and suppressing secretion of chylomicron accompanied therewith and, consequently, activity of reducing blood cholesterol and triglyceride.

Further, it is considered that coumarin derivatives provided in the present invention may be utilized for preventing or treating Alzheimer's disease, multiple risk syndrome and metabolic syndrome.

### Summary of Invention

In order to find clinically more useful compounds, the present inventors continued research intensively and, as a result, found that novel coumarin derivatives having a certain substituent on the phenyl group at the 3-position of the coumarin skeleton unexpectedly have excellent ACAT inhibitory activity and lipid-rich plaque regressing activity and, moreover, have sufficient lipid-rich plaque regressing activity even at such a lower concentration as does not influence on a blood cholesterol level, which resulted in completion of the present invention.

That is, the present invention relates to:
(1) a compound represented by the formula [I]: 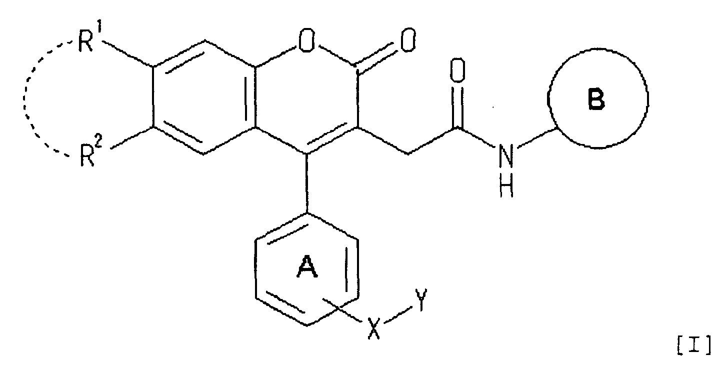 wherein R¹ and R² are each a hydrogen atom, a halogen atom, an optionally substituted linear hydrocarbon group, or a hydroxyl group which may be substituted with an optionally substituted linear hydrocarbon group, or R¹ and R² may be taken together with the adjacent carbon atoms to form an optionally substituted cyclic hydrocarbon or a dihydrofuran ring which may be substituted with an oxo group; ring A is an optionally further substituted benzene ring; B is an optionally substituted aromatic ring; X is a bond or a spacer whose main chain consists of 1 to 6 atoms; Y is an optionally esterified carboxyl group, an optionally substituted carbamoyl group, a cyano group, or an optionally substituted heterocyclic group bearing a hydrogen atom capable of being deprotonated; provided that 3-[3-[7-chloro-3-(2-[[4-chloro-2-(trifluoromethyl)phenyl]amino]-2-oxoethyl)-6-methyl-2-oxo-2H-chromen-4-yl]phenyl]-2-propionic acid, ethyl 3-[3-[7-chloro-3-(2-[[4-chloro-2-(trifluoromethyl)phenyl]amino]-2-oxoethyl)-6-methyl-2-oxo-2H-chromen-4-yl]phenyl]-2-propionate, methyl (2E)-3-[3-[7-chloro-3-(2-[[4-chloro-2-(trifluoromethyl)phenyl]amino]-2-oxoethyl)-6-methyl-2-oxo-2H-chromen-4-yl]phenyl]-2-propenoate, (2E)-3-[3-[7-chloro-3-(2-[[4-chloro-2-(trifluoromethyl)phenyl]amino]-2-oxoethyl)-6-methyl-2-oxo-2H-chromen-4-yl]phenyl]-2-propenoic acid, ethtyl (2E)-3-[3-[7-chloro-3-(2-[[4-chloro-2-(trifluoromethyl)phenyl]amino]-2-oxoethyl)-6-methyl-2-oxo-2H-chromen-4-yl]phenyl]-2-propenoate, ethyl (2E)-3-[3-[7-chloro-3-(2-[[4-fluoro-2-(trifluoromethyl)phenyl]amino]-2-oxoethyl)-6-methyl-2-oxo-2H-chromen-4-yl]phenyl]-2-propenoate, and (2E)-3-[3-[7-chloro-3-(2-[[4-fluoro-2-(trifluromethyl)phenyl]amino]-2-oxoethyl)-6-methyl-2-oxo-2H-chromen-4-yl]phenyl]-2-propenoic acid are excluded, or a salt thereof;
(2) the compound according to the above (1), whererin the formula [I] is the formula [I']: 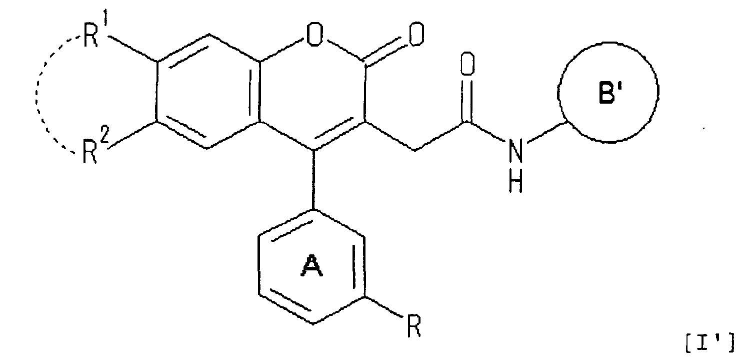 wherein ring B' is an optionally substituted benzene ring or an optionally substituted pyridine ring, R is an optionally esterified carboxyl group, or a linear hydrocarbon group which is substituted with an optionally esterified carboxyl group, and other symbols are as defined in the above (1);
(3) the compound according to the above (1), wherein R¹ and R² are each a hydrogen atom, a halogen atom or an optionally substituted linear hydrocarbon group, or R¹ and R² may be taken together with the adjacent carbon atoms to form an optionally substituted cyclic hydrocarbon;
(4) the compound according to the above (1), wherein R¹ and R² are each a halogen atom or an optionally substituted C₁₋₇ alkyl group;
(5) the compound according to the above (1), wherein R¹ is a halogen atom and R² is a linear hydrocarbon group which is substituted with an optionally substituted amino group;
(6) the compound according to the above (1), wherein R¹ is a halogen atom and R² is a linear hydrocarbon group which is substituted with an optionally substituted cyclic amino group;
(7) the compound according to the above (1), wherein the cyclic hydrocarbon is C₅₋₇ cyclic hydrocarbon;
(8) the compound according to the above (1), wherein ring B is a benzene ring which is substituted with a halogenated alkyl group and/or a halogen atom;
(9) the compound according to the above (1), whrerin R is a group represented by the formula -(CH₂)ₙ-R' wherein R' is an optionally esterified carboxyl group and n is an integer of 0 to 6;
(10) the compound according to the above (2), wherein R is a group represented by the formula -CH=CH-(CH₂)_{n'}-R' wherein R' is an optionally esterified carboxyl group and n' is an integer of 0 to 4;
(11) the compound according to the above (2), wherein R is a group represented by the formula -(CH=CH)_{n"}-R' wherein R' is an optionally esterified carboxyl group and n" is an integer of 1 to 3;
(12) 3-[3-[7-chloro-3-(2-[[4-fluoro-2-(trifluoromethyl)phenyl]amino]-2-oxoethyl)-6-methyl-2-oxo-2H-chromen-4-yl]phenyl]propionic acid, (2E)-3-[3-[7-chloro-6-methyl-2-oxo-3-(2-oxo-2-[[2-(trifluoromethyl)phenyl]amino]ethyl)-2H-chromen-4-yl]phenyl]-2-propenoic acid, 3-[3-[7-chloro-6-methyl-2-oxo-3-(2-oxo-2-[[2-(trifluoromethyl)phenyl]amino]ethyl)-2H-chromen-4-yl]phenyl]propionic acid, (2E)-3-[3-[6-chloro-3-(2-[[4-fluoro-2-(trifluoromethyl)phenyl]amino]-2-oxoethyl)-7-methyl-2-oxo-2H-chromen-4-yl]phenyl]-2-propenoic acid, 3-[3-[6-chloro-3-(2-[[4-fluoro-2-(trifluoromethyl)phenyl]amino]-2-oxoethyl)-7-methyl-2-oxo-2H-chromen-4-yl]phenyl]propionic acid, (2E)-3-(3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}phenyl)acrylic acid, (2E)-3-(3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}phenyl)acrylic acid, 3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}benzoic acid, 3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}benzoic acid or a salt thereof;
(13) a prodrug of the compound according to the above (1) or a salt thereof;
(14) a pharmaceutical composition comprising the compound according to the above (1) or (13) or a salt thereof;
(15) the pharmaceutical composition according to the above (14), which is a lipid-rich regressing agent or an ACAT inhibitor;
(16) the pharmaceutical composition according to the above (14), which is a prophylactic or therapeutic agent against acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or an agent for regressing, inhibiting progression of or stabilizing an arteriosclerotic lesion;
(17) the agent for regressing, inhibiting progression of or stabilizing an arteriosclerotic lesion according to the above (16), which is combined with a HMG-CoA reductase inhibitor;
(18) a method for regressing a lipid-rich plaque or inhibiting ACAT in a mammal, which comprises administering an effective amount of the compound according to the above (1) or a salt thereof to the mammal;
(19) a method for preventing or treating acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or regressing, inhibiting progression of or stabilizing an arteriosclerotic lesion in a mammal, which comprises administering an effective amount of the compound according to the above (1) or a salt thereof to the mammal;
(20) the method for regressing, inhibiting progression of or stabilizing an arteriosclerotic lesion according to the above (19), which comprises administering the compound according to the above (1) or a salt thereof in combination with a HMG-CoA reductase inhibitor;
(21) use of the compound according to the above (1) or a salt thereof for production of a lipid-rich plaque regressing agent or an ACAT inhibitor;
(22) use of the compound according to the above (1) or a salt thereof for production of a prophylactic or therapeutic agent against acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or an agent for regressing, inhibiting progression of or stabilizing an arteriosclerotic lesion; and
(23) the use of the compound according to the above (1) or a salt thereof for production of an agent for regressing, inhibiting progression of or stabilizing an arteriosclerotic lesion according to the above (22), which is combined with a HMG-CoA reductase inhibitor.

In the formula [I], R¹ and R² are each a hydrogen atom, a halogen atom, an optionally substituted linear hydrocarbon group or a hydroxyl group which may be substituted with an optionally substituted linear hydrocarbon group, or R¹ and R² may be taken together with the adjacent carbon atoms to form an optionally substituted cyclic hydrocarbon or a dihydrofuran ring which may be substituted with an oxo group.

As the "linear hydrocarbon group" of the "optionally substituted linear hydrocarbon group" and the "hydroxyl which may be substituted with an optionally substituted linear hydrocarbon group" represented by R¹ and R², for example, an alkyl group, an alkenyl group, an alkynyl group and the like are used. Alternatively, a group in which two or three of carbon-carbon bonds of an alkyl group are converted into double bonds, such as an alkadienyl group or an alkatrienyl group may be used.

As the alkyl group, for example, a linear or branched alkyl group having 1 to 7 carbon atoms is used and, preferably, for example, a linear or branched alkyl group having 1 to 4 carbon atoms such as methyl, ethyl, propyl, isopropyl, butyl, isobutyl, sec-butyl or tert-butyl is used.

As the alkenyl group, for example, an alkenyl group having 2 to 6 carbon atoms such as ethenyl, propenyl, isopropenyl, butenyl, isobutenyl or sec-butenyl is used and, preferably, for example, an alkenyl group having 2 to 4 carbon atoms such as ethenyl, propenyl, isopropenyl or isobutenyl is used.

As the alkynyl group, an alkynyl group having 2 to 6 carbon atoms such as ethynyl, propynyl, isopropynyl, butynyl, isobutynyl or sec-butynyl is used and, preferably, an alkynyl group having 2 to 4 carbon atoms such as ethynyl, propynyl, isopropynyl or isobutynyl is used.

Examples of the group in which two or three of carbon-carbon bonds of an alkyl group are converted into double bonds include a group in which two or three of carbon-carbon bonds of a linear or branched C₂₋₇ alkyl group (preferably, linear alkyl group) are converted into double bonds and, preferably, an alkadienyl group having 4 to 6 carbon atoms such as butadienyl, and an alkatrienyl group such as 1,3,5-hexatrienyl are used.

As the linear hydrocarbon group, a linear or branched alkyl group having 1 to 6 carbon atoms is preferable, and a linear or branched C₁₋₄ alkyl group such as methyl, ethyl, propyl, isopropyl, butyl, isobutyl, sec-butyl or tert-butyl is particularly preferable.

Examples of a substituent for the "optionally substituted linear hydrocarbon group" and the "hydroxy group which may be substituted with an optionally substituted linear hydrocarbon group" represented by R¹ and R² include an optionally substituted aryl group, an optionally substituted cycloalkyl group, an optionally substituted cycloalkenyl group, an optionally substituted heterocyclic group, an optionally substituted amino group, an optionally substituted hydroxyl group, an optionally substituted thiol group, an acyl group, a halogen atom (e.g. fluorine, chlorine, bromine, iodine), an oxo group, a carboxyl group, a nitro group, a cyano group, an optionally substituted alkyl group and the like. The "linear hydrocarbon group" may be substituted with 1 to 5 (preferably, 1 to 3) of these optional substituents at substitutable positions.

Examples of the "aryl group" of the "optionally substituted aryl group" include a C₆₋₁₆ aryl group such as phenyl, naphthyl, anthryl, phenanthryl, acenaphthylenyl and the like. Inter alia, a C₆₋₁₀ aryl group such as phenyl, 1-naphthyl, 2-naphthyl and the like is preferable. Examples of a substituent for the aryl group include (i) an optionally halogenated C₁₋₆ alkoxy group (e.g. methoxy, ethoxy, propoxy, trifluoromethoxy etc.), (ii) a halogen atom (e.g. fluorine, chlorine, bromine, iodine), (iii) an optionally halogenated C₁₋₆ alkyl group (e.g. methyl, ethyl, propyl, trifluoromethyl etc.) and the like. The aryl group may be substituted with 1 to 2 of these optional substituents.

Examples of the "cycloalkyl group" of the "optionally substituted cycloalkyl group" include a C₃₋₇ cycloalkyl group such as cyclopropyl, cyclobutyl, cyclopentyl, cyclohexyl, cycloheptyl and the like. A substituent for the cycloalkyl group and the number of the substituent are similar to those for the aforementioned optionally substituted aryl group.

Examples of the "cycloalkenyl gruop" of the "optionally substituted cycloalkenyl group" include a C₃₋₆ cycloalkenyl group such as cyclopropenyl, cyclobutenyl, cyclopentenyl, cyclohexenyl and the like. A substituent for the cycloalkenyl group and the number of the substituent are similar to those for the aforementioned optionally substituted aryl group.

Examples of the "heterocyclic group" of the "optionally substituted heterocyclic group" include an aromatic heterocyclic group and a saturated or unsaturated non-aromatic heterocyclic group (aliphatic heterocyclic group) which contain at least one, preferably 1 to 4 heteroatoms selected from oxygen, sulfur and nitrogen as an atom constituting a ring system (ring atom). Non-aromatic heterocyclic group is preferable.

Examples of the "aromatic heterocyclic group" include a 5 to 6-membered aromatic monocyclic heterocyclic group (e.g. furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, frazanyl, 1,2,3-thiadiazolyl, 1,2,4-thiadiazolyl, 1,3,4-thiadiazolyl; 1,2,3-triazolyl, 1,2,4-triazolyl, tetrazolyl, pyridyl, pyridazinyl, pyrimidinyl, pyrazinyl, triazinyl etc.) and an aromatic fused heterocyclic group in which 2 to 3 of 5- to 6-membered rings (the aforementioned 5 to 6-membered aromatic monocyclic heterocyclic ring, benzene ring etc.) are fused (e.g.: benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzoisothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thiantrenyl, phenathrizinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.). Inter alia, a 5- to 6-membered aromatic monocyclic heterocyclic group such as furyl, thienyl, pyrazinyl, pyridyl and pyrimidinyl is preferable.

Examples of the "non-aromatic heterocyclic group" include a 4- to 9-membered non-aromatic monocyclic heterocyclic group such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl and the like (in particular, 5- to 9-membered cyclic amino group which may contain 1 to 3 heteroatoms such as an oxygen atom or a sulfur atom in addition to a nitrogen atom, such as pyrrolidinyl, piperidinyl, piperazinyl, morpholinyl and 3,6-dihydropyridine-1(2H)-yl), a fused heterocyclic group of 1 to 2 (preferably 1) of the aforementioned non-aromatic monocyclic heterocyclic groups and 1 to 2 (preferably 1) of benzene rings, such as 2,3-dihydroindolyl, 1,3-dihydroisoindolyl and the like, a fused heterocyclic group of 1 to 2 (preferably 1) of the aforementioned non-aromatic monocyclic heterocyclic groups and 1 to 2 (preferably 1) of the aforementioned 5- to 6-membered aromatic monocyclic heterocyclic group, and a non-aromatic heterocyclic group in which a part or all of the double bonds of the aforementioned aromatic monocyclic heterocyclic group or aromatic fused heterocyclic group are saturated, such as 1,2,3,4-tetrahydroquinolyl, 1,2,3,4-tetrahydroisoquinolyl and the like.

The heterocyclic group may be substituted with 1 to 4, preferably 1 to 2 substituents. Examples of such a substituent include an optionally halogenated C₁₋₆ alkyl group (e.g. methyl, ethyl, propyl, n-butyl, n-hexyl etc.), an optionally halogenated C₆₋₁₂ aryl group (e.g. phenyl), a hydroxy-C₆₋₁₂ aryl group (e.g. 4-hydroxyphenyl), an optionally halogenated C₁₋₄ alkylsulfonyl group (e.g. methylsulfonyl), a C₇₋₁₅ aralkyl group (e.g. benzyl), an optionally halogenated C₁₋₄ alkoxy-C₁₋₄ alkyl group (e.g. propoxyethyl etc.), a 5- to 9-membered heterocyclic group which contains 1 to 3 heteroatoms such as a nitrogen atom, an oxygen atom and a sulfur atom in addition to carbon atoms (e.g. piperidyl, piperazinyl, morpholinyl, thienyl, furyl, pyridinyl, pyrimidinyl, thiazolyl, benzothiazolyl, benzoisothiazolyl, benzoxazolyl, benzisoxazolyl etc.), a hydroxy group, an oxo group, a thioxo group and the like.

Examples of a substituent for the "optionally substituted amino group" (including an amino group and a mono- or di-substituted amino group) include an optionally halogenated lower (C₁₋₆) alkyl group (e.g. methyl, ethyl, propyl etc.), an optionally halogenated C₆₋₁₂ aryl group (e.g. phenyl), a 5- to 9-membered heterocyclic group which contains 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazolyl, benzisothiazolyl, benzoxazolyl, benzisoxazolyl etc.), an optionally halogenated C₁₋₄ alkylcarbonyl group (e.g. methylcarbonyl, ethylcarbonyl etc.), a C₆₋₁₂ aryl-carbonyl group (e.g. benzoyl etc.), an optionally halogenated C₁₋₄ alkyl-sulfonyl group, and an optionally halogenated C₁₋₄ alkoxy-C₁₋₄ alkyl group. In addition, the two substituents of a di-substituted amino group may be taken together with the nitrogen atom to form a "cyclic amino group". The "cyclic amino group" includes a 3- to 8-membered (preferably 5- to 6-membered) cyclic amino group such as 1-azetidinyl, 1-pyrrolidinyl, piperidinyl, morpholinyl, thiomorpholinyl (the sulfur atom may be oxidized), and 1-piperazinyl which may be substituted at the 4-position with optionally halogenated lower alkyl (e.g. C₁₋₆ alkyl such as methyl, ethyl, propyl, isopropyl, butyl, tert-butyl, pentyl, hexyl etc.), optionally halogenated aralkyl (e.g. C₇₋₁₀ aralkyl such as benzyl, phenethyl etc.), optionally halogenated aryl (e.g. C₆₋₁₀ aryl such as phenyl, 1-naphthyl, 2-naphthyl etc.) or the like.

Examples of the "optionally substituted alkyl group" include a C₁₋₆ alkyl group (e.g. methyl, ethyl, propyl, n-butyl, n-hexyl etc.) which may be substituted with a halogen atom (e.g. fluorine, chlorine, bromine, iodine) or the like.

Examples of the "optionally substituted hydroxyl group" include a hydroxyl group, an optionally halogenated C₁₋₁₆ alkoxy group, preferably an optionally halogenated C₁₋₄ alkoxy group, more preferably a C₁₋₄ alkoxy group (e.g. methoxy, ethoxy, propoxy, butoxy, t-butoxy etc.), a C₁₋₆ alkyl-carbonyloxy group (e.g. methylcarbonyloxy, ethylcarbonyloxy, butylcarbonyloxy etc.), an aminocarbonyloxy group, and a mono- or di-C₁₋₄ alkylaminocarbonyloxy group.

Examples of the "optionally substituted thiol group" include a thiol group, an optionally halogenated C₁₋₁₆ alkylthio group, preferably optionally halogenated C₁₋₄ alkylthio group, more preferably C₁₋₄ alkylthio group (e.g. methylthio, ethylthio etc.) and a 5- to 9-membered heterocycle, containing 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazolyl, benzisothiazolyl, benzoxazolyl, benzisoxazolyl etc.),-thio group (e.g. 2-pyridylthio).

Examples of the "acyl group" include a formyl group, a C₁₋₆ alkyl-carbonyl group (preferably C₁₋₄ alkyl-carbonyl group (e.g. methylcarbonyl, ethylcarbonyl)), a C₁₋₄ alkoxycarbonyl group (e.g. methoxycarbonyl), an optionally halogenated C₁₋₆ alkyl-sulfonyl group (preferably C₁₋₄ alkylsulfonyl group (e.g. methylsulfonyl, ethylsulfonyl)), a C₁₋₄ alkoxy-sulfonyl group (e.g. methoxysulfonyl), a benzyloxycarbonyl group, a C₃₋₆ cycloalkyl-carbonyl group, a carbamoyl group, a mono- or di-C₁₋₄ alkylcarbamoyl group and the like.

More specifically, as a substituent for the linear hydrocarbon group, 1 to 4 substituents selected from a halogen atom; an amino group; a mono-or di-C₁₋₄ alkylamino group; a carboxyl group; a C₁₋₄ alkoxycarbonyl group; a hydroxy group; an optionally halogenated C₁₋₄ alkoxy group; a C₃₋₆ cycloalkyl group; a nitro group; a cyano group; an optionally halogenated C₁₋₄ alkylthio group; a cyclic amino group substituted with 1 to 2 substituents selected from (i) a C₁₋₄ alkyl group, (ii) a C₁₋₄ alkylsulfonyl group, (iii) a C₆₋₁₂ aryl group which may be substituted with a halogen atom or a hydroxy group, (iv) a C₇₋₁₅ aralkyl group, (v) a C₁₋₄ alkoxy-C₁₋₄ alkyl group, (vi) a 5- to 9-membered heterocyclic group containing 1 to 3 heteroatoms such as a nitrogen atom, an oxygen atom and a sulfur atom in addition to carbon atoms and (vii) a hydroxy group (e.g. 5- to 9-membered cyclic amino group which may contain 1 to 3 heteroatoms such as oxygen atom and sulfur atom in addition to a nitrogen atom, more specifically, for example, pyrrolidinyl, piperidinyl, piperazinyl, morpholinyl etc.); a C₁₋₄ alkyl-carbonylamino group; an aminocarbonyloxy group; a mono- or di-C₁₋₄ alkylaminocarbonyloxy group; a C₁₋₄ alkylsulfonylamino group; a C₁₋₄ alkoxy-carbonyl group; a benzyloxycarbonyl group; a carboxyl group; a C₁₋₆ alkylcarbonyl group; a C₃₋₆ cycloalkyl-carbonyl group; a carbamoyl group; a mono- or di-C₁₋₄ alkylcarbamoyl group; a C₁₋₆ alkylsulfonyl group; a C₁₋₆ alkyl-carbonyloxy group; an amino group substituted with C₁₋₄ alkyl and a 5- to 9-membered heterocyclic group containing 1 to 3 heteroatoms such as a nitrogen atom, an oxygen atom and a sulfur atom in addition to carbon atoms; an amino group substituted with C₁₋₄ alkyl and C₁₋₄ alkyl-carbonyl; an amino group substituted with C₁₋₄ alkyl and C₆₋₁₂ aryl-carbonyl; a C₁₋₆ alkyl-carbonyloxy group; a mono or di-C₁₋₄ alkoxy-C₁₋₄ alkylamino group; a 5- to 9-membered heterocycle, containing 1 to 3 heteroatoms such as a nitrogen atom, an oxygen atom and a sulfur atom in addition to carbon atoms,-thio group; and an oxo group are used.

As each of R¹ and R², a halogen atom (e.g. a fluorine atom, a chlorine atom, a bromine atom), an optionally substituted C₁₋₇ alkyl group (preferably C₁₋₄ alkyl group such as methyl, ethyl and propyl, particularly preferably methyl), an optionally substituted C₂₋₆ alkenyl group (preferably ethenyl) and a hydroxyl group which may be substituted with an optionally substituted C₁₋₇ alkyl group (preferably, hydroxy group, C₁₋₄ alkoxy group such as methoxy) are preferable. Inter alia, a halogen atom and an optionally substituted C₁₋₇ alkyl group are preferable. The "C₁₋₇ alkyl group" of the "optionally substituted C₁₋₇ alkyl group" may have an oxo group as such a substituent. When the C₁₋₇ alkyl group is substituted with an oxo group at the α-position, it may form a C₁₋₇ alkanoyl group such as formyl and acetyl.

As a substituent for the "optionally substituted C₁₋₇ alkyl group", preferred are, for example,
(i) a hydroxy group,
(ii) a mono- or di-C₁₋₄ alkylamino group (e.g. dimethylamino, diethylamino),
(iii) an amino group substituted with C₁₋₄ alkyl and a 5-to 9-membered heterocyclic group containing 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazoyl, benzisothiazolyl, benzoxazolyl, benzisoxazolyl etc.) (e.g. methyl(2-pyridyl)amino),
(iv) an amino group substituted with C₁₋₄ alkyl and C₁₋₄ alkyl-carbonyl (e.g. methyl(methylcarbonyl)amino),
(v) an amino group substituted with C₁₋₄ alkyl and C₆₋₁₂ aryl-carbonyl (e.g. methyl(benzoyl)amino),
(vi) a mono or di-C₁₋₄ alkoxy-C₁₋₄ alkyl-amino group (e.g. butoxypropylamino),
(vii) a 5- to 9-membered cyclic amino group which may contain 1 to 3 heteroatoms such as an oxygen atom and a sulfur atom in addition to a nitrogen atom, which may be substituted with C₆₋₁₂ aryl optionally substituted with 1 to 4 substituents selected from C₁₋₄ alkyl (e.g. methyl), a halogen atom, a hydroxy group and optionally halogenated C₁₋₄ alkyl (e.g. phenyl, 4-hydroxyphenyl, 4-chlorophenyl, 3-methylphenyl), C₁₋₄ alkylsulfonyl (e.g. methylsulfonyl), C₇₋₁₅ aralkyl optionally substituted with 1 to 4 substituents selected from a halogen atom, hydroxy group and optionally halogenated C₁₋₄ alkyl (e.g. benzyl), C₁₋₄ alkoxy-C₁₋₄ alkyl (e.g. propoxyethyl etc.), a 5- to 9-membered heterocyclic group containing 1 to 3 heteroatoms such as a nitrogen atom, an oxygen atom and a sulfur atom in addition to carbon atoms (e.g. piperidyl, piperazinyl, morpholinyl, thienyl, furyl, pyridyl, pyrimidinyl, thiazolyl, benzothiazolyl, benzisothiazolyl, benzoxazolyl, benzisoxazolyl), a hydroxy group and the like (e.g. pyrrolidinyl, piperidyl, piperazinyl, morpholinyl, 3,6-dihydropyridin-1(2H)-yl) (preferably piperazinyl substituted with a phenyl group at the 4-position; wherein the phenyl group may be halogenated),
(viii) a C₁₋₆ alkyl-carbonyloxy group (e.g. methylcarbonyloxy, ethylcarbonyloxy, butylcarbonyloxy etc.),
(ix) a 5- to 9-membered heterocycle, containing 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazolyl, benzisothiazolyl, benzoxazolyl, benzisoxazolyl etc.),-thio group (e.g. 2-pyridylthio).

As a substituent for the C₂₋₆ alkenyl group, for example, C₁₋₄ alkoxy-carbonyl (e.g. methoxycarbonyl) is preferable.

In the formula [I], examples of the "cyclic hydrocarbon", when R¹ and R² are taken together with the adjacent carbon atoms to form an optionally substituted cyclic hydrocarbon, include a saturated or unsaturated cyclic aliphatic hydrocarbon (e.g. cycloalkane, cycloalkene, cycloalkadiene etc.) and an aromatic hydrocarbon.

Examples of the "cycloalkane" include cyclopropane, cyclobutane, cyclopentane, cyclohexane, cycloheptane, cycloctane, cyclononane and the like and, inter alia, C₃₋₇ cycloalkane such as cyclopropane, cyclobutane, cyclopentane, and cyclohexane is preferable.

Examples of the "cycloalkene" include C₅₋₆ cycloalkene such as cyclopentene, cyclohexene, cyclobutene, cyclopentene and the like.

Examples of the "cycloalkadiene" include C₅₋₆ cycloalkadiene such as 2,4-cyclopentadiene, 2,4-cyclohexadiene, 2,5-cyclohexadiene and the like.

Examples of the "aromatic hydrocarbon" include a monocyclic or fused polycyclic aromatic hydrocarbon having 6 to 16 carbon atoms such as a benzene ring, a naphthalene ring, an anthracene ring, a phenanthrene ring, an acenaphthalene ring and the like and, inter alia, C₆₋₁₀ aryl such as a benzene ring and a naphthalene ring is particularly preferable.

Preferable examples of the cyclic hydrocarbon, when R¹ and R² are taken together with the adjacent carbon atoms to form a cyclic hydrocarbon, include a C₅₋₇ cyclic hydrocarbon. More preferable examples of the cyclic hydrocarbon include a saturated or unsaturated cyclic aliphatic hydrocarbon (e.g. cycloalkane, cycloalkene, cycloalkadiene etc.). Particularly preferable examples of the cyclic hydrocarbon include cyclopropane, cyclobutane, cyclopentane, cyclohexane, cycloheptane, cycloctane, cyclononane and the like. Inter alia, C₃₋₇ cycloalkane such as cyclopropane, cyclobutane, cyclopentane and cyclohexane is particularly preferable.

A substituent for the "optionally substituted cyclic hydrocarbon" is the same as a substituent for the aforementioned "optionally substituted linear hydrocarbon group". As a substituent for the aforementioned unsaturated cyclic aliphatic hydrocarbon, an oxo group, a hydroxy group and the like are preferable.

Preferable examples of R¹ and R² include a hydrogen atom, a halogen atom, and an optionally substituted linear hydrocarbon group, or they may be taken together with the adjacent carbon atoms to form an optionally substituted cyclic hydrocarbon. Inter alia, as R¹ and R², a halogen atom and an optionally substituted C₁₋₇ alkyl group are preferable. Inter alia, a halogen atom and methyl are preferable.

In a preferable example of R¹ and R², R¹ is a halogen atom and R² is a linear hydrocarbon group substituted with an optionally substituted amino group (in particular, R² is a linear hydrocarbon group substituted with an optionally substituted cyclic amino group).

In the formula [I], ring A represents an optionally further substituted benzene ring.

In the formula [I], ring B represents an optionally substituted aromatic ring.

Examples of the "aromatic ring" of the "optionally substituted aromatic ring" represented by ring B include aromatic hydrocarbon and an aromatic heterocyclic ring.

Examples of the "aromatic hydrocarbon" include a monocyclic or fused polycyclic aromatic hydrocarbon having 6 to 16 carbon atoms, such as a benzene ring, a naphthalene ring, an anthracene ring, a phenanthrene ring, an acenaphthalene ring and the like. Inter alia, a benzene ring is particularly preferable.

Examples of the "aromatic heterocyclic ring" include a 5- to 6-membered aromatic monocyclic heterocyclic ring (e.g. furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine etc.), and a 8- to 16-membered aromatic fused heterocyclic ring in which 2 to 3 of 5- to 6-membered rings (e.g. the aforementioned 5- to 6-membered aromatic monocyclic heterocyclic rings or a benzene ring) are fused (e.g. benzofuran, isobenzofuran, benzo[b]thiophene, indole, isoindole, 1H-indazole, benzimidazole, benzoxazole, 1,2-benzisoxazole, benzothiazole, 1,2-benzisothiazole, 1H-benzotriazole, quinoline, isoquinoline, cinnoline, quinazoline, quinoxaline, phthalazine, naphthyridine, purine, pteridine, carbazole, α-carboline, β-carboline, γ-carboline, acridine, phenoxazine, phenothiazine, phenazine, thianthrene, phenanthridine, phenathroline, indolizine, pyrrolo[1,2-b]pyridazine, pyrazolo[1,5-a]pyridine, imidazo[1,2-a]pyridine, imidazo[1,2-b]pyrazole, imidazo[1,5-a]pyridine, imidazo[4,5-c]pyridine, pyrazolo[1,5-a]pyrimidine, pyrazolo[1,5-c]pyrimidine, pyrazolo[3,4-d]pyrimidine, imidazo[1,2-b]pyridazine, imidazo[1,5-b]pyridazine, pyrazolo[3,4-b]pyridine, imidazo[1,2-a]pyrimidine, 1,2,4-triazolo[4,3-a]pyridine, 1,2,4-triazolo[4,3-b]pyridazine, [1,2,4]triazolo[1,2-a]pyridazine, [1,2,3]triazolo[1,5-a]pyrimidine, [1,2,4]triazolo[1,5-c]pyrimidine, [1,2,4]triazolo[1,5-a]pyridine, [1,2,4]triazolo[4,3-a]pyridine, benzo[1,2,5]thiadiazole, benzo[1,2,5]oxadiazole, pyrazolo[5,1-b]thiazole, pyrrolo[2,1-f][1,2,4)triazine, pyrrolo[1,2-b]pyridazine, pyrrolo[2,3-d]pyrimidine, pyrrolo[2,3-b]pyridine, thieno[3,2-b]pyrimidine, thieno[2,3-b]pyridine, thieno[2,3-c]pyridine, thieno[3,2-b]pyridine, thieno[3,2-c]pyridine, pyrido[2,3-b]pyrazine, pyrido[3,4-b]pyrazine, pyrido[2,3-d]pyrimidine, pyrido[3,2-d]pyrimidine, pyrido[4,3-d]pyrimidine) etc. Inter alia, a 5- to 6-membered aromatic monocyclic heterocyclic ring such as furan, thiophene, pyrazine, pyridine and pyrimidine is preferable.

In the formula [I], a substituent for an optionally further substituted benzene ring represented by ring A or an optionally substituted aromatic ring represented by ring B includes
(i) an optionally halogenated C₁₋₄ alkyl group (e.g. methyl, chloromethyl, difluoromethyl, trichloromethyl, trifluoromethyl, ethyl, 2-bromoethyl, 2,2,2-trifluoroethyl, propyl, isopropyl, 3,3,3-trifluoropropyl, butyl etc.);
(ii) a C₁₋₄ alkyl group substituted with an amino group (e.g. aminomethyl, 2-aminoethyl etc.);
(iii) a C₁₋₄ alkyl group substituted with a mono- or di-C₁₋₄ alkylamino group (e.g. methylaminomethyl, dimethylaminomethyl, 2-methylaminoethyl, 2-dimethylaminoethyl etc.);
(iv) a C₁₋₄ alkyl group substituted with a carboxyl group (e.g. carboxymethyl, carboxyethyl etc.);
(v) a C₁₋₄ alkyl group substituted with a C₁₋₄ alkoxycarbonyl group (e.g. methoxycarbonylethyl, ethoxycarbonylethyl, etc.);
(vi) a C₁₋₄ alkyl group substituted with a hydroxy group (e.g. hydroxymethyl, hydroxyethyl etc.);
(vii) a C₁₋₄ alkyl group substituted with a C₁₋₄ alkoxy group which may be substituted with a C₁₋₄ alkoxy group or a phenoxy group (e.g. methoxymethyl, methoxyethyl, ethoxyethyl etc.);
(viii) a C₃₋₆ cycloalkyl group (e.g. cyclopropyl, cyclobutyl, cyclopentyl, cyclohexyl etc.);
(ix) a halogen atom (e.g. fluorine, chlorine, bromine, iodine);
(x) a nitro group;
(xi) a cyano group;
(xii) a hydroxy group;
(xiii) an optionally halogenated C₁₋₄ alkoxy group (e.g. methoxy, difluoromethoxy, trifluoromethoxy, ethoxy, 2,2,2-trifluoroethoxy, propoxy, butoxy, isopropoxy etc.), C₁₋₄ alkoxy groupwhich may be substituted with a C₁₋₄ alkoxy group or a phenoxy group;
(xiv) an optionally halogenated C₁₋₄ alkylthio group (e.g. methylthio, difluoromethylthio, trifluoromethylthio, ethylthio, propylthio, isopropylthio, butylthio etc.), a C₁₋₄ alkylthio group which may be substituted with a C₁₋₄ alkoxy group or a phenoxy group;
(xv) an amino group;
(xvi) a mono-or di-C₁₋₄ alkylamino group (e.g. methylamino, ethylamino, propylamino, dimethylamino, diethylamino, etc.);
(xvii) a cyclic amino group (e.g. 5- to 9-membered cyclic amino group which may contain 1 to 3 heteroatoms such as an oxygen atom and a sulfur atom in addition to the nitrogen atom, specifically, pyrrolidinyl, piperidyl, piperazinyl, morpholinyl etc.);
(xviii) a C₁₋₄ alkyl-carbonylamino group (e.g. acetylamino, propionylamino, butyrylamino etc.);
(xix) an aminocarbonyloxy group;
(xx) a mono- or di-C₁₋₄ alkylamino-carbonyloxy group (e.g. methylaminocarbonyloxy, ethylaminocarbonyloxy, dimethylaminocarbonyloxy, diethylaminocarbonyloxy etc.);
(xxi) a C₁₋₄ alkylsulfonylamino group (e.g. methylsulfonylamino, ethylsulfonylamino, propylsulfonylamino etc.);
(xxii) a C₁₋₄ alkoxy-carbonyl group (e.g. methoxycarbonyl, ethoxycarbonyl, propoxycarbonyl, isobutoxycarbonyl etc.);
(xxiii) a benzyloxycarbonyl group;
(xxiv) a carboxyl group;
(xxxv) a C₁₋₆ alkyl-carbonyl group (e.g. methylcarbonyl, ethylcarbonyl, butylcarbonyl etc.);
(xxvi) a C₃₋₆ cycloalkyl-carbonyl (e.g. cyclohexylcarbonyl etc.);
(xxvii) a carbamoyl group;
(xxviii) a mono- or di-C₁₋₄ alkylcarbamoyl group (e.g. methylcarbamoyl, ethylcarbamoyl, propylcarbamoyl, butylcarbamoyl, diethylcarbamoyl, dibutylcarbamoyl etc.);
(xxix) a C₁₋₆ alkylsulfonyl group (e.g. methylsulfonyl, ethylsulfonyl, propylsulfonyl etc.); C₃₋₆ cycloalkylsulfonyl (e.g. cyclopentylsulfonyl, cyclohexylsulfonyl etc.);
(xxx) a C₁₋₆ alkyl group substituted with a cyclic amino group (e.g. 5- to 9-membered cyclic amino group which may contain 1 to 3 heteroatoms such as an oxygen atom and a sulfur atom in addition to the nitrogen atom, specifically, pyrrolidinyl, piperidyl, piperazinyl, 3,6-dihydropyridin-1(2H)-yl, [1,3]thiazolo[4,5-b]pyridin-3(2H)-yl, morpholinyl etc.) substituted with 1 or 2 substituents selected from (a) C₁₋₄ alkyl (e.g. methyl), (b) C₁₋₄ alkylsulfonyl (e.g. methylsulfonyl), (c) a C₆₋₁₂ aryl group which may have optionally halogenated C₁₋₄ alkyl (e.g. methyl, trifluoromethyl), halogen (e.g. fluorine, chlorine) or a hydroxy group (e.g. phenyl, naphthyl, hydroxyphenyl, methylphenyl, chlorophenyl etc.), (d) C₇₋₁₅ aralkyl (e.g. benzyl etc.), (e) C₁₋₄ alkoxy-C₁₋₄ alkyl (e.g. propoxyethyl etc.), (f) a 5- to 9-membered heterocyclic group containing 1 to 3 heteroatoms such as a nitrogen atom, an oxygen atom and a sulfur atom in addition to carbon atoms (e.g. piperidyl, piperazinyl, morpholinyl, thienyl, furyl, pyridyl, pyrimidinyl, thiazolyl, benzothiazolyl, benzisothiazolyl, benzoxazolyl, benzisoxazolyl etc.), (g) hydroxy, thiol, oxo and thioxo (e.g. morpholinomethyl, 4-phenyl-1-piperazinylmethyl, 2-morpholinoethyl, 3-piperazinylpropyl, 4-methylsulfonyl-piperazinylmethyl, 4-benzyl-1-piperazinylmethyl, 4-(4-hydroxyphenyl)-1-piperazinylmethyl, 4-hydroxypiperidinylmethyl, 4-hydroxy-4-phenyl-piperidylmethyl, 4-phenylpiperidylmethyl, 4-(2-pydyl)-1-piperazinylmethyl, 4-(4-hydroxyphenyl)-1-piperazinylmethyl, (4-phenyl-3,6-dihydropyridin-1(2H)-yl)methyl etc.);
(xxxi) a C₁₋₄ alkyl group substituted with a C₁₋₆ alkyl-carbonyloxy group (e.g. methylcarbonyloxy, ethylcarbonyloxy, butylcarbonyloxy, etc.);
(xxxii) a C₁₋₄ alkyl group substituted with an amino group substituted with C₁₋₄ alkyl and a 5- to 9-membered heterocyclic group containing 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazolyl, benzisothiazolyl, benzoxazolyl, benzisoxazolyl etc.) (e.g. methyl(2-pyridyl)amino);
(xxxiii) a C₁₋₄ alkyl group substituted with an amino group substituted with C₁₋₄ alkyl and C₁₋₄ alkyl-carbonyl (e.g. methyl(methylcarbonyl)amino);
(xxxiv) a C₁₋₄ alkyl group substituted with an amino group substituted with C₁₋₄ alkyl and C₆₋₁₂ aryl-carbonyl (e.g. methyl(benzoyl)amino);
(xxxv) a C₁₋₄ alkyl group substituted with a C₁₋₆ alkyl-carbonyloxy group (e.g. methylcarbonyloxy, ethylcarbonyloxy, butylcarbonyloxy etc.);
(xxxvi) a C₁₋₄ alkyl group substituted with a mono or di-C₁₋₄ alkoxy-C₁₋₄ alkyl-amino group (e.g. butoxypropylamino);
(xxxvii) a C₁₋₄ alkyl group substituted with a 5- to 9-membered heterocycle, containing 1 to 3 heteroatoms such as a nitrogen atom, an oxygen atom and a sulfur atom in addition to carbon atoms (e.g. thienyl, furyl, pyridyl, pyrimidinyl, thiazolyl, benzothiazolyl, benzisothiazolyl, benzoxazolyl, benzisoxazolyl, etc.),-thio group (e.g. 2-pyridylthio);
(xxxviii) an oxo group;
(xxxiv) a C₁₋₄ alkoxy-carbonyl C₂₋₆ alkenyl group (e.g. methoxycarbonylvinyl etc.);
(xxxx) a C₂₋₆ alkenyl group substituted with a carboxyl group (e.g. carboxyvinyl etc.);
(xxxxi) a Cᵢ-₄ alkyl group substituted with a cyano group (e.g. cyanomethyl etc.);
(xxxxii) a C₆₋₁₀ aryl group (e.g. phenyl, naphthyl etc.), phenoxy, benzoyl, phenoxycarbonyl, phenyl-C₁₋₄ alkylcarbamoyl, phenylcarbamoyl, phenyl-C₁₋₄ alkylcarbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino [each phenyl group or each naphthyl group may be substituted with 1 to 3 substituents such as a C₁₋₄ alkyl group (e.g. methyl, ethyl, propyl, butyl, isopropyl etc.), a C₁₋₄ alkoxy group (e.g. methoxy, ethoxy, n-propyloxy, i-propyloxy, n-butyloxy etc.), a halogen atom (e.g. chloro, bromo, iodo etc.), a hydroxy group, a benzyloxy group, an amino group, a mono- or di-C₁₋₄ alkylamino group (e.g. methylamino, dimethylamino, ethylamino, diethylamino, diisopropylamino etc.), a nitro group, and a C₁₋₆ alkylcarbonyl group (e.g. 1-oxoethyl, 1-oxopropyl, 1-oxobutyl etc.) at substitutable position] and the like. The benzene ring or the aromatic ring may be substituted with 1 to 5, preferably 1 to 3 of these substituents at substitutable positions, wherein these substituents may be the same as or different from each other.

Preferable examples of such a substituent include (i) a halogen atom (e.g. fluorine, chlorine, bromine etc.), (ii) an optionally halogenated C₁₋₄ alkyl group (e.g. methyl, chloromethyl, difluoromethyl, trifluoromethyl, ethyl, propyl, isopropyl etc.), (iii) a C₃₋₆ cycloalkyl group (e.g. cyclopropyl, cyclobutyl etc.), (iv) a hydroxy group, (v) an optionally halogenated C₁₋₄ alkoxy group (e.g. methoxy, difluoromethoxy, trifluoromethoxy, ethoxy etc.), (vi) an optionally halogenated C₁₋₄ alkylthio group (e.g. methylthio, trifluoromethylthio, ethylthio, etc.), (vii) an amino group, (viii) a mono- or di-C₁₋₄ alkylamino group (e.g. methylamino, ethylamino, dimethylamino, diethylamino etc.), (ix) a C₁₋₄ alkoxy-carbonyl group (e.g. methoxycarbonyl, ethoxycarbonyl etc.), (x) a C₁₋₆ alkyl group substituted with a cyclic amino group (e.g. 5- to 9-membered cyclic amino group which may contain 1 to 3 heteroatoms such as an oxygen atom and a sulfur atom in addition to the nitrogen atom, specifically, pyrrolidinyl, piperidyl, morpholinyl etc.) which may be substituted with C₆₋₁₂ aryl group (e.g. phenyl, naphthyl etc.) (e.g. morpholinomethyl, 4-phenyl-1-piperazinylmethyl, 2-morpholinoethyl, 3-piperazinylpropyl etc.) and (xi) a carboxyl group. Particularly preferred are (i) a halogen atom (e.g. fluoro, chloro etc.), (ii) C₁₋₄ alkyl (e.g. methyl, ethyl etc.) (iii) a C₃₋₆ cycloalkyl group (e.g. cyclopropyl, cyclobutyl, etc.), (iv) a hydroxy group, (v) a C₁₋₄ alkoxy group (e.g. methoxy, ethoxy etc.), (vi) a C₁₋₆ alkyl group substituted with a cyclic amino group (e.g. 5-to 9-membered cyclic amino group which may contain 1 to 3 heteroatoms such as an oxygen atom and a sulfur atom in addition to the nitrogen atom, specifically, pyrrolidinyl, piperidyl, piperazinyl, 3,6-dihydropyridin-1(2H)-yl, morpholinyl, etc.) which may be substituted with a C₆₋₁₂ aryl group (e.g. phenyl, naphthyl etc.) (e.g. morpholinomethyl, 4-phenyl-1-piperazinylmethyl, 2-morpholinoethyl, (4-phenyl-3,6-dihydropyridin-1(2H)-ylmethyl), 3-piperazinylpropyl etc.) and (vii) a carboxyl group.

As ring A, a benzene ring which may be further substituted with an alkyl group, an optionally halogenated alkyl group or a halogen atom in addition to the substituent represented by the formula -X-Y is preferable, and a benzene ring which may be further substituted with a C₁₋₆ alkyl group, a halogenated C₁₋₄ alkyl group or a halogen atom in addition to the substituent represented by the formula -X-Y is particularly preferable.

As ring B, a benzene ring or a pyridine ring which each may be substituted with a halogenated alkyl group and/or a halogen atom is preferable (more preferably, a benzene ring substituted with a halogenated alkyl group and/or a halogen atom) and, inter alia, a benzene ring which may be substituted with a halogenated C₁₋₄ alkyl group (preferably trifluoromethyl) and/or a halogen atom is particularly preferable (more preferably, a benzene ring substituted with a halogenated C₁₋₄ alkyl group and/or a halogen atom).

In the formula [I], X represents a bond or a spacer whose main chain consists of 1 to 6 atoms.

As the "spacer whose main chain consists of 1 to 6 atoms" represented by X, divalent groups comprising 1 to 3 selected from -O-, -S-, -CO-, -SO-, -SO₂-, -NR³- (R³ represents a hydrogen atom, optionally halogenated C₁₋₆ alkyl, optionally halogenated C₁₋₆ alkyl-carbonyl, optionally halogenated C₁₋₆ alkylsulfonyl) and an optionally halogenated divalent C₁₋₆ linear hydrocarbon group are used.

Preferable examples of the "spacer whose main chain consists of 1 to 6 atoms" include:
(1) C₁₋₆ alkylene (e.g. -CH₂-, -(CH₂)₂-, -(CH₂)₃-, -(CH₂)₄-, -(CH₂)₅-, -(CH₂)₆-, -CH(CH₃)-, -C(CH₃)₂-, -(CH(CH₃))₂-, -(CF₂)₂-, -(CH₂)₂C(CH₃)₂-, -(CH₂)₃C(CH₃)₂-, etc.);
(2) C₂₋₆ alkenylene (e.g. -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂-, etc.);
(3) C₂₋₆alkynylene (e.g. -C≡C-, -CH₂-C≡C-, -CH₂-C≡C-CH₂-CH₂-, etc.);
(4) -(CH₂)_{W1}O(CH₂)_{W2}-, -(CH₂)_{W1}S(CH₂)_{W2}--(CH₂)_{W1}CO(CH₂)_{W2}-, -(CH₂)_{W1}SO(CH₂)_{W2}-, -(CH₂)_{W1}SO₂(CH₂)_{W2}-, -(CH₂)_{W1}NR³(CH₂)_{W2}-;
(5) -(CH₂)_{W3}CONR³(CH₂)_{W4}-, -(CH₂)_{W3}NR³CO(CH₂)_{W4}-, -(CH₂)_{W3}SO₂NR³(CH₂)_{W4}-, -(CH₂)_{W3}NR³SO₂(CH₂)_{W4}-, -(CH₂)_{W3}COO(CH₂)_{W4}-, -(CH₂)_{W3}OCO(CH₂)_{W4}-;
(6) -(CH₂)_{W5}NR³CONR^{3b}(CH₂)_{W6}-;
wherein R³ is as defined above; R^{3b} is as defined in R³; w1 and w2 represent an integer of 0 to 5, and w1+w2 is 0 to 5; w3 and w4 represent an integer of 0 to 4, and w3+w4 is 0 to 4; w5 and w6 represent an integer of 0 to 3, and w5+w6 is 0 to 3.

The "spacer whose main chain consist of 1 to 6 atoms" represented by X is preferably an optionally halogenated divalent C₁₋₆ linear hydrocarbon group and, inter alia, C₂₋₆ alkylene (e.g. a group represented by the formula -(CH₂)ₙ-, wherein n represents an integer of 0 to 6; n is preferably an integer of 1 to 4, more preferably 2), and C₂₋₆ alkenylene (e.g. a group represented by the formula -CH=CH-(CH₂)_{n'}-, wherein n' represents an integer of 0 to 4, a group represented by the formula -(CH=CH)_{n"}-, wherein n" represents an integer of 1 to 3; n' is preferably an integer of 0 to 2, more prefrably 0, and n" is preferably an integer of 1 to 2, more preferably 1) are preferable.

In the formula [I], Y represents an optionally esterified carboxyl group, an optionally substituted carbamoyl group, a cyano group, or an optionally substituted heterocyclic group having a hydrogen atom which can be deprotonated.

Examples of the "optionally esterified carboxyl group" represented by Y include, in addition to free carboxyl, lower alkoxycarbonyl, aryloxycarbonyl, aralkyloxycarbonyl and the like.

Examples of the "lower alkoxycarbonyl" include C₁₋₆ alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl and the like. Inter alia, C₁₋₃ alkoxycarbonyl such as methoxycarbonyl, ethoxycarbonyl, propoxycarbonyl and the like is preferable.

As the "aryloxycarbonyl", C₇₋₁₂ aryloxycarbonyl such as phenoxycarbonyl, 1-naphthoxycarbonyl, 2-naphthoxycarbonyl and the like is preferable.

As the "aralkyloxycarbonyl", C₇₋₁₀ aralkyloxycarbonyl such as benzyloxycarbonyl, phenethyloxycarbonyl and the like (preferably, C₆₋₁₀ aryl-C₁₋₄ alkoxy-carbonyl etc.) is preferable.

The "aryloxycarbonyl" and the "aralkyloxycarbonyl" may be substituted. The kind and number of such substituents are the same as those of the above-mentioned aryl and aralkyl groups which are exemplified as substituents for the "optionally substituted linear hydrocarbon group" represented by R¹ and R².

Examples of the "optionally substituted carbamoyl group" represented by Y include, in addition to unsubstituted carbamoyl, N-monosubstituted carbamoyl and N,N-disubstituted carbamoyl.

Examples of a substituent for the "N-monosubstitued carbamoyl" include lower alkyl (e.g. C₁₋₆ alkyl such as methyl, ethyl, propyl, isopropyl, butyl, isobutyl, tert-butyl, pentyl, hexyl etc.), lower alkenyl (e.g. C₂₋₆ alkenyl such as vinyl, allyl, isopropenyl, propenyl, butenyl, pentenyl, hexenyl etc.), cycloalkyl (e.g. C₃₋₆ cycloalkyl such as cyclopropyl, cyclobutyl, cyclopentyl, cyclohexyl etc.), aryl (e.g. C₆₋₁₀ aryl such as phenyl, 1-naphtyl, 2-naphtyl etc.), aralkyl (e.g. C₇₋₁₀ aralkyl such as benzyl, phenethyl etc., preferably phenyl-C₁₋₄ alkyl etc.), arylalkenyl (e.g. C₈₋₁₀ arylalkenyl such as cinnamyl etc., preferably phenyl-C₂₋₄ alkenyl etc.), a heterocyclic group (e.g. the same as the above-mentioned "heterocyclic group" which are exemplified as substituents for the "optionally substituted linear hydrocarbon group" represented by R¹ and R²), and amino which may be substituted with 1 to 2 C₁₋₆ alkyl. The lower alkyl, the lower alkenyl, the cycloalky, the aryl, the aralkyl, the arylalkenyl and the heterocyclic group may be substituted, and examples of such a substituent include a hydroxyl group, optionally substituted amino (the amino may be substituted with 1 or 2 substituents such as lower alkyl (e.g. C₁₋₆ alkyl such as methyl, ethyl, propyl, isopropyl, butyl, isobutyl, tert-butyl, pentyl, hexyl etc.), acyl (e.g. C₁₋₆ alkanoyl such as formyl, acetyl, propionyl and pivaloyl, benzoyl etc.), carboxyl, C₁₋₆-alkoxycarbonyl etc.), a halogen atom (e.g. fluorine, chlorine, bromine, iodine etc.), a nitro group, a cyano group, lower alkyl which may be substituted with 1 to 5 halogen atoms (e.g. fluorine, chlorine, bromine, iodine etc.), lower alkoxy which may be substituted with 1 to 5 halogen atoms (e.g. fluorine, chlorine, bromine, iodine etc.), and the like. Examples of the lower alkyl include C₁₋₆ alkyl such as methyl, ethyl, n-propyl, isopropyl, n-butyl, isobutyl, sec-butyl, tert-butyl, pentyl, hexyl and the like, and in particular, methyl, ethyl and the like are preferable. Examples of the lower alkoxy include C₁₋₆ alkoxy such as methoxy, ethoxy, n-propoxy, isopropoxy, n-butoxy, isobutoxy, sec-butoxy, tert-butoxy and the like, and in particular, methoxy, ethoxy and the like are preferable. Preferably, the lower alkyl, the lower alkenyl, the cycloalky, the aryl, the aralkyl, the arylalkenyl and the heterocyclic group may be substituted with 1 or 2 or 3 (preferably 1 or 2) substituents, wherein the substituents may be the same or different from each other.

The "N,N-disubstituted carbamoyl" means a carbamoyl group having two substituents on the nitrogen atom. Examples of one of such two substituents are the same as those of the aforementioned "N-monosubstituted carbamoyl", and examples of the other include lower alkyl (e.g. C₁₋₆ alkyl such as methyl, ethyl, propyl, isopropyl, butyl, tert-butyl, pentyl, hexyl etc.), C₃₋₆ cycloalkyl (e.g. cyclopropyl, cyclobutyl, cyclopentyl, cyclohexyl etc.), C₇₋₁₀ aralkyl (e.g. benzyl, phenethyl etc., preferably phenyl-C₁₋₄ alkyl etc.) and the like. Alternatively, the two substituents may be taken together with the nitrogen atom to form a cyclic amino. Examples of such a cyclic aminocarbonyl group include a 3- to 8-membered (preferably 5- to 6-membered) cyclic aminocarbonyl group such as 1-azetidinylcarbonyl, 1-pyrrolidinylcarbonyl, piperidinocarbonyl, morpholinocarbonyl, thiomorpholinocarbonyl (wherein the sulfur atom may be oxidized), 1-piperazinylcarbonyl, and 1-piperazinylcarbonyl which may be substituted with lower alkyl (e.g. C₁₋₆ alkyl such as methyl, ethyl, propyl, isopropyl, butyl, tert-butyl, pentyl, hexyl etc.), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.), or the like at the 4-position, and the like.

As the "heterocyclic group bearing a hydrogen atom capable of being deprotonated" of the "optionally substituted heterocyclic group bearing a hydrogen atom capable of being deprotonated" represented by Y, a 5- to 7-membered (preferably 5-membered) monocyclic heterocyclic group containing at least one of a nitrogen atom, a sulfur atom and an oxygen atom (preferably a nitrogen-containing heterocyclic group) bearing a hydrogen atom capable of being deprotonated (that is, capable of leaving to form a proton) (that is, having an active proton) is used. Examples of the "heterocyclic group bearing a hydrogen atom capable of being deprotonated" include tetrazol-5-yl and a group represented by the formula: 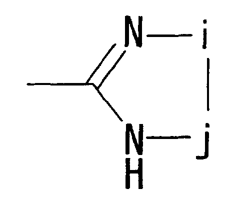 wherein i represents -O- or -S-, and j represents >C=O, >C=S or >S(O)2, (inter alia, 2,5-dihydro-5-oxo-1,2,4-oxadiazol-3-yl, 2,5-dihydro-5-thioxo-1,2,4-oxadiazol-3-yl, and 2,5-dihydro-5-oxo-1,2,4-thiadiazol-3-yl are preferable).

The "heterocyclic group bearing a hydrogen atom capable of being deprotonated" may be protected with an optionally substituted lower alkyl group (preferably C₁₋₄ alkyl) or an acyl group. Examples of the optionally substituted lower alkyl group include C₁₋₄ alkyl which may be substituted with phenyl optionally substituted with C₁₋₃ alkyl, nitro, or C₁₋₃ alkoxy, or C₁₋₃ alkoxy (e.g. methyl, triphenylmethyl, methoxymethyl, ethoxymethyl, p-methoxybenzyl, p-nitrobenzyl). Examples of the acyl group include lower (C₂₋₅) alkanoyl and benzoyl.

Y is preferably an optionally esterified carboxyl group, more preferably, carboxyl or lower alkoxycarboxyl, and most preferably, carboxyl.

The formula [I] is preferably the formula [I']: 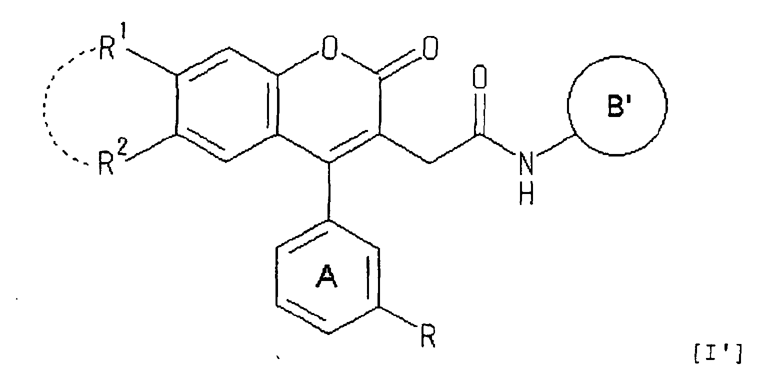 wherein ring B' represents an optionally substituted benzene ring or an optionally substituted pyridine ring, R represents an optionally esterified carboxyl group or a linear hydrocarbon group which is substituted with an optionally esterified carboxyl group, and the other symbols are as defined in claim 1.

The "optionally esterified carboxyl group" represented by R includes the same group as the "optionally esterified carboxyl group" represented by Y.

The "optionally esterified carboxyl group" of the "linear hydrocarbon group which is substituted with an optionally esterified carboxyl group" represented by R includes the same group as the "optionally esterified carboxyl group" represented by Y.

The "linear hydrocarbon group" of the "linear hydrocarbon group which is substituted with an optionally esterified carboxyl group" represented by R includes the same groups as C₁₋₆ alkylene, C₁₋₆ alkenylene and C₁₋₆ alkynylene which are preferably exemplified as the "spacer whose main chain consists of 1 to 6 atoms" represented by X.

In the formula [I'], R is preferably a group represented by the formula -(CH₂)ₙ-R', wherein R' represents an optionally esterified carboxyl group and n represents an integer of 0 to 6; a group represented by the formula -CH=CH- (CH₂)_{n'}-R' , wherein R' represents an optionally esterified carboxyl group and n' represents an integer of 0 to 4; or a group represented by the formula -(CH=CH)_{n"}-R', wherein R' represents an optionally esterified carboxyl group and n" represents an integer of 1 to 3. In the above formulae, n is preferably an integer of 1 to 4 (more preferably 2), n' is preferably an integer of 0 to 2 (more preferably 0), and n" is preferably an integer of 1 to 2 (more preferably 1).

The "optionally esterified carboxyl group" represented by R' includes the same group as the "optionally esterified carboxyl group" represented by Y, and, inter alia, carboxyl is particularly preferable.

The present invention also includes the free form or pharmaceutically acceptable salt of a compound represented by the formula [I]. Examples of such a salt, when the compound represented by the formula [I] has an acidic group such as a carboxyl group, a heterocyclic group bearing a hydrogen atom capable of being deprotonated and the like, include salts with inorganic bases (e.g. alkali metal such as sodium, potassium etc., alkaline earth metal such as calcium, magnesium etc., transition metal such as zinc, iron, copper etc.) or organic bases (e.g. organic amines such as trimethylamine, triethylamine, tris(hydroxymethyl)amine, pyridine, picoline, ethanolamine, diethanolamine, triethanolamine, dicyclohexylamine, and N,N'-dibenzylethylenediamine, and basic amino acids such as arginine, lysine and ornithine).

When the compound represented by the formula [I] has a basic group such as an amino group, examples of such a salt include salts with inorganic acids, organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), or acidic amino acids such as aspartic acid and glutamic acid.

The compound represented by the formula [I] or a salt thereof may be used as a prodrug. The prodrug refers to a compound which is converted into the compound represented by the formula [I] or a salt thereof as a result of a reaction with an enzyme or gastric acid under the physiological condition in a living body, that is, a compound which undergoes enzymatic oxidation, reduction or hydrolysis to form the compound represented by the formula [I] or a salt thereof or a compound which is hydrolyzed with gastric acid to form the compound represented by the formula [I] or a salt thereof. Examples of a prodrug of the compound represented by the formula [I] or a salt thereof include, when the compound represented by the formula [I] or a salt thereof has an amino group, the compound in which the amino group is acylated, alkylated or phosphorylated (e.g. a compound obtained by subjecting an amino group in the compound represented by the formula [I] or a salt thereof to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tertbutylation); when the compound represented by the formula [I] or a salt thereof has a hydroxyl group, the compound in which the hydroxy group is acylated, alkylated, phosphorylated or borated (e.g. a compound obtained by subjecting the hydroxy group to acetylation, palmitoylation, propanoylation, pivaloylation, succinylation, fumarylation, alanylation or dimethylaminomethylcarbonylation); when the compound represented by the formula [I] or a salt thereof has a carboxyl group, the compound in which the carboxyl group is esterified or amidated (e.g. a compound obtained by subjecting the carboxyl group to ethylesterification, phenylesterification, carboxymethylesterification, dimethylaminomethylesterification, pivaloyloxymethylesterification, ethoxycarbonyloxyethylesterification, phthalizylesterification, (5-methyl-2-oxo-1,3-dioxolen-4-yl)methylesterification, cyclohexyloxycarbonylethylesterification or methylamidation); and the like. These prodrugs can be prepared from the compound represented by the formula [I] or a salt thereof by a method known per se.

In addition, a prodrug of the compound represented by the formula [I] or a salt thereof may be changed into the compound represented by the formula [I] or a salt thereof under the physiological condition as described in "Development of Medicaments", vol.7, Molecular Design, p.163-198 published by Hirokawashoten in 1990.

In addition, the compound represented by the formula [I] or a salt thereof may be hydrous or anhydrous.

In addition, the compound represented by the formula [I] or a salt thereof may be labelled with an isotope element (e.g. ³H, ¹⁴C, ³⁵S, ¹²⁵I etc.).

Among the compounds represented by the formula [I], preferred are:
3-[3-[7-chloro-3-[2-[[4-fluoro-2-(trifluoromethyl)phenyl]amino]-2-oxoethyl]-6-methyl-2-oxo-2H-chromen-4-yl]phenyl]propionic acid, (2E)-3-[3-[7-chloro-6-methyl-2-oxo-3-(2-oxo-2-[[2-(trifluoromethyl)phenyl]amino]ethyl)-2H-chromen-4-yl]phenyl]-2-propenoic acid, 3-[3-[7-chloro-6-methyl-2-oxo-3-(2-oxo-2-[[2-(trifluoromethyl)phenyl]amino]ethyl)-2H-chromen-4-yl]phenyl]propionic acid, (2E)-3-[3-[6-chloro-3-(2-[[4-fluoro-2-(trifluoromethyl)phenyl]amino]-2-oxoethyl)-7-methyl-2-oxo-2H-chromen-4-yl]phenyl]-2-propenoic acid, 3-[3-[6-chloro-3-(2-[[4-fluoro-2-(trifluoromethyl)phenyl]amino]-2-oxoethyl)-7-methyl-2-oxo-2H-chromen-4-yl]phenyl]propionic acid, (2E)-3-(3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}phenyl)acrylic acid, (2E)-3-(3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}phenyl)acrylic acid, 3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromeh-4-yl}benzoic acid, 3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}benzoic acid; and a salt thereof.

The compound represented by the formula [I] or a salt thereof can be prepared according to the method disclosed, for example, in European Patent Application Publication No.585913, European Patent Application Publication No.602598, JP-A 6-263736 or International Publication WO 02/06264, or by the following method.

The compound [I] or a salt thereof can be prepared, for example, by carbonylation or Heck reaction of a compound represented by the formula [II]: 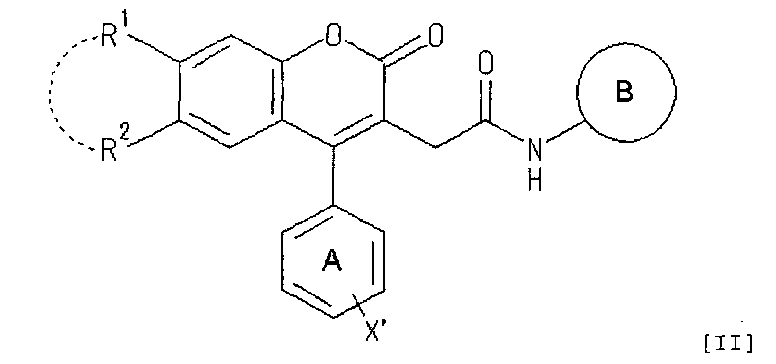 wherein X' represents halogen or a trifluoromethanesulfonyl group and other symbols are as defined above, or a salt thereof and then, if necessary, hydrogenation or hydrolysis, or a combination of both reactions.

The carbonylation reaction can be performed according to the method described, for example, in A.Schoenberg, et al., J.Org.Chem., 39, 3318-3326 (1974), M.Hidai, et al., Bull. Chem. Soc.Jpn, 48, 2075-2077 (1975), D.Valentine, Jr., et.al., J.Org.Chem., 46, 4616-4617 (1981). That is, the compound [II] is treated with a palladium catalyst and a base under carbon monoxide atmosphere and then reacted with a nucleophile to carbonylate the compound [II].

Carbon monoxide is used usually at 1 to 20 atm., preferably 1 to 10 atm.

The palladium catalyst includes palladium (II) acetate, palladium (II) chloride, dihalobis(triarylphosphine)palladium (II) (e.g. dichlorobis(triphenylphosphine)palladium(II), dibromobis(triphenylphosphine)palladium(II), diiodobis(triphenylphosphine)palladium (II), dichlorobis(tritolylphosphine)palladium(II) etc.), and haloarylbis(triarylphosphine)palladium(II) (e.g. chlorophenylbis(triphenylphosphine)palladium (II) etc.). The amount of the catalyst used is usually 0.005 to 0.1 mol, preferably 0.01 to 0.05 mol per 1 mol of the compound [II]. In addition, when 1 to 50 mol (preferably 2 to 20 mol) of triarylphosphine (e.g. triphenylphosphine, tri(o-tolyl)phosphine etc.) or bis(diarylphosphino)alkyl (e.g. 1,4-bis(diphenylphosphino)butane, 1,3-bis(diphenylphosphino)propane, 1,2-bis(diphenylphosphino)ethane etc.) coexists with 1 mol of a catalyst, the reaction may progress advantageously.

The base includes secondary amine (e.g. diethylamine, dicyclohexylamine etc.), tertiary amine (e.g. triethylamine, tributylamine, tetramethylethylenediamine etc.) and carbonate (e.g. sodium carbonate, potassium carbonate, sodium dicarbonate). The amount of the base used is usually 1 to 10 mol, preferably 1 to 3 mol per 1 mol of the compound [II].

The nucleophile includes water and lower alcohol (e.g. methanol, ethanol, butanol). The amount of the nucleophile used is usually 1 to 100 mol, preferably 1 to 10 mol per 1 mol of the compound [II].

The carbonylation reaction is performed in the presence or the absence of a solvent. The solvent includes amides (e.g. dimethylformamide, N-methylpyrrolidinone, hexamethylphosphoric triamide) and nitriles (acetonitrile, benzonitrile etc.). The amount of the solvent used is usually about 1 to 100 ml, preferably about 10 to 50 ml per 1 g of the compound [II].

The reaction temperature is usually about 10°C to 200°C, preferably about 20°C to 150°C. The reaction time is about 1 to 100 hours, preferably about 5 to 80 hours depending on a carbon monoxide pressure, the amount and the kind of a catalyst, a base or a reaction solvent, the reaction temperature, and the like.

The Heck reaction can be performed according to the method, for example, described in R.F.Heck, Org. Reactions, 27, 345-390(1982). That is, the compound [II] is reacted with olefin in the presence of a palladium catalyst and a base.

The olefin includes a compound represented by CH₂=CH-(CH₂)ₙ,-R' wherein R' represents an optionally esterified carboxyl group and n' represents an integer of 0 to 4, and a compound represented by CH₂=CH-(CH=CH)_{n'''}-R' wherein R' represents an optionally esterified carboxyl group and n''' represents an integer of 1 to 2, which are commercially available or prepared by a method known per se (e.g. a method described in R.S.Sandler and W.Karo, "Organic Functional Group Preparations I", Academic Press, 1983, Chapter 2 (p.39-81), Chapter 9 (p.236-288), Chapter 10 (p.289-315)). The amount of olefin used is usually 1 to 10 mol, preferably 1 to 3 mol per 1 mol of the compound [II].

The catalyst, the base and the reaction solvent are the same as those for the carbonylation reaction. In addition, when nickel [II] bromide or sodium iodide coexists, the reaction may progress advantageously.

The reaction temperature is usually about 10°C to 200°C, preferably about 20°C to 150°C. The reaction time is about 1 hour to 100 hours, preferably about 5 hours to 80 hours depending on the amount and the kind of a catalyst, a base or a reaction solvent, the reaction temperature and the like.

The hydrogenation reaction after the carbonylation reaction or the Heck reaction can be performed by a method known per se (e.g. a method described in P.Rylander, "Catalytic Hydrogenation in Organic Syntheses), Academic Press, 1979). The hydrolysis reaction follows, for example, the method of the reaction step 3 described hereinbelow.

The starting compound [II] or a salt thereof used in the aforementioned reaction can be prepared by the method described, for example, in European Patent Application Publication No.585913, JP-A 7-10844 gazette or International Publication WO 02/06264, the similar method, or the following method: 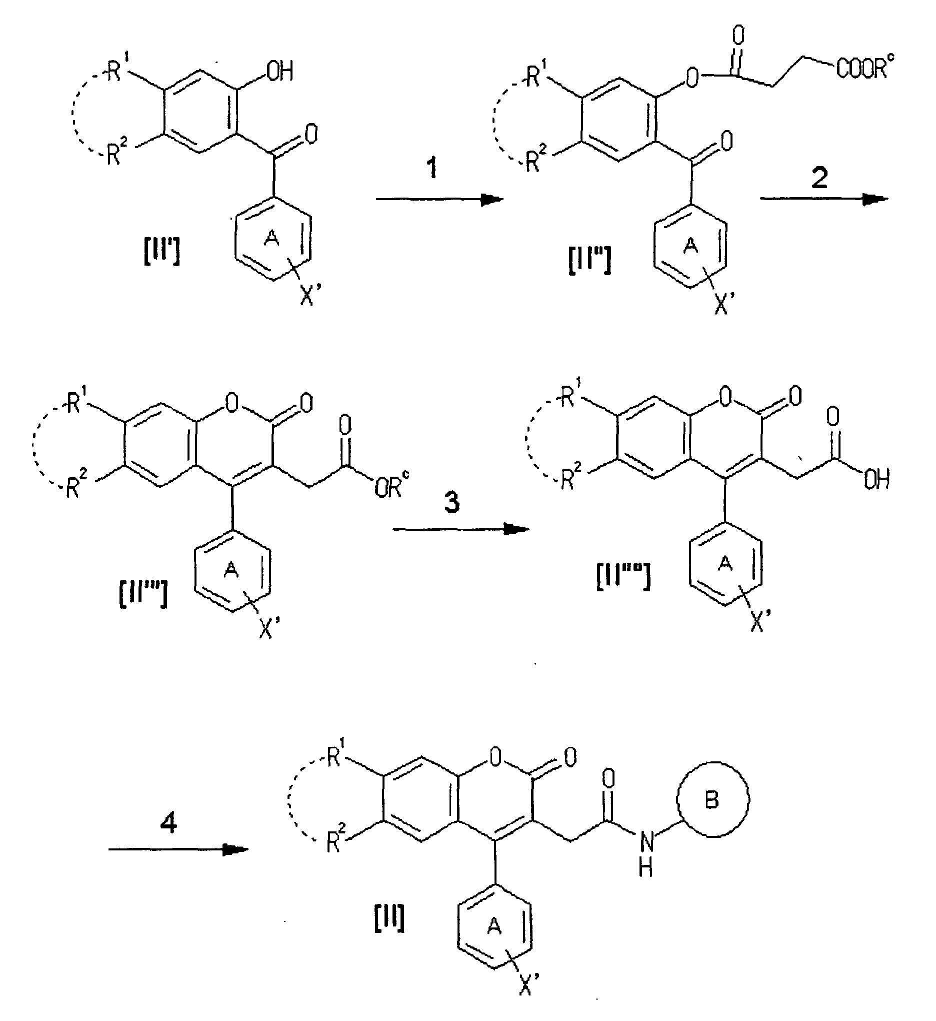 wherein R^{c} represents an alkyl group (e.g. methyl, ethyl, propyl, t-butyl etc.) and other symbols are as defined above.

The reaction step 1 is performed by condensing the compound [II'] or a salt thereof and a reactive derivative of succinic acid monoester.

As the reactive derivative of succinic acid monoester, for example, acid halide (e.g. acid chloride etc.) of succinic acid monoalkylester (e.g. methylester, ethylester, propylester) is used. In particular, ethylsuccinic chloride is preferable. The amount of a reactive derivative of succinic acid monoester used is usually equivalent to 10-fold molar amount, preferably equivalent to 3-fold molar amount based on the amount of the compound [II'] or a salt thereof.

The reaction is usually performed advantageously in the presence of a base. As the base, an organic or inorganic base is used. The organic base includes tertiary amines (e.g. triethylamine, diisopropylethylamine, diazabicycloundecene). The inorganic base includes alkali metal hydroxide such as lithium hydroxide, sodium hydroxide, potassium hydroxide and the like; alkali metal carbonate such as sodium carbonate, potassium carbonate, cesium carbonate and the like; alkali metal hydrogencarbonate such as sodium hydrogencarbonate, potassium hydrogencarbonate and the like; alkali metal hydride such as sodium hydride, potassium hydride and the like. The amount of a base used is usually equivalent to about 10-fold molar amount, preferably equivalent to 3-fold molar amount based on the amount of the compound [II'] or a salt thereof.

The reaction can be advantageously performed in a solvent. As the solvent, a solvent having no adverse influence on a reaction is used and includes hydrocarbons (e.g. pentane, hexane, cyclohexane, benzene, toluene etc.), halogenated hydrocarbons (e.g. dichloromethane, chloroform etc.), ethers (e.g. diethyl ether, tetrahydrofuran, dioxane etc.), amides (e.g. N,N-dimethylformamide, hexamethylphosphoric triamide etc.), ureas (e.g. 1,3-dimethyl-3,4,5,6-tetrahydro-2(1H)-pyrimidine etc.) and nitriles (e.g. acetonitrile, propionitrile etc.). The solvent may be a single or a mixture of two or more solvents in an appropriate ratio. The amount of a solvent used is usually about 1 to 100 ml, preferably about 10 to 50 ml per 1 g of the compound [II'] or a salt thereof. The reaction temperature is usually about -20°C to the boiling point of a solvent used in the reaction, preferably about 25°C to 100°C.

The reaction time is about 10 minutes to 24 hours, preferably about 20 minutes to 12 hours depending on the kind of a base or a reaction solvent used, the reaction temperature and the like.

The reaction step 2 is performed by treating the compound [II"] with a base. As the base, for example, the same bases as those exemplified as a base for the reaction step 1 can be used. The amount of a base used is usually about 0.1-fold to about 10-fold molar amount, preferably about 0.1-fold to 1-fold molar amount based on the amount of the compound [II"] or a slat thereof.

The reaction can be performed advantageously in a solvent. As the solvent, a solvent having no adverse influence on the reaction is used and includes hydrocarbons (e.g pentane, hexane, cyclohexane, benzene, toluene etc.), halogenated hydrocarbons (e.g. dichloromethane, chloroform etc.), ethers (e.g. diethyl ether, tetrahydrofuran, dioxane etc.), amides (e.g. N,N-dimethylformamide, hexamethylphosphoric triamide etc.), ureas (e.g. 1,3-dimethyl-3,4,5,6-tetrahydro-2(1H)-pyrimidine etc.) and nitriles (e.g. acetonitrile, propionitrile etc.). The solvent may be a single or a mixture of two or more solvents in an appropriate ratio. The amount of the solvent used is usually about 1 to 100 ml, preferably about 10 to 50 ml per 1 g of the compound [II"]. The reaction temperature is usually about 20°C to the boiling point of a solvent used in the reaction, preferably about 25°C to about 120°C.

The reaction time is about 30 minutes to 24 hours, preferably about 1 hour to 12 hours depending on the kind of a base or a reaction solvent, the reaction temperature and the like.

The reaction may progress advantageously by removing water produced during the reaction with the Dean-Stark dehydration apparatus or the like.

Alternatively the reaction step 1 and reaction step 2 may be performed in one step. For example, the compound [II'''] or a salt thereof can be prepared from the compound [II"] or a salt thereof in one step by using acid halide (e.g. acid chloride etc.) of succinic acid monoalkylester (e.g. methylester, ethylester, propylester) as a reactive derivative of succinic acid monoester and tertiary amines (e.g. triethylamine, diisopropylethylamine, diazabicycloundecene etc.) as a base in an excessive amount. In this case, the amount of acid halide used is usually about 1.5-fold to 10-fold molar amount, preferably about 1.5-fold to 3-fold molar amount based on the amount of the compound [II"] or a salt thereof. The amount of a base used is usually about 2-fold 10-fold molar amount, preferably about 2-fold to 5-fold molar amount based on the amount of the compound [II"] or a salt thereof.

The reaction can be performed advantageously in a solvent. As the solvent, a solvent having no adverse influence on a reaction is used. The kind and the amount of a solvent used are the same as those for the reaction step 1. The reaction temperature is usually about 20°C to the boiling point of a solvent used in the reaction, preferably about 25°C to 60°C. The reaction time is about 30 minutes to 24 hours, preferably about 30 minutes to 4 hours depending on the kind of acid halide or a base, the kind of a reaction solvent, the reaction temperature and the like.

The reaction step 3 can be performed by treating the compound [II'''] with an acid or a base.

As the acid, organic acid (e.g. formic acid, acetic acid, trichloroacetic acid, trifluoroacetic acid, benzenesulfonic acid, p-toluenesulfonic acid etc.) or inorganic acid (e.g. hydrochloric acid, hydrobromic acid, sulfuric acid, nitric acid, phosphoric acid etc.) can be used. The acid may be a mixture of two or more acids in an appropriate ratio. As the base, for example, alkali metal hydroxide (e.g. lithium hydroxide, sodium hydroxide, potassium hydroxide etc.), alkali metal carbonate (e.g. sodium carbonate, potassium carbonate, cesium carbonate etc.) or alkali metal hydrogencarbonate (e.g. sodium hydrogencarbonate, potassium hydrogencarbonate etc.) is used. The amount of an acid or a base used is usually about 1-fold to 100-fold molar amount, preferably about 1-fold to 10-fold molar amount based on the amount of the compound [II'''].

The reaction can be advantageously performed in a solvent. As the solvent, a solvent having no adverse influence on the reaction is used and includes hydrocarbons (e.g. pentane, hexane, cyclohexane, benzene etc.), lower alcohols (e.g. methanol, ethanol, propanol etc.), ethers (e.g. diethyl ether, tetrahydrofuran, dioxane etc.), amides (e.g. N,N-dimethylformamide, hexamethylphosphoric triamide etc.) and ureas (e.g. 1,3-dimethyl-3,4,5,6-tetrahydro-2(1H)-pyrimidine etc.). In the case of the reaction with an acid, the aforementioned acid may be also used as a solvent. The solvent may be a single, a mixture of two or more solvents in an appropriate ratio, or a mixed solvent with water. The amount of a solvent used is usually about 1 to 100 ml, preferably about 10 to 50 ml per 1 g of the compound [II'']. The reaction temperature is usually about -20°C to the boiling point of a solvent used in the reaction, preferably about 15°C to 120°C. The reaction time is about 10 minutes to 24 hours, preferably 30 minutes to 12 hours depending on the kind of an acid or a reaction solvent, the reaction temperature and the like.

The reaction step 4 can be performed by reacting the compound [II''''], a salt thereof or a reactive derivative of the carboxyl group thereof with a compound represented by the formula [III]: 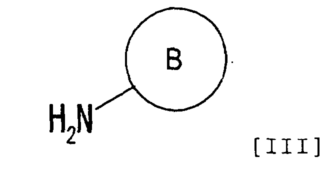 wherein the symbol is as defined above, or a salt thereof. As the reactive derivative of the carboxylic acid, for example, acid halide (e.g. chloride, bromide, etc.), acid anhydride, mixed acid anhydride (e.g. anhydride with methylcarbonic acid, anhydride with ethylcarbonic acid, anhydride with isobutylcarbonic acid), or active ester (e.g. ester with hydroxysuccinic acid imide, ester with 1-hydroxybenzotriazole, ester with N-hydroxy-5-norbornene-2,3-dicarboxyimide, ester with p-nitrophenol, ester with 8-oxyquinoline) is used. Inter alia, acid halide is preferable.

Alternatively, the compound [II] or a salt thereof may be prepared by reacting the compound [II''''] or a salt thereof with a compound represented by the formula [III] or a salt thereof in the presence of a coupling reagent. The coupling reagent includes carbodiimides (e.g. dicyclohexylcarbodiimide, N-[3-(dimethylamino)propyl]-N'-ethylcarobodiimide, N-cyclohexyl-N'-(2-morpholin-4-ylethyl)carobodiimide, N-cyclohexyl-N'-[4-(diehtylamino)cyclohexyl]carobodiimide etc.), carbonyldiimidazole, N-ethyl-5-phenylisoxazolium-3'-sulfonate, N-ethyl-2'-hydroxybenzisoxazoliumtrifluoroborate, 2-ethoxy-1-ethoxycarbonyl-1,2-dihydroquinoline, 2-isobutyloxy-1-isobutyloxycarobonyl-1,2-dihydroquinoline, (benzotriazolyl-N-hydroxytrisdiethylaminophosphoniumhexafluorophosphate and diphenylphosphorylazide. When carbodiimide is used together with an additive, the reaction may progress advantageously. As the additive, N-hydroxysuccinimide, 1-hydroxybenzotriazole, 3-hydroxy-4-oxo-3,4-dihydro-1,2,3-benzotriazine, N-hydroxy-5-norbornene-2,3-dicarboxylic acid imide, ethyl 2-hydroxyimino-2-cyanoacetate, 2-hydroxyimino-2-cyanoacetamide or the like is used.

A salt of the compound [II''''] or [III] includes the same salts as those of the compound [I] as described above.

The reaction is usually performed in a solvent having no adverse influence on the reaction (e.g. halogenated hydrocarbons such as chloroform, dichloromethane, ethyl ether, tetrahydrofuran, dioxane, dimethoxyethane, ethyl acetate, benzene, toluene, pyridine and N,N-dimethylformamide, ethers, esters, hydrocarbons, aromatic amines, amides etc.). The present reaction can be performed in the presence or the absence of a base. The reaction temperature is usually about -10°C to 120°C, preferably about 0°C to 100°C. The reaction time is usually about 5 minutes to 48 hours, preferably about 0.5 to 24 hours. The amount of the compound [III] or a salt thereof used is about 1 to 5 mol equivalent, preferably about 1 to 3 mol equivalent per 1 mol of the compound [II''''] or a salt thereof or a reactive derivative thereof. The base includes alkylamines such as triethylamine, cyclic amines such as N-methylmorpholine and pyridine, aromatic amines such as N,N-dimethylaniline and N,N-diethylaniline, alkali metal carbonate such as sodium carbonate and potassium carbonate, and alkali metal hydrogencarbonate such as sodium hydrogencarbonate and potassium hydrogencarbonate. The amount of a base used is about 1 to 5 mol equivalent, preferably about 1 to 3 mol equivalent per 1 mol of the compound [II''''] or a salt thereof. When a solvent immiscible with water is used in the present reaction, water may be added to the reaction system at an appropriate ratio to perform the reaction in a two-phase system. When a coupling reagent is used, the reaction is usually preferably performed under non-aqueous condition. The amount of a coupling reagent used is about 1 to 10 mol equivalent, preferably about 1 to 3 mol equivalent per 1 mol of the compound [II''''] or a salt thereof. When an additive is further used, the amount to be used is about 1 to 5 mol equivalent, preferably about 1 to 2 mol equivalent per 1 mol of a coupling regent.

Further, coumarinamide fused with cycloalkane having an oxo group may be synthesized by subjecting coumarinamide fused with cycloalkane to oxidation reaction in an appropriate stage of the synthesis. The oxidation reaction is performed using an oxidizing agent (e.g. permanganate, chromate etc.) according to a method known per se (e.g. A.B.Smith, III, et al. the Journal of Organic Chemistry, vol.50, p.3239-3241, 1985).

When R¹, R², ring A, ring B or Y of the compound (I) has a functional group capable of converting into the desired substituent (e.g. a carboxyl group, an amino group, a hydroxy group, a carbonyl group, a thiol group, an ester group, a sulfo group, a halogen atom etc.), the functional group can be converted by a method known per se or the similar method to prepare the desired compound.

For example, a carboxyl group can be converted by esterification, reduction, amidation, conversion into an optionally protected amino group, or the like. An amino group can be converted by amidation, sulfonylation, nitrosation, allkylation, arylation, imidation, or the like. A hydroxy group can be converted by esterification, carbamoylation, sulfonylation, allkylation, arylation, oxidation, halogenation, or the like. A carbonyl group can be converted by reduction, oxidation, imination (including oximation and hydrazonation), (thio) ketalization, alkylidenation, thiocarbonylation, or the like. A thiol group can be converted by alkylation, oxidation, or the like. An ester group can be converted by reduction, hydrolysis, or the like. A sulfo group can be converted by sulfonamidation, reduction, or the like. A halogen atom can be converted by various nucleophilic displacement reactions, various coupling reactions, or the like.

As salts of the compounds [II'], [II"], [II'''] and [II''''] used in the aforementioned reactions, the same salts as those of the compound [I] are used.

In each reaction of the above-described process for preparing the compound [I] or a salt thereof and each reaction for synthesizing the starting compound, when the starting compound has an amino group, a carboxyl group or a hydroxy group as a substituent, a conventional protecting group used in peptide chemistry may be introduced into the substituent and after the reaction, the protecting group may be removed as necessary to obtain the desired compound.

As a protecting group for an amino group, for example, formyl, optionally substituted C₁₋₆ alkylcarbonyl (e.g. acetyl, ethyl carbonyl etc.), phenylcarbonyl, C₁₋₆ alkyloxycarbonyl, (e.g. methoxycarbonyl, ethoxycarbonyl etc.), phenyloxycarbonyl, C₇₋₁₀ aralkyl-carbonyl (e.g. benzylcarbonyl etc.), trityl, phthaloyl or N,N-dimethylaminomethylene is used. As a substituent for them, a halogen atom (e.g. fluorine, chlorine, bromine, iodine etc.), C₁₋₆ alkyl-carbonyl (e.g. methylcarbonyl, ethylcarbonyl, butylcarbonyl, etc.), a nitro group or the like is used. The number of substituents is around 1 to 3.

As a protecting group for a carboxyl group, for example, optionally substituted C₁₋₆ alkyl (e.g. methyl, ethyl, n-propyl, i-propyl, n-butyl, tert-butyl etc.), phenyl, trityl or silyl is used. As a substituent for them, a halogen atom (e.g. fluorine, chlorine, bromine, iodine, etc.), formyl, C₁₋₆ alkyl-carbonyl (e.g. acetyl, ethylcarbonyl, butylcarbonyl, etc.), a nitro group or the like is used. The number of substituents is around 1 to 3.

As a protecting group for a hydroxy group, for example, optionally substituted C₁₋₆ alkyl (e.g. methyl, ethyl, n-propyl, i-propyl, n-butyl, tert-butyl, etc.), phenyl, C₇₋₁₀ aralkyl (e.g. benzyl, etc.), formyl, C₁₋₆ alkyl-carbonyl (e.g. acetyl, ethylcarbonyl etc.), phenyloxycarbonyl, benzoyl, C₇₋₁₀ aralkyl-carbonyl (e.g. benzylcarbonyl etc.), pyranyl, furanyl, silyl or the like is used. As a substituent for them, a halogen atom (e.g. fluorine, chlorine, bromine, iodine etc.), C₁₋₆ alkyl (e.g. methyl, ethyl, n-propyl etc.), phenyl, C₇₋₁₀ aralkyl (e.g. benzyl etc.), a nitro group or the like is used. The number of substituents is around 1 to 4.

As a method of removing a protecting group, a method known per se or the similar method is used. For example, a method of treating a protecting group with acid, base, reduction, ultraviolet-ray, hydrazine, phenylhydrazine, sodium N-methyldithiocarbamate, tetrabutylammonium fluoride, or palladium acetate is used.

The compound [I] or a salt thereof obtained by the aforementioned method can be isolated and purified by, for example, usual separation means such as recrystallization, distillation and chromatography. When the present compound [I] thus obtained is in free from, it can be converted into a salt by a method known per se or the similar method (e.g. neutralization) and, conversely, when the present compound [I] is obtained as a salt form thereof, it can be converted into the free form or another salt by a method known per se or the similar method.

When there are optical isomers of the compound [I], these individual optical isomers and a mixture thereof are included in the scope of the present invention. If desired, these isomers may be optically resolved according to a known per se means, or may be prepared individually.

Since the compound [I], a salt thereof and a prodrug thereof of the present invention (hereinafter, abbreviated as the compound of the present invention in some cases) are low toxic and safe and have lipid-rich plaque regressing activity, the compound of the present invention is useful for preventing or treating acute myocardial infarction, acute coronary syndrome such as unstable angina, peripheral artery occlusion, restenosis after percutaneous coronary plasty (PTCA), restenosis after stent placement, myocardial infarction, ischemic heart failure such as angina, arteriosclerosis, intermittent claudication, cerebral apoplexy (e.g. cerebral infarction, cerebral embolus, cerebral hemorrhage), lacnar infarction, cerebral vascular dementia, and the like of a mammal (e.g. mouse, rat, rabbit, dog, cat, cow, pig, monkey, human, etc.), and are useful as a defoaming agent.

Further, the compound of the present invention has ACAT inhibitory activity (preferably, macrophage ACAT inhibitory activity, subtype 1 ACAT inhibitory activity), and can be used as a safe prophylactic or therapeutic agent against hypercholesterolemia, hypertriglyceridemia, hyperlipemia, atherosclerosis and diseases derived therefrom (e.g. ischemic heart failure such as myocardial infarction, and cerebral vascular disorder such as cerebral infarction or cerebral apoplexy) in a mammal (e.g. mouse, rat, rabbit, dog, cat, cow, pig, monkey, human etc.).

The present invention also provides an agent for regressing, suppressing progression of or stabilizing an arteriosclerotic lesion, which contains the present compound. Such an agent for regressing, suppressing progression of or stabilizing an arteriosclerotic lesion is preferably used in combination with a HMG-CoA reductase inhibitor.

The compound of the present invention can be also used as a prophylactic or therapeutic agent against Alzheimer's disease, multiple risk syndrome and metabolism syndrome.

In treatment of these diseases, the compound of the present invention may be used alone or may be used in combination with other pharmaceutical components including other lipid lowering agents or cholesterol lowering agents, myocardial protecting agents, coronary disease treating agents, diabetes treating agents, thyroid dysfunction treating agents, nephrotic syndrome treating agents, osteoporosis treating agents and chronic renal failure treating agents. In this case, each of these compounds is preferably administered as an oral formulation or, if necessary, may be administered in a form of a suppository as a rectal formulation. In this case, examples of a possible component to be combined include fibrates [e.g. clofibrate, bezafibrate, gemfibrosil, fenofibrate, Wy-1463, GW9578 etc.], nicotinic acid, derivatives and analogues thereof [e.g. acipimox and probcol], bile acid-binding resin [e.g. cholestyramine, cholestipol etc.], cholesterol absorption suppressing compounds [e.g: sitosterol, neomycin etc.], cholesterol biosynthesis inhibiting compounds [e.g. HMG-CoA reductase inhibitor such as lovastatin, simvastatin, pravastatin, fluvastatin, atrovastatin, pitavastatin, rosuvastatin etc.], squalene epoxidase inhibitors [e.g. NB-598 and analogues etc.], and HDL increasing agents due to inhibition of cholesterol ester transporting protein [JTT-705, CP-529-414 etc.].

Still other possible components to be combined are oxidosqualine-lanosterol cyclase, for example, a decalin derivative, an azadecalin derivative and an indane derivative.

In addition, when combining with:
diabetes treating agent [actos, losiglitazon, kinedak, penfill, humalin, euglucon, glimicron, daonil, novolin, monotard, insulins, glucobay, dimelin, rastinon, bacilcon, deamelin S, iszilins, biguanide agent]; thyroid dysfunction treating agent [dried thyroid gland (thyreoid), levothyroxine sodium (thyradin-S), liothyronidin sodium (thyronine, Thyronamin);
nephrotic syndrome treating agent: prednisolone (predonine), prednisolone succinate sodium (predonine), methylprednisolone succinate sodium (Solu-Medrol), betamethasone (rinderon)]; anti-coagulating agent [dipyridamole (Persantin), dilazep dihydrochloride (cornelian), ticlopidine, clopidogrel, FXa inhibitor]; chronic renal failure treating agent [diuretics [e.g. furosemide (Lasix), bumetanide (lunetoron), azosemide (diart)], depressor (e.g. ACE inhibitor (enalapril maleate (renivase)) and Ca antagonist (manidipine), α-receptor blocker, angiotensin II receptor antagonist (candesartan cilexetil); an oral administration is preferred.

In view of lipid-rich plaque regressing activity and ACAT inhibitory activity, the compound of the present invention is suitable for preventing and treating thrombus formation. For this purpose, the compound of the present invention is administered alone or in combination with the following known treating agents, preferably via an oral route:
thrombus formation preventing or treating agent: anticoagulating inhibitor [e.g. heparin sodium, heparin potassium, warfarin potassium (warfarin), Xa inhibitor], thrombolytic agent [e.g. tPA, urokinase], anti-platelet agent [e.g. aspirin, sulfinpyrazone (anturan), dipyridamole (persantin), ticlopidine (panaldine), cilostazol (pletaal), GPIIb/IIIa antagonist (ReoPro), clopidogrel];
coronary vasodilating agent: nifedipine, diltiazem, nicorandil, nitrous acid agent;
myocardial protecting agent: cardiac ATP-K opener, endothelin antagonist, urotensin antagonist, or the like.

The compound of the present invention may be also used, against the above-mentioned diseases, in combination with a biological preparation (e.g. antibody, vaccine preparation etc.) or as combined therapy in combination with genetic therapy or the like. Examples of the antibody and the vaccine preparation include, in addition to a vaccine preparation against angiotensin II, a vaccine preparation CETP, a CETP antibody, a TNF α antibody, an antibody against other cytokine, an amyloid β vaccine preparation, and 1-type diabetes vaccine (DIAPEP-277 of Peptor, etc.), an antibody or a vaccine preparation against cytokine, renin or angiotensin enzyme and a product thereof, an antibody or a vaccine preparation against an enzyme and a protein involved in blood lipid metabolism, an antibody or a vaccine against an enzyme and a protein involved in a coagulation or fibrinolysis system in blood, and an antibody or a vaccine preparation against a protein involved in saccharide metabolism or insulin resistance. Examples of genetic therapy include therapy using a gene relating to cytokine, a renin or angiotensin enzyme and a product thereof, therapy using DNA decoy such as NFκB decoy, therapy using antisense, therapy using a gene relating to an enzyme and a protein involved in blood lipid metabolism (e.g. gene relating to metabolism, excretion and absorption of cholesterol, triglyceride, HDL-cholesterol or blood phospholipid), therapy using a gene relating to an enzyme and a protein (e.g. growth factors such as HGF and VEGF) involved in vascularization therapy directed to peripheral vessel obstruction or the like, therapy using a gene relating to a protein involved in saccharide metabolism or insulin resistance, and antisense against cytokine such as TNF. Alternatively, the compound of the present invention can be also used in combination with vascularization therapy utilizing various organs regeneration such as heart regeneration, kidney regeneration, pancreas regeneration and vessel regeneration or transplantation of marrow cells (marrow mononuclear cell, marrow stem cell etc.).

The compound of the present invention can be used orally or parenterally by injection, drip, inhalation rectal administration or local administration and can be used as it is, or as a pharmaceutical composition (e.g. powders, granules, tablets, pills, capsules, injections, syrups, emulsions, elixirs, suspensions, solutions etc.). That is, at least one of the compounds of the present invention can be used alone, or as a mixture with a pharmaceutically acceptable carrier (adjuvant, excipient, additive, and/or diluent).

A pharmaceutical composition can be formulated according to a conventional method. Such a formulation can be usually prepared by mixing/kneading an active component with additives such as an excipient, a diluent, a carrier and the like. Herein, a parenteral administration includes subcutaneous injection, intravenous injection, intramuscular injection, intraperitoneal injection and dripping infusion. A formulation for injection, for example, a sterile injection aqueous suspension or oily suspension can be prepared using a suitable dispersing agent or wetting agent and a suspending agent by a method known in the art. The sterile formulation for injection may be a sterile injectable solution or suspension in a diluent or a solvent which is non-toxic and can be administered parenterally, such as an aqueous solution. Examples of an acceptable vehicle or solvent which can be used include water, Ringer's solution and isotonic saline. As a solvent or a suspending solvent, a aseptic non-volatile oil can be also used usually. For such purpose, any non-volatile oil or fatty acid can be used, including natural or synthetic or semi-synthetic fatty oil and fatty acid, as well as natural or synthetic or semi-synthetic mono- or di- or triglycerides.

A suppository for rectal administration can be prepared by mixing an active ingredient with a suitable non-stimulating additive, for example, a substance which is solid at a normal temperature but is liquid at the temperature of intestinal tract to melt in a rectum whereby releasing the active ingredient, such as cacao butter and polyethylene glycols.

It is also effective to combine with a suitable base (e.g. polymer of butyric acid, polymer of glycolic acid, copolymer of butyric acid-glycolic acid, a mixture of a polymer of butyric acid and a polymer of glycolic acid, polyglycerol fatty acid ester etc.) to obtain a sustained-release formulation.

Examples of a solid dosage form for oral administration include the aforementioned powders, granules, tablets, pills, and capsules. Formulation with such a dosage form can be prepared by mixing and/or kneading an active ingredient compound with at least one additive, for example, sucrose, lactose, cellulose, mannitol (D-mannitol), maltitol, dextran, starches (e.g. cornstarch), microcrystalline cellulose, agar, alginates, chitins, chitosans, pectins, tragacanth gums, gum arabic, gelatins, collagens, casein, albumin, synthetic or semi-synthetic polymers or glycerides. Such a dosage form can contain further additives as usual, including inert diluents, lubricants such as magnesium stearate, preservatives such as parabens and sorbic acid, antioxidant such as ascorbic acid, α-tocopherol and cysteine, disintegrants (e.g. croscarmellose sodium), binders (e.g. hydroxypropyl cellulose), thickening agents, buffering agents, sweeteners, flavoring agents and perfumes. Tablets and pills may be also enteric coated. Examples of oral liquid formulations include pharmaceutically acceptable emulsions, syrups, elixirs, suspensions and solutions, which may contain inert diuents which are conventionally used in the art, for example, water and, if necessary, additives. Such oral liquid formulations can be prepared by the conventional method, for example, by mixing an active ingredient, an inert diluent and, if necessary, other additives. An oral formulation usually contain about 0.01 to 99W%, preferably about 0.1 to 90W%, normally about 0.5 to 50W% of the active ingredient compound of the present invention, though the amount may vary depending on the dosage form.

The dose for a certain patient is determined depending on the age, body weight, general condition, sex, diet, administration time, administration mode, excretion rate, drug combination, and a degree of the disease treated currently as well as other factors.

A lipid-rich plaque regressing agent containing the compound of the present invention is low toxic, and can be used safely. Its daily dose varies depending on the condition and body weight of a patient, the type of the compound, the administration route and the like and, for example, when used as a prophylactic or therapeutic agent against hyperlipemia, it may be about 1 to 500 mg, preferably about 10 to 200 mg as an active ingredient [I] in an oral formulation, and about 0.1 to 100 mg, preferably about 1 to 500 mg, usually about 1 to 20 mg as an active ingredient [I] in a parenteral formulation for an adult (about 60 kg). No toxity is observed in these ranges.

The present invention also provides:
(1) a pharmaceutical composition comprising the compound of the present invention with a concomitant drug (hereinafter, abbreviated as a concomitant formulation),
(2) a method for regressing lipid-rich plaque or a method for inhibiting ACAT, which comprises administering a combination of an effective amount of the compound of the present invention and an effective amount of a concomitant drug to a mammal, and
(3) a method for preventing or treating acute myocardial infarction, acute coronary syndrome such as unstable angina, peripheral artery occlusion, restenosis after percutaneous coronary plasty (PTCA), restenosis after stent placement, atherosclerosis, myocardial infarction, ischemic heart failure such as angina, arteriosclerosis, intermittent claudication, cerebral vascular disorder such as cerebral apoplexy (e.g. cerebral infarction, cerebral embolus, cerebral hemorrhage), lacnar infarction, cerebral vascular dementia, Alzheimer's disease, multiple risk syndrome and metabolism syndrome, hyperlipemia, hypercholestorolemia, hypertriglyceridemia or thrombus formation, which comprises administering a combination of an effective amount of the compound of the present invention and an effective amount of a concomitant drug to a mammal.

Examples of a concomitant drug which can be used with the compound of the present invention include the aforementioned pharmaceutical components other than the compound of the present invention and other hyperlipemia treating agent, a diuretic, a hypertension treating agent, a cardiac failure treating agent, an arrhythmia treating agent, an anti-coagulant, an anti-platelet agent, a diabetes treating agent, a HDL increasing agent, an unstable plaque stabilizing agent, a vasodilator, an vasoconstrictor, a vasopressor, an antibacterial agent, an antifungal agent, non-steroidal antiinflammatory agent, a steroidal agent, an immunoregulator, an antiprotozoal agent, an anti-ulcer agent, an antitussive or expectorant, a sedative, an anesthetic, an antianxiety agent, an antipsychotic agent, a muscle relaxant, an antiepilepsy agent, an antidepressant, a narcotic antagonist, an antitumor agent, an anti-allergic agent, a vitamin, a vitamin derivative, a bone-calcium metabolizing agent, an osteoporosis treating agent, an arthritis treating agent, an anti-rheumatic agent, an anti-asthmatic agent, a pollakiuria or urin incontinence treating agent, a renal failure or nephropathy treating agent, an atopic dermatitis treating agent, an allergic rhinitis treating agent, an endotoxin antagonist or antibody, a signal transmission inhibitor, an inflammatory mediating effect inhibitor, an inflammatory mediating effect inhibiting antibody, an antiinflammatory mediating effect inhibitor, and an antiinflammatory mediating effect inhibiting agent. Inter alia, a hyperlipemia treating agent, a diuretic, a hypertension treating agent, a cardiac failure treating agent, an arrhythmia treating agent, an anti-coagulant, an anti-platelet agent, a diabetes treating agent, a HDL increasing agent, and an unstable plaque stabilizing agent are preferable. Examples of a concomitant drug other than the aforementioned pharmaceutical components are specifically listed below:
(1) Hyperlipemia treating agent
   HMG-CoA reductase inhibitor (e.g. fluvastatin, cerivastatin, atorvastatin etc.), fibrates (e.g. simfibrate, clofibrate aluminium, clinofibrate, fenofibrate etc.), anion exchange resin (e.g.cholestylramide etc.), nicotinic acid formulation (e.g. nicomol, niceritrol, tocopherol nicotinate etc.), polyvalent unsaturated fatty acid derivative (e.g. ethyl icosapentate, polyene phosphatidylcholine, melinamide etc.), vegetable sterol (e.g. gamma-oryzanol, soysterol etc.), elastase, sodium dextran sulfate, squalene synthetase inhibitor, CETP inhibitor, ethyl 2-chloro-3-[4-(2-methyl-2-phenylpropoxy)phenyl]propionate [Chem. Pharm. Bull], 38, 2792-2796(1990)].
(2) Diuretic
   thiazide diuretic (benzylhydro-chorothiazide, cyclopenthiazide, ethiazide, hydrochlorothiazide, hydroflumethiazide, methyclothiazide, penfluthiazide, polythiazide, trichloromethiazide etc.), loop diuretic (chlortalidone, clofenamide, indapamide, mefruside, meticrane, sotolazone, tripamide, quinethazone, metolazole, furosemide, mefruside etc.), potassium retaining diuretic (spironolacton, triamterene etc.).
(3) Hypertension treating agent
   [1] Sympathetic nerve suppressant
      α₂ stimulant (e.g. clonidine, guanabenz, guanfacine, methyldopa etc.), ganglionic blocking agent (e.g. hexamethonium, trimethaphan etc.), presynaptic blocker (e.g. alseroxylon, dimethylaminoreserpinate, rescinamine, reserpine, syrosingopine etc.), neuron blocker (e.g. betanidine, guananethidine etc.), α₁ blocker (e.g. bunazosin, doxazocin, prazosin, terazosin, urapidil etc.), β blocker (e.g. propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.).
   [2] Vasodilator
      calcium channel antagonist (e.g. manidipine, nicardipine, nilvadipine, nisoldipine, nitrendipine, benidipine, amlodipine, aranidipine etc.), phthalazine derivative (e.g. budralazine, cadralazine, ecarazine, hydralazine, todralazine etc.).
   [3] ACE inhibitor
      alacepril, captopril, cilazapril, delapril, enalapril, lisinopril, temocapril, trandolapril, quinapril, imidapril, benazepril, perindopril etc.
   [4] Angiotensin II receptor antagonist
      losartan, candesartan, valsartan, telmisartan, irbesartan, forasartan etc.
   [5] Diuretic (e.g. the aforementioned diuretics)
(4) Cardiac failure treating agent
   cardiotonic agent (e.g. digitoxin, digoxin, methyldigoxin, lanatoside C, proscillaridine), α,β-stimulant (e.g. epinephrine, norepinephrine, isoproterenol, dopamine, docarpamine, dobutamine, denopamine etc.), phosphodiesterase inhibitor (e.g. amrinone, milrinone, olprinone hydrochloride etc.), calcium channel sensitivity promoter (e.g. pimobendan etc.), nitrate agent (e.g. nitroglycerin, isosorbide nitrate etc.), ACE inhibitor (e.g. the aforementioned ACE inhibitors etc.), diuretic (e.g. the aforementioned diuretics etc.), carperitide, ubidecarenone, vesnarinone, aminophylline etc.).
(5) Arrhythmia treating agent
   sodium channel blocker (e.g. quinidine, procainamide, disopyramide, ajimaline, cibenzoline, lidocaine, diphenyl hydantoin, mexiletine, propafenone, flecainide, pilsicanide, phenytoin etc.), β-blocker (e.g. propranolol, alprenolol, bufetolol, oxprenolol, atenolol, acebutolol, metoprolol, bisoprolol, pindolol, carteolol, arotinolol etc.), potassium channel blocker (e.g. amiodarone etc.), calcium channel blocker (e.g. verapamil, diltiazem etc.) etc.
(6) Anticoagulant and antiplatelet agent
   sodium citrate, activated protein C, tissue factor pathway inhibitor, anti-thrombin III, dalteparin sodium, argatroban, gabexate, sodium ozagrel, ethyl icosapentate, beraprost sodium, alprostadil, pentoxifylline, tisokinase, streptokinase etc.
(7) Diabetes treating agent
   sulfonyl urea (e.g. tolbutamide, chlorpropamide, glycopyramide, acetohexamid, tolazamide, glibenclamide, glybuzole etc.), biguanide (e.g. metformin hydrochloride, buformin hydrochloride etc.), α-glucosidase inhibitor (e.g. voglibose, acarbose etc.), insulin sensitizer (e.g. pioglitazone, troglitazone etc.), insulin, glucagon, diabetic complication treating agent (e.g. epalrestat etc.) etc.
(8) HDL increasing agent
   squalene synthetase inhibitor, CETP inhibitor, LPL activator etc.
(9) Unstable plaque stabilizing agent
   MMP inhibitor, kinase inhibitor etc.
(10) Vasodilator
   oxyphedrine, diltiazem, tolazoline, hexobendine, bamethan, clonidine, methyldopa, guanabenz etc.
(11) Vasoconstrictor
   dopamine, dobutamine, denopamine etc.
(12) Hypertensive agent
   dopamine, dobutamine, denopamine, digitoxin, digoxin, methyldigoxin, lanatoside C, G-Strophantin etc.
(13) Antibacterial agent
   [1] Sufonamide
      sulfamethizole, sulfisoxazole, sulfamonomethoxin, sulfamethizole, salazosulfapyridine, silver sulfadiazine etc.
   [2] Quinolone
      nalidixic acid, pipemidic acid trihydrate, enoxacin, norfloxacin, ofloxacin, tosufloxacin, tosilate, ciprofloxacin hydrochloride, lomefloxacin hydrochloride, sparfloxacin, fleroxacin etc.
   [3] Anti-tuberculous agent
      isoniazid, ethambutol (ethambutol hydrochloride), p-aminosalicylic acid (calcium p-aminosalicylate), pyrazinamide, ethionamide, prothionamide, rifampicin, streptomycin sulfate, kanamycin sulfate, cycloserine etc.
   [4] Anti acid-fast bacteria agent
      diaphenylsulfone, rifampicin etc.
   [5] Anti-viral agent
      idoxuridine, acyclovir, vidarabine, ganciclovir etc.
   [6] Anti-HIV agent
      zidovudine, didanosine, zalcitabine, indinavir sulfate ethanol adduct, ritonavir etc.
   [7] Anti-spirochete agent
   [8] Antibiotic
      tetracyclin hydrochloride, ampicillin, piperacillin, gentamycin, dibekacin, kanendomycin, lividomycin, tobramycin, amikacin, fradiomycin, sisomicin, tetracyclin, oxytetracyclin, rolitetracyclin, doxycyclin, ampicillin, piperacillin, ticarcillin, cefalotin, cefapirin, cefaloridine, cefaclor, cefalexin, cefroxadine, cefadroxil, cefamandole, cefotoam, cefroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazon, ceftizoxime, moxalactam, thienamycin, sulfazecin, azthreonam or a salt thereof, griseofulvin, lankacidin [J. Antibiotics, 38, 877-885(1985)] etc.
(14) Antifungal agent
   [1] Polyethylene-based antibiotic (e.g. amphotericin B, nystatin, trichomycin)
   [2] Griseofulvin, pyrrolnitrin etc.
   [3] Cytosine metabolism antagonist (e.g. flucytosine)
   [4] Imidazole derivative (e.g. econazole, clotrimazole, miconazole nitrate, bifonazole, croconazole)
   [5] Triazole derivative (e.g. fluconazole, itraconazole, azole-based compound [2-[(1R, 2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl-3-(2H, 4H)-1,2,4-triazolone]
   [6] Thiocarbamic acid derivative (e.g. trinaphthol)
   [7] Echinocandin-based derivative (e.g. caspofamgine, FK-463, V-Echinocadin) etc.
(15) Non-steroidal antiinflammatory agent
   acetaminophen, fenasetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprodin, flurbiprofen, fenbufen, pranoprofen, floctafenine, epirizol, tiaramide hydrochloride, zaltoprofen, gabexate mesilate, camostat mesilate, ulinastatin, colchicine, probenecid, sulfinpyrazone, benzbromarone, allopurinol, sodium gold thiomalate, sodium hyaluronate, sodium salicylate, morphine hydrochloride, salicylic acid, atropine, scopolamine, morphine, pethidine, levorphanol; ketoprofen, naproxen, oxymorphone or a salt thereof.
(16) Steroidal agent
   dexamethasone, hexestrol, methimazole, betamethasone, triamcinolone, triamcinolone acetonide, fluorocinonide, fluorocinolone acetonide, prednisolone, methylprednisolone, cortisone acetate, hydrocortisone, fluorometholone, beclometasone dipropionate, estriol etc.
(17) Immunoregulating agent
   cyclosporin, tacrolimus, gusperimus, azathioprine, anti-lymph serum, dried sulfonated-immunogloburin, erythropoietin, colony stimulating factor, interleukin, interferon etc.
(18) Antiprotozoal agent
   metronidazole, tinidazole, diethylcarbamadine citrate, quinine hydrochloride, quinine sulfate etc.
(19) Anti-ulcer agent
   metoclopramide, histidine hydrochloride, lansoprazole, metoclopramide, pirenzepine, cimetidine, ranitidine, famotidine, urogastrine, oxethazaine, proglumide, omeprazole, sucralfate, sulpiride, cetraxate, gefarnate, aldioxa, teprenone, prostaglandin etc.
(20) Bronchospasmolytic expectorant
   ephedrine hydrochloride, noscapine hydrochloride, codeine phosphate, dihydrocodeine phosphate, isoproterenol hydrochloride, ephedrine hydrochloride,' methylephedrine hydrochloride, noscapine hydrochloride, aroclamide, chlorfesianol, picoperidamine, cloperastine, protokylol, isoproterenol, sulbutamol, terbutaline, oxymetebanol, morphine hydrochloride, dextromethorphan hydrobromide, oxycodone hydrochloride, dimemorfan phosphate, tipepidine hibenzate, pentoxyverine citrate, clofedanol hydrochloride, benzonatate, guaifenesin, bromhexine hydrochloride, ambroxol hydrochloride, acetylcysteine, ethylcysteine hydrochloride, carbocysteine etc.
(21) Sedative
   chlorpromazine hydrochloride, atropine sulfate, phenobarbital, barbital, amobarbital, pentobarbital, thiopental sodium, thiamylal sodium, nitrazepam, estazolam, flunitrazepam, haloxazolam, triazolam, flunitrazepam, bromovalerylurea, chloral hydrate, triclofos sodium etc.
(22) Anesthetic
   (22-1) Local anesthetic
      cocaine hydrochloride, procaine hydrochlodie, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine etc.
   (22-2) Systemic anesthetic
      [1] Inhalation anesthetic(e.g. ether, halothane, nitrous oxide, enflurane, enflurane),
      [2] Intravenous anesthetic (e.g. ketamine, droperidol, thiopental sodium, thiamylal sodium, pentobarbital) etc.
(23) Anxiolytic agent
   diazepam, lorazepam, oxazepam, clordiazepoxide, medazepam, oxazolam, cloxazolam, clotiazepam, prazepam, etizolam, fludiazepam, hydroxyzine etc.
(24) Antipsychotic agent
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clomipramine hydrochloride, sulpiride, zotepine etc.
(25) Muscle relaxant
   pridinol, tubocurarine, pancuronium, tolperisone hydrochloride, chlorphenesin carbamate, baclofen, chlormezanone, mephenesin, chlozoxazone, eperisone, tizanidine, etc.
(26) Antietileptic agent
   phenytoin, ethosuximide, acetazolamide, chlordiazepoxide, trimethadione, carbamazepine, phenobarbital, primidone, sulthiam, sodium valproate, clonazepam, diazepam, nitrazepam etc.
(27) Antidepressant
   imipramine, clomipramine, noxiptiline, pheneridine, amitriptyline hydrochloride, nortriptyline, amoxapine, mianserin hydrochloride, maprotiline hydrochloride, sulpiride, fluvoxamine maleate, trazodone hydrochloride etc.
(28) Anesthetic antagonist
   levallorphan, nalorphine, naloxone or a salt thereof etc.
(29) Antitumor agent
   6-O-(N-Chloroacetylcarbamoyl), fumagilol, bleomycin, methotrexate, actinomycin D, mitomycin C, daunorubicin, adriamycin, neocarcinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, zisulphan, thiotepa, procarbazine hydrochloride, cisplatin, azathioprine, mercaptoprine, tegafur, carmofur, cytarabine, methyltestosterone, testosterone propionate, testosterone enanthate, mepitiostane, fosfestrol, clormadinone acetate, leuproline acetate, buserelin acetate etc.
(30) Anti-allergic agent
   diphenhydramine, chlorphenyramine, tripelennamine, methodiramine, clemizole, diphenylpyraline, methoxyphenamine, sodium cromoglicate, tranilast, repirinast, amlexanox, ibudilast, ketotifen, terfenadine, mequitazine, azelastine, epinastine, ozagrel hydrochloride, pranlukast hydrate, seratrodast, etc.
(31) Lipid-soluble vitamin
   [1] Vitamin A: vitamin A₁, vitaminA₂ and retinol palmitate
   [2] Vitamin D: vitamin D₁, D₂, D₃, D₄ and D₅
   [3] Vitamin E: α-tocopherol, β-tocopherol, γ-tocopherol, δ-tocopherol, dl-α-tocopherol nicotinate
   [4] Vitamin K: vitamin K₁, K₂, K₃ and K₄
   [5] Folic acid (vitamin M) etc.
(32) Vitamin derivative
   various vitamin derivatives, for example, vitamin D₃ derivative including 5,6-trans-cholecalciferol, 2,5-hydroxycholecalciferol and 1-α-hydroxycholecalciferol, vitamin D₂ derivative including 5,6-trans-ergocalciferol etc.
(33) Anti-asthmatic agent
   isoprenaline hydrochloride, salbutamol sulfate, procaterol hydrochloride, terbutaline sulfate, trimetoquinol hydrochloride, tubobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlukast hydrate, seratrodast, dexamethasone, prednisolone, hydrocortisone, beclometaason dipropionate etc.
(34) Pollakiuria or urine incontinence treating agent
   flavoxate hydrochloride etc.
(35) Atopic dermatitis treating agent
   sodium cromoglicate etc.
(36) Allergic rhinitis treating agent
   sodium cromoglicate, chlorphenyramine maleate, alimemazine tartrate, clemastine fumarate, homochlorcyclizine hydrochloride, terfenadine, mequitazine etc.
(37) Dementia treating agent
   acetylcholine estrase inhibitor (e.g. donepezil, tacrine, rivastigmine, galanthamine etc.) etc.
(38) Others
   hydroxycam, diaserine, megestrol acetate, nicergoline, prostaglandins etc.

By means of a combination of the compound of the present invention with a concomitant drug, for example, the following effects are exerted.
(1) The dose or side effects of the compound of the present invention, a salt thereof or a prodrug thereof and a concomitant drug can be lower than those when given alone.
(2) A synergistic therapeutic effect can be obtained against diseases such as acute myocardial infarction, acute coronary syndrome such as unstable angina, peripheral artery occlusion, restenosis after percutaneous coronary plasty (PTCA), restenosis after stent placement, hypercholestorolemia, atherosclerosis, myocardial infarction, ischemic heart failure such as angina, cerebral vascular disorder such as cerebral apoplexy or cerebral infarction, Alzheimer's disease or thrombus formation.
(3) A wide therapeutic effect can be obtained against various diseases accompanied with diseases such as acute myocardial infarction, acute coronary syndrome such as unstable angina, peripheral artery occlusion, restenosis after percutaneous coronary plasty (PTCA),.restenosis after stent placement, hypercholestorolemia, atherosclerosis, myocardial infarction, ischemic heart failure such as angina, cerebral vascular disorder such as cerebral apoplexy or cerebral infarction, Alzheimer's disease or thrombus formation.

When using the concomitant formulation of the present invention, the timing of administering the compound of the present invention and a concomitant drug are is limited, and the compound of the present invention or its pharmaceutical composition and a concomitant drug or its pharmaceutical composition may be administered at the same time, or may be administered at a certain time interval to a subject. The dose of a concomitant drug may be in accordance with to a clinically used dose, and can be appropriately selected depending on an administration subject, an administration route, disease, a combination and the like.

The administration mode of the concomitant formulation of the present invention is not particularly limited, and it is enough that the compound of the present invention and a concomitant drug are combined upon administration. Examples of such administration mode include (1) administration of a single formulation obtained by formulating the compound of the present invention and a concomitant drug simultaneously, (2) simultaneous administration of two formulations obtained by formulating the compound of the present invention and a concomitant drug separately, via an identical route, (3) sequential and intermittent administration of two formulations obtained by formulating the compound of the present invention and a concomitant drug separately, via an identical route, (4) simultaneous administration of two formulations obtained by formulating the compound of the present invention and a concomitant drug separately, via different routes, (5) sequential and intermittent administration of two for mualtions obtained by formulating the compound of the present invention and a concomitant drug separately, via different routes (e.g. the compound of the present invention or its pharmaceutical composition followed by a concomitant drug or its pharmaceutical composition, or inverse order) and the like.

The concomitant formulation of the present invention is low toxic, and thus the compound of the present invention and/or a concomitant drug describe above are mixed with a pharmacologically acceptable carrier according to a method known per se to form a pharmaceutical composition such as tablets (including sugar-coated tablets and film coating tablets), powders, granules, capsules (including soft capsules), solutions, injections, suppositories, sustained-release formulations and the like, which can be safely given orally or parenterally (e.g. topically, rectally, intravenously). Injections can be administered intravenously, intramuscularly, subcutaneously, into organs, or directly into lesions.

Examples of the pharmacological acceptable carrier which may be used in preparing the concomitant formulation of the present invention include various organic or inorganic carrier materials which are conventionally used as pharmaceutical materials, for example, excipients, lubricants, binders and disintegrants in a solid formulation, or solvents, dissolution aids, suspending agents, isotonizing agents, buffering agents and soothing agents in a liquid formulation. Further, if necessary, usual additives such as preservatives, antioxidants, coloring agents, sweeteners, adsorbents, wetting agents and the like may be conveniently added in suitable amounts.

Examples of excipients include lactose, white sugar, D-mannitol, starch, cornstarch, crystalline cellulose, light anhydrous silicic acid and the like.

Examples of lubricants include magnesium stearate, calcium stearate, talc, colloidal silica and the like.

Examples of binders include crystalline cellulose, white sugar, D-mannitol, dextrin, hydroxypropyl cellulose, hydroxypropylmethyl cellulose, polyvinylpyrrolidone, starch, sucrose, gelatin, methyl cellulose, sodium carboxymethyl cellulose and the like.

Examples of disintegrats include starch, carboxymethyl cellulose, potassium carboxymethyl cellulose, sodium carboxymethyl starch, L-hydroxypropyl cellulose and the like.

Examples of solvents include water for injection, alcohol, propylene glycol, macrogol, sesame oil, corn oil, olive oil and the like.

Examples of dissolution aids include polyethylene glycol, propylene glycol, D-mannitol, benzyl benzoate, ethanol, trisaminomethane, cholesterol, triethanolamine, sodium carbonate, sodium citrate and the like.

Examples of suspending agents include surfactants such as stearyltriethanolamine, sodium laurylsulfate, laurylaminopropionic acid, lecithin, benzalkonium chloride, benzethonium chloride, glycerin monostearate and the like; hydrophilic polymers such as polyvinyl alcohol, polyvinylpyrrolidone, sodium carboxymethyl cellulose, methyl cellulose, hydroxymethyl cellulose, hydroxyethyl cellulose, hydroxypropyl cellulose and the like.

Examples of isotonizing agents include glucose, D-sorbitol, sodium chloride, glycerin, D-mannitol and the like.

Examples of buffering agents include buffer solutions of phosphate, acetate, carbonate, citrate and the like.

Examples of soothing agents include benzyl alcohol and the like.

Examples of preservatives include p-hydroxybenzoate, chlorobutanol, benzyl alcohol, phenethyl alcohol, dehydroacetic acid, sorbic acid and the like.

Examples of antioxidants include sulfite, ascorbic acid, α-tocopherol and the like.

The ratio between the compound of the present invention and a concomitant drug in the concomitant formulation of the present invention may be selected appropriately depending on an administration subject, an administration route, disease and the like.

For example, the content of the compound of the present invention in the concomitant formulation of the present invention varies depending on the dosage form, and is usually about 0.01 to 100% by weight, preferably about 0.1 to 50% by weight, more preferably about 0.5 to 20% by weight of the entire formulation.

The content of a concomitant drug in the concomitant formulation of the present invention varies depending on the dosage form, and is usually about 0.01 to 100% by weight, preferably about 0.1 to 50% by weight, more preferably about 0.5 to 20% by weight of the entire formulation.

The content of additives such as a carrier and the like in the concomitant formulation of the present invention varies depending on the dosage form, and is usually about 1 to 99.99% by weight, preferably about 10 to 90% by weight of the entire formulation.

In addition, the same content may be used also when the compound of the present invention and a concomitant drug are formulated separately.

Such a formulation can be prepared,by a method known per se which is used generally in a pharmaceutical process.

For example, the compound of the present invention or a concomitant drug can be formulated with a dispersant (e.g. Tween 80 (manufactured by Atlas Powder, USA), HCO60 (manufactured by Nikko Chemical), polyethylene glycol, carboxymethyl cellulose, sodium alginate, hydroxypropylmethyl cellulose, dextrin etc.), a stabilizer (e.g. ascorbic acid, sodium pyrosulfite etc.), a surfactant (e.g. Polysorbate 80, macrogol etc.), a solubilizing agent (e.g. glycerin, ethanol etc.), a buffering agent (e.g. phosphoric acid and alkali metal salt thereof, citric acid and alkali metal salt thereof), an isotonizing agent (e.g. sodium chloride, potassium chloride, mannitol, sorbitol, glucose etc.), a pH modifier (e.g. hydrochloric acid, sodium hydroxide etc.), a preservative (e.g. ethyl p-hydroxybenzoate, benzoic acid, methylparaben, propylparaben, benzyl alcohol etc.), a dissolving agent (e.g. concentrated glycerin, meglumine etc.), a solubilizing aid (e.g. propylene glycol, white sugar etc.), a soothing agent (e.g. glucose, benzyl alcohol etc.) or the like into an aqueous injection, or dissolved, suspended or emulsified in a vegetable oil such as an olive oil, a sesame oil, a cottonseed oil or a corn oil, or a solubilizing aid such as propylene glycol to form oily injection, whereby producing an injection formulation.

For an oral dosage form, the compound of the present invention or a concomitant drug may be compression molded together with an excipient (e.g. lactose, white sugar, starch etc.), a disintegrant (e.g. starch, calcium carbonate etc.), a binder (e.g. starch, gum arabic, carboxymethyl cellulose, polyvinylpyrrolidone, hydroxypropyl cellulose etc.) or a lubricant (e.g. talc, magnesium stearate, polyethylene glycol 6000 etc.) according to a method known per se into the desired shape, which may be then, if necessary, coated for the purpose of taste masking, enteric property or sustained release performance according to a method known per se. As the coating agent, for example, hydoroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by Rohm, Germany, methacrylic acid-acrylic acid copolymer) and a pigment (e.g. iron oxide red, titanium dioxide etc.) are used. An oral dosage form may be a rapid release formulation or a sustained release formulation.

For a suppository, the compound of the present invention or a concomitant drug may be formulated into an oily or aqueous solid, semi-solid or liquid suppository according to a method known per se. Examples of an oily base used in the aforementioned composition include higher fatty acid glycerides [e.g. cacao butter, witepsols (manufactured by Dynamite Nobel, German) etc.], medium fatty acids (e.g. migliols (manufactured by Dynamite Nobel, German) etc.), and vegetable oils (e.g. sesame oil, soybean oil, cottonseed oil). In addition, examples of the aqueous base include polyethylene glycol and propylene glycol, and examples of the aqueous gel base include natural gums, cellulose derivatives, vinyl polymers and acrylic acid polymers.

Examples of the sustained-release formulation include a sustained-release microcapsule.

Although a sustained-release microcapsule can be obtained by a method known per se, for example, a sustained-release formulation shown in the following Section [2] is formed and administered in a preferred case.

The compound of the present invention is preferably formulated into an oral dosage form such as a solid formulation (e.g. powder, granule, tablet, capsule) or into a rectal formulation such as suppository. In particular, an oral dosage form is preferable.

A concomitant drug may be formulated into the aforementioned dosage form depending on the kind of a drug and the like.

The followings are the descriptions with regard to [1] injection formulation of the compound of the present invention or a concomitant drug and preparation thereof, [2] sustained-release formulation or rapid release formulation of the compound of the present invention or a concomitant drug and preparation thereof, [3] sublingual tablet, buccal or intraoral instantaneous disintegrating formulation of the compound of the present invention or a concomitant drug and preparation thereof, and [4] solid dispersion of the compound of the present invention or a concomitant drug and preparation thereof.

### [1] Injection formulation and preparation thereof

An injection formulation is preferably obtained by dissolving the compound of the present invention or a concomitant drug in water. Such an injection formulation may contain benzoate or/and salicylate.

The injection formulation is obtained by dissolving the compound of the present invention or a concomitant drug and, optionally, benzoate or/and salicylate in water.

Examples of the benzoate or salicylate include alkali metal salts such as sodium and potassium, alkaline earth metal salts such as calcium and magnesium, an ammonium salt, a meglumine salt, as well as a salt of an organic acid such as tromethamol.

The concentration of the compound of the present invention or a concomitant drug in an injection formulation is 0.5 to 50 w/v%, preferably around 3 to 20 w/v%. The concentration of benzoate or/and salicylate is 0.5 to 50 w/v%, preferably 3 to 20 w/v%.

The injection formulation may further contain additives which are generally used in an injection formulation, for example, a stabilizing agent (ascorbic acid, sodium pyrosulfite etc.), a surfactant (Polysorbate 80, macrogol etc.), a solubilizing agent (glycerin, ethanol etc.), a buffering agent (phosphoric acid and its alkali metal salt, citric acid and its alkali metal salt etc.), an isotonizing agent (sodium chloride, potassium chloride, etc.), a dispersant (hydroxypropylmethyl cellulose, dextrin), a pH modifier (hydrochloric acid, sodium hydroxide etc.), a preservative (ethyl p-hydroxybenzoate, benzoic acid etc.), a dissolving agent (concentrated glycerin, meglumine etc.), a solubilizing aid (propylene glycol, white sugar etc.) and a soothing agent (glucose, benzyl alcohol etc.). Such additives are generally incorporated at a ratio usually used in an injection formulation.

The pH of an injection formulation is preferably adjusted to 2 to 12, preferably 2.5 to 8.0 by addition of a pH modifier.

An injection formulation is obtained by dissolving the compound of the present invention or a concomitant drug and, optionally, benzoate or/and salicylate and, if necessary, the aforementioned additives in water. These components may be dissolved in any order as appropriate similarly to a conventional preparation for preparing an injection formulation.

An aqueous solution for injection is preferably warmed, and may be provided as an injection formulation after sterilizing by filtration or autoclave similarly to a conventional injection formulation.

An aqueous solution for injection is preferably autoclaved at 100°C to 121°C for 5 to 30 minutes.

An injection formulation may be also a solution imparted with antibacterial property, so that it can be used as a formulation for multiple-divided doses.

### [2] Sustained release or rapid release formulation and preparation thereof

A sustained release formulation in which a core comprising the compound of the present invention or a concomitant drug is, optionally, coated with a coating agent such as a water-insoluble substance or a swelling polymer is preferable. For example, a sustained release oral formulation for a single daily dose is preferable.

Examples of a water-insoluble substance used in a coating agent include cellulose ethers such as ethyl cellulose and butyl cellulose, cellulose esters such as cellulose acetate and cellulose propionate, polyvinyl esters such as polyvinyl acetate and polyvinyl butyrate, acrylic acid-based polymer such as acrylic acid/methacrylic acid copolymer, methyl methacrylate copolymer, ethoxyethyl methacrylate/cinnamoethyl methacrylate/aminoalkyl methacrylate copolymer, polyacrylic acid, polymethacrylic acid, methacrylic acid alkylamide copolymer, poly(methyl methacrylate), polymethacrylate, polymethacrylamide, aminoalkyl methacrylate copolymer, poly(methacrylic acid anhydride), glycidyl methacrylate copolymer, inter alia, Eudragit series (Rohm Pharma) such as Eudragit RS-100, RL-100, RS-30D, RL-30D, RL-PO, RS-PO (ethyl acrylate/methyl methacrylate/chlorotrimethyl methacrylate/ethyl ammonium copolymer) and Eudragit NE-30D (methyl methacrylate/ethyl acrylate copolymer), hydrogenated oils such as hydrogenated castor oil (e.g. Lovely Wax (Freund Sangyo) etc.), waxes such as carnauba wax, fatty acid glycerin ester and paraffin, polyglycerin fatty acid ester and the like.

As the swelling polymer, a polymer having an acidic leaving group and exhibiting pH-dependent swelling is preferable, and an acidic leaving group-bearing polymer that swells little in an acidic region such as 'stomach and swells extensively at a neutral region such as small intestine or large intestine is preferable.

Examples of such a polymer having a acidic leaving group and exhibiting pH-dependent swelling include crosslinked-type polyacrylic acid polymers such as Carbomer 934P, 940, 941, 974P, 980, 1342 etc., polycarbophil, calcium polycarbophil (all aforementioned polymers are manufactured by BF Goodrich), HIBIS Wako 103, 104, 105, 304 (all manufactured by Wako Pure Chemical Co., Ltd.).

A coating agent used in a sustained-release formulation may further contain a hydrophilic substance.

Examples of the hydrophilic substance include polysaccharides optionally having a sulfate group such as pullulan, dextrin and alkali metal alginate, polysaccharides having a hydroxyalkyl group or a carboxyalkyl group such as hydroxypropyl cellulose, hydroxypropylmethyl cellulose and sodium carboxymethyl cellulose, methyl cellulose, polyvinylpyrrolidone, polyvinyl alcohol, and polyethylene glycol.

The content of a water-insoluble substance in the coating agent for a sustained-release formulation is about 30 to about 90% (w/w), preferably about 35 to about 80% (w/w), more preferably about 40 to 75% (w/w), and the content of a swelling polymer is about 3 to about 30% (w/w), preferably about 3 to about 15% (w/w). The coating agent may further contain a hydrophilic substance and, in this case, the content of a hydrophilic substance in the coating agent is about 50% (w/w) or less, preferably about 5 to about 40% (w/w), more preferably about 5 to about 35% (w/w). Herein, the % (w/w) indicates a % by weight based on the coating composition which is the remainder of the coating solution after deleting any solvent (e.g. water, lower alcohol such as methanol, ethanol etc.).

A sustained-release formulation is manufactured by preparing a core containing a drug, and then coating the resulting core with a coating solution that is obtained by melting a water-insoluble substance with heating or a wetting polymer or by dissolving or dispersing a water-insoluble substance or a swelling polymer in a solvent, as exemplified below.

### I. Preparation of a core containing a drug

The form of a core containing a drug coated with a coating agent (hereinafter, simply referred to as a core in some cases) is not particularly limited, but preferably, it is a particle such as a granule or a fine particle.

When the core is a granule or a fine particle, its average particle diameter is preferably about 150 to 2,000 µm, more preferably about 500 to about 1,400 µm.

Preparation of the core can be performed by a standard method. For example, a drug is mixed with an appropriate excipient, binder, disintegrant, lubricant, stabilizer or the like and then subjected to wet extrusion granulation or fluidized bed granulation to prepare a core.

The content of a drug in a core is about 0.5 to about 95% (w/w), preferably about 5.0 to about 80% (w/w), more preferably about 30 to 70% (w/w).

An excipient to be contained in a core includes saccharides such as white sugar, lactose, mannitol and glucose, starch, crystalline cellulose, potassium phosphate, and cornstarch. Inter alia, crystalline cellulose and corn starch are preferable.

A binder includes polyvinyl alcohol, hydroxypropyl cellulose, polyethylene glycol, polyvinylpyrrolidone, Pluronic F68, gum arabic, gelatin and starch. A disintegrant includes calcium carboxymethyl cellulose (ECG505), sodium coroscarmellose (Ac-Di-Sol), crosslinked-type polyvinylpyrrolidone (crospovidone) and low-substituted hydroxypropyl cellulose (L-HPC). Inter alia, hydroxypropyl cellulose, polyvinylpyrrolidone, and low-substituted hydroxypropyl cellulose are preferable. A lubricant or a deflocculating agent includes talc, magnesium stearate and an inorganic salt thereof. A glidant includes polyethylene glycol. A stabilizer includes acids such as tartaric acid, citric acid, succinic acid, fumaric acid and maleic acid.

In addition to the aforementioned methods, the core may be also prepared by agitating granulation wherein an inert carrier particle as a seed for the core is sprayed with a binder dissolved in a suitable solvent such as water or a lower alcohol (e.g. methanol, ethanol etc.) with being supplemented portionwise with a drug or a mixture thereof with an excipient and a lubricant, as well as a pan coating method, a fluidized bed coating method and a melting granulation. An inert carrier particle includes particles made of white sugar, lactose, starch, crystalline cellulose or waxes, and an average particle diameter thereof is preferably about 100 µm to about 1,500 µm.

In order to separate a drug contained in the core from a coating agent, the surface of the core may be covered with a protective material. As the protective material, for example, the aforementioned hydrophilic substance or water-insoluble substance is used. As the protective material, preferably, polyethylene glycol or polysaccharides having a hydroxyalkyl group or a carboxyalkyl group, more preferably, hydroxypropylmethyl cellulose or hydroxypropyl cellulose is used. The protective material may contain an acid such as tartaric acid, citric acid, succinic acid, fumaric acid, maleic acid and the like as a stabilizer, or a lubricant such as talc. When a protective material is used, it is coated at a rate of about 1 to about 15% (w/w), preferably about 1 to about 10% (w/w), more preferably about 2 to about 8% (w/w) based on a core.

A protective material can be coated by a standard coating method, and specifically a core is spray-coated by a fluidized bed coating method or a pan coating method.

### II. Coating of core with coating agent

The core obtained in the above I is coated with a coating solution in which a water-insoluble substance and a pH-dependent swelling polymer as described above and a hydrophilic substance are melted with heating or are dissolved or dispersed in a solvent, to prepare a sustained release formulation.

Examples of a method of coating a core with a coating solution include a spray-coating method.

The ratio between a water-insoluble substance, a swelling polymer and a hydrophilic substance in a coating solution may be appropriately selected so that the content of each ingredient in a coating becomes the aforementioned content.

The rate of a coating agent is about 1 to about 90% (w/w), preferably about 5 to about 50% (w/w), more preferably about 5 to 35% (w/w) based on the core (excluding a protective material coating).

As a solvent for a coating solution, water or an organic solvent may be used alone, or a mixture of the both may be used. Upon use of a mixed solvent, the ratio between water and an organic solvent (water/organic solvent: weight ratio) may vary in the range of 1 to 100%, preferably 1 to about 30%. The organic solvent is not particularly limited as far as it dissolves a water-insoluble substance. For example, lower alcohols such as methyl alcohol, ethyl alcohol, isopropyl alcohol, n-butyl alcohol and the like, lower alkanones such as acetone, acetonitrile, chloroform, methylene chloride, or the like may be used as the organic solvent. Among them, lower alcohols are preferable, and ethyl alcohol and isopropyl alcohol are particularly preferable. Water and a mixture of water and an organic solvent are preferably used as a solvent for a coating agent. In such a case, if needed, an acid such as tartaric acid, citric acid, succinic acid, fumaric acid and maleic acid may be added to a coating solution in order to stabilize the coating solution.

When the coating is performed by spray-coating, a standard coating method can be used, and specifically, a coating solution is spray-coated on a core by a fluidized bed coating method, a pan coating method or the like. Upon this, if needed, talc, titanium dioxide, magnesium stearate, calcium stearate or light anhydrous silicic acid as a lubricant, or glycerin fatty acid ester, hydrogenated castor oil, triethyl citrate, cetyl alcohol or stearyl alcohol as a plasticizer may be added.

After coating with a coating agent, an antistatic agent such as talc may be also incorporated therein if necessary.

An instantaneous release formulation may be liquid (solution, suspension, emulsion etc.) or solid (particle, pill, tablet etc.). An oral formulation and a parenteral formulation such as an injection are used, and an oral formulation is preferable.

An instantaneous release formulation may usually contain carriers, additives or excipients (hereinafter, abbreviated as an excipient in some cases) which are conventionally used in pharmaceutical field in addition to a drug which is an active ingredient. A pharmaceutical excipient used is not particularly limited as far as it is an excipient which is usually used as a pharmaceutical excipient. Examples of an excipient for oral solid formulation include lactose, starch, cornstarch, crystalline cellulose (Avicel PH101 manufactured by Asahi Kasei), powdery sugar, granulated sugar, mannitol, light anhydrous silicic acid, magnesium carbonate, calcium carbonate and L-cysteine. Preferable examples thereof include cornstarch and mannitol. These excipients may be used alone or in combination with each other. The content of an excipient is, for example, about 4.5 to about 99.4 w/w%, preferably about 20 to about 98.5 w/w%, more preferably about 30 to about 97w/w% based on the total amount of an instantaneous release formulation.

The content of a drug in an instantaneous release formulation can be appropriately selected from the range of about 0.5 to about 95%, preferably about 1 to about 60% based on the total amount of the instantaneous release formulation.

When an instantaneous release formulation is an oral solid formulation, it usually contains a disintegrant in addition to the aforementioned ingredients. As such a disintegrant, for example, calcium carboxymethyl cellulose (ECG-505 manufactured by GOTOKU CHEMICAL COMPANY LTD.), sodium coroscarmellose (Ac-Di-Sol manufactured by Asahi Kasei), crospovidone (e.g. Coridone CL manufactured by BASF), low-substituted hydroxypropyl cellulose (manufactured by Shin-Etsu Chemical Co., Ltd.), carboxymethyl starch (manufactured by Matsutani Chemical Industry Co., Ltd.), sodium carboxymethyl starch (Exprotab manufactured by Kimurasangyo) and partial α starch (PCS manufactured by Asahi Kasei) are used. For example, disintegrants which disintegrate a granule by contacting with water to effect water absorption or swelling, or to make a channel between a core-forming active ingredient and an excipient, can be used. These disintegrants may be used alone or in combination with each other. The amount of a disintegrant to be incorporated is appropriately selected depending on the kind and amount of a drug used and the design of releasing performance and the like, and it is for example about 0.05 to about 30 w/w%, preferably about 0.5 to about 15w/w% based on the total amount of an instantaneous release formulation.

When an instantaneous release formulation is an oral solid formulation, it may contain optionally further additives which are conventionally used for a solid formulation in addition to the aforementioned components. As such an additive, for example, a binder (e.g. sucrose, gelatin, gum arabic powder, methyl cellulose, hydroxypropyl cellulose, hydroxypropylmethyl cellulose, carboxymethyl cellulose, polyvinylpyrrolidone, plullan, dextrin etc.), a lubricant (e.g. polyethylene glycol, magnesium stearate, talc, light anhydrous silicic acid (e.g. Aerosil (Nippon aerosil)), a surfactant (e.g. anionic surfactant such as sodium alkylsulfate, nonionic surfactant such as polyoxyethylene fatty acid ester, polyoxyethylene sorbitan fatty acid ester, polyoxyethylene castor oil derivative etc.), a coloring agent (e.g. tar pigment, caramel, red ocher, titanium dioxide, riboflavin) and, if necessary, a corrigent (e.g. sweetener, perfume etc.), an adsorbent, a preservative, a wetting agent and an antistatic agent are used. In addition, as a stabilizer, an organic acid such as tartaric acid, citric acid, succinic acid and fumaric acid may be added.

As a binder, hydroxypropyl cellulose, polyethylene glycol and polyvinylpyrrolidone are preferably used.

An instantaneous release formulation can be prepared based on a standard formulation technique by mixing the aforementioned respective ingredients and, if necessary, further kneading and molding. The mixing is performed by a generally used method, for example, by mixing and kneading. Specifically, for example, when an instantaneous release formulation is formed into a particle, the particle can be prepared by mixing ingredients using a vertical granulator, a universal kneader (manufactured by HATA IRON WORKS Co., Ltd.), a fluidized bed granulator FD-5S (manufactured by POWREX CORPORATION) or the like and, thereafter, granulating a mixture by a wet extrusion granulation method or a fluidized bed granulation method, according to the same procedure as the process for preparing a core of the aforementioned sustained release formulation.

The instantaneous release formulation and sustained release formulation thus obtained may be formulated respectively into separate dosage forms by a standard method as they are or together with pharmaceutical excipients as appropriate, and then may be administered in combination with each other at the same time or at an arbitrary administration interval. Alternatively, the instantaneous release formulation and the sustained release formulation, as they are or together with pharmaceutical excipients, may be formulated into one oral dosage form (e.g. granule, fine particle, tablet, capsule). The instantaneous release formulation and the sustained release formulation may be formulated into granules or fine particles, which are then filled in a single capsule for oral administration.

### [3] Sublingual tablet, buccal or intraoral instantaneous disintegrating formulation and preparation thereof

A sublingual table, a buccal formulation or an intraoral instantaneous disintegrating formulation may be a solid formulation such as a tablet or may be an oral mucosal patch tablet (film).

As a sublingual tablet, a buccal or an intraoral instantaneous disintegrating formulation, a formulation containing the compound of the present invention or a concomitant drug and an excipient is preferable. In addition, a auxiliary agent such as a lubricant, an isotonizing agent, a hydrophilic carrier, a water-dispersible polymer and a stabilizer may be contained. In addition, in order to make absorption easy and enhance bioavailability, β-cyclodextrin or β-cyclodextrin derivatives (e.g. hydroxypropyl-β-cyclodextrin) may be contained.

Examples of the excipient include lactose, white sugar, D-mannitol, starch, crystalline cellulose and light anhydrous silicic acid. Examples of the lubricant include magnesium stearate, calcium stearate, talc and colloidal silica and in particular, magnesium stearate and colloidal silica are preferable. Examples of the isotonizing agent include sodium chloride, glucose, fructose, mannitol, sorbitol, lactose, saccharose, glycerin and urea and, in particular, mannitol is preferable. Examples of the hydrophilic carrier include swelling hydrophilic carriers such as crystalline cellulose, ethyl cellulose, crosslinked polyvinylpyrrolidone, light anhydrous silicic acid, silicic acid, dicalcium phosphate and calcium carbonate and in particular, crystalline cellulose (e.g. microcrystalline cellulose etc.) is preferable. Examples of the water-dispersible polymer include gum (e.g. tragacanth gurn, gum acacia, guar gum), alginate (e.g. sodium alginate), cellulose derivatives (e.g. methyl cellulose, carboxymethyl cellulose, hydroxymethyl cellulose, hydroxypropyl cellulose, hydroxypropylmethyl cellulose), gelatin, water-soluble starch, polyacrylic acid (e.g. Carbomer), polymethacrylic acid, polyvinyl alcohol, polyethylene glycol, polyvinylpyrrolidone, polycarbofil, and ascorbate palmitate ester. Hydroxypropylmethyl cellulose, polyacrylic acid, alginate, gelatin, carboxymethyl cellulose, polyvinylpyrrolidone and polyethlylene glycol are preferable. In particular, hydroxypropylmethyl cellulose is preferable. Examples of the stabilizer include cysteine, thiosorbitol, tartaric acid, citric acid, sodium carbonate, ascorbic acid, glycine and sodium sulfite. In particular, citric acid and ascorbic acid are preferable.

A sublingual tablet, a buccal or intraoral instantaneous disintegrating formulation can be prepared by mixing the compound of the present invention or a concomitant drug and an excipient by a method known per se. Further, optionally, the aforementioned auxiliary agent such as a lubricant, an isotonizing agent, a hydrophilic carrier, a water-dispersible polymer, a stabilizer, a coloring agent, a sweetener and a preservative may be mixed therein. The aforementioned ingredients are mixed at the same time or at a certain time interval, and the mixture is then compressed into a sublingual tablet, a buccal tablet or an intraoral instantaneous disintegrating tablet. In order to obtain an appropriate hardness, a solvent such as water and alcohol may be used to wet the mixture before or after the tableting with compression, and then dried finally.

When molded into a mucosal patch tablet (film), the compound of the present invention or a concomitant drug, the aforementioned water-dispersible polymer (preferably, hydroxypropyl cellulose, hydroxypropylmethyl cellulose), and an excipient are dissolved in a solvent such as water, and the resulting solution is cast into a film. Further, additives such as a plasticizer, a stabilizer, an antioxidant, a preservative, a coloring agent, a buffering agent and a sweetener may be added. In order to impart an appropriate elasticity to a film, glycols such as poylethylene glycol and propylene glycol may be added, or in order to enhance adhesion of a film to the mucosal lining of an oral cavity, a bioadhesive polymer (e.g. polycarbofil, carbopol) may be added. Casting is attained by pouring a solution onto a non-adhesive surface, spreading the solution to a uniform thickness (preferably, around 10 to 1000 micron) with a coating equipment such as a doctor blade, and then drying the solution to form a film. The film thus formed is dried at room temperature or under warming, and is cut into the desired surface area.

Preferable examples of an intraoral instantaneous disintegrating formulation include a solid rapid-diffusing formulation comprising a net of the compound of the present invention or a concomitant drug, and a water-soluble or water-diffusing carrier which is inert to the compound of the present invention or a concomitant drug. The net is obtained by sublimating a solvent from a solid composition comprising a solution of the present composition or a concomitant drug in an appropriate solvent.

The composition of an intraoral instantaneous disintegrating formulation preferably contains a matrix-forming agent and a secondary ingredient in addition to the compound of the present invention or a concomitant drug.

Examples of the matrix-forming agent include gelatins, dextrins, and animal proteins and vegetable proteins such as soybean, wheat and psyllium seed proteins; gummy substances such as gum arabic, guar gum, agar and xanthan; polysaccharides; alginic acids; carboxymethyl celluloses; carrageenans; dextrans; pectins; synthetic polymers such as polyvinylpyrrolidone; and substances derived from gelatin-gum arabic complex. Further, the examples include saccharides such as mannitol, dextrose, lactose, galactose and trehalose; cyclic saccharides such as cyclodextrin; inorganic salts such as sodium phosphate, sodium chloride and aluminum silicate; amino acids having 2 to 12 carbon atoms such as glycine, L-alanine, L-aspartic acid, L-glutamic acid, L-hydroxyproline, L-isoleucine, L-leucine and L-phenylalanine.

One or more kinds of the matrix-forming agents can be introduced into a solution or a suspension before solidification. Such matrix-forming agent may be present in addition to a surfactant, or may be present without a surfactant. The matrix-forming agent can assist maintenance of the diffused state of the compound of the present invention or a concomitant drug in a solution or a suspension, in addition to formation of a matrix.

The composition may contain secondary ingredients such as a preservative, an antioxidant, a surfactant, a thickener, a coloring agent, a pH modifier, a flavor, a sweetener and a taste masking agent. Examples of the suitable coloring agent include iron oxide red, black and yellow, and FD&C dyes such as FD&C Blue No.2 and FD&C Red No.40 of Ellis and Eberald. Examples of the suitable flavor include mint, raspberry, licorice, orange, lemon, grapefruit, caramel, vanilla, cherry and grape flavor and a combination thereof. Examples of the suitable pH modifier include citric acid, tartaric acid, phosphoric acid, hydrochloric acid and maleic acid. Examples of the suitable sweetener include aspartame, acesulfame K and thaumatine. Examples of the suitable taste masking agent include sodium bicarbonate, ion exchange resin, cyclodextrin inclusion compound, adsorbent substance and microcapsulated apomorphine.

The formulation contains the compound of the present invention or a concomitant drug usually in an amount of 0.1 to about 50% by weight, preferably about 0.1 to 30% by weight. The formulation is preferably a formulation (the aforementioned sublingual tablet, buccal) which allows 90% or more of the compound of the present invention or a concomitant drug to be dissolved (in water) in about 1 minute to about 60 minutes, preferably about 1 minute to 15 minutes, more preferably about 2 minutes to about 5 minutes, or an intraoral instantaneous disintegrating formulation which is disintegrates in 1 to 60 seconds, preferably 1 to 30 seconds, more preferably 1 to 10 seconds after being placed in an oral cavity.

The content of the excipient in the total formulation is about 10 to about 99% by weight, preferably about 30 to about 90% by weight. The content of β-cyclodextrin or a β-cyclodextrin derivative in the total formulation is 0 to about 30% by weight. The content of the lubricant in the total formulation is about 0.01 to about 10% by weight, preferably about 1 to about 5% by weight. The content of the isotonizing agent in the total formulation is about 0.1 to about 90% by weight, preferably about 10 to about 70% by weight. The content of the hydrophilic carrier in the total formulation is about 0.1 to about 50% by weight, preferably about 10 to about 30% by weight. The content of the water-dispersible polymer in the total formulation is about 0.1 to about 30% by weight, preferably about 10 to about 25% by weight. The content of the stabilizer in the total formulation is about 0.1 to about 10% by weight, preferably about 1 to about 5% by weight. The aforementioned formulation may further contain additives such as a coloring agent, a sweetener and an preservative, if necessary.

### [4] Solid dispersion of the compound of the present invention or a concomitant drug and preparation thereof

When the compound of the present invention [hereinafter, referred to as a lipid-rich plaque regressing substance in some cases] or a concomitant drug is hardly soluble or insoluble in water, then it may be formulated into a solid dispersion (e.g. solid dispersion containing a hardly water-soluble or water-insoluble lipid-rich plaque regressing substance and a hydrophilic polymer).

Herein, the "solid dispersion" refers to a dispersion in which one or two or more active ingredients (preferably, amorphous active ingredient) are dispersed in a carrier inert in the solid state (e.g. hydrophilic polymer), which can be prepared, for example, by a melting method, a solvent method or a melting-solvent method (J.Pharm.Sci., Vol.60, 1281-1302, 1971).

The average particle diameter of a solid dispersion is not particularly limited, but it is usually at least about. 0.05 µm, preferably about 0.1 µm, more preferably about 1 µm, further preferably 3 µm, and not more than about 30mm, preferably about 100 µm, more preferably about 50 µm, further preferably about 10 µm.

As a hydrophilic polymer used in the solid dispersion, for example, a water-soluble polymer, an enteric polymer, and a polymer soluble in stomach are used. Inter alia, an enteric polymer is preferably used.

As the water-soluble polymer, for example, (1) hydroxyalkyl cellulose such as hydroxypropyl cellulose, hydroxypropylmethyl cellulose and the like; cellulose derivatives such as alkyl cellulose such as methyl cellulose or ethyl cellulose, and the like; (2) polyalkenylpyrrolidone such as poylvinylpyrrolidone and the like; (3) polyalkylene glycol such as polyethylene glycol and the like are used.

As the enteric polymer, for example, hydroxyalkyl cellulose phthalate such as hydroxypropylmethyl cellulose phthalate; hydroxyalkyl cellulose acetate succinate such as hydroxypropylmethyl cellulose acetate succinate; carboxyalkyl cellulose such as carboxymethylethyl cellulose; cellulose acetate phthalate; a copolymer of ethyl acrylate and methacrylic acid such as methacrylic acid copolymer L-100-55; a copolymer of methyl methacrylate and methacrylic acid such as methacrylic acid copolymer L or methacrylic acid copolymer S are used.

As the polymer soluble in stomach, for example, aminoalkyl methacrylate copolymer E; and polyvinylacetal diethylaminoacetate are used.

In addition, hydrophilic polymers which can disperse a hardly water-soluble or insoluble lipid-rich plaque regressing substance, including a copolymer of ethyl acrylate and methyl methacrylate containing a small amount of a quaternary ammonium group such as methacrylic acid copolymer RL and methacrylic acid copolymer RS, carboxymethyl cellulose, carboxyvinyl polymer, polyvinyl alcohol, gum arabic, sodium aliginate, aliginic acid propylene glycol ester, agar, gelatin and chitosan is used. These hydrophilic polymers may be used bin combination with each other.

Among those listed above, as the hydrophilic polymer, hydroxyalkyl cellulose, alkyl cellulose, polyalkenylpyrrolidone, polyalkylene glycol, methacrylic acid copolymer, and carboxymethyl cellulose are preferable. In particular, hydroxypropylmethyl cellulose phthalate, polyvinylpyrrolidone, hydroxypropylmethyl cellulose, carboxymethylethyl cellulose, and methacrylic acid copolymer L are suitable.

The solid dispersion may contain additives which are used in pharmaceutical field generally.

Such an additive includes pharmaceutically acceptable carriers such as various organic or inorganic carrier substances which are conventionally used as a pharmaceutical material, and it is incorporated as an excipient, a lubricant, a binder, a disintegrant or a surfactant. In addition, if needed, pharmaceutical additives such as a preservative, an antioxidant, a coloring agent and a sweetener may be used.

Preferable examples of the excipient include lactose, white sugar, D-mannitol, starch, crystalline cellulose, sucrose, porous starch, mannitol, calcium silicate (trade name: Fluorite RE), magnesium metasilicate aluminate (trade name: Neusilin), light anhydrous silicic acid (trade name: Cylicia), white sugar/starch spherical granule (trade name; Nonpareil), crystalline cellulose/carboxymethyl cellulose (trade name: Avicel RC), and hydroxypropyl starch.

Preferably examples of the lubricant include magnesium stearate, calcium stearate, talc and colloidal silica.

Preferable examples of the binder include crystalline cellulose, white sugar, D-mannitol, dextrin, hydroxypropyl cellulose, hydroxypropylmethyl cellulose, and polyvinylpyrrolidone.

Preferable examples of the disintegrant include starch, carboxymethyl cellulose, calcium carboxymethyl cellulose, sodium croscarmellose, sodium carboxymethyl starch, methyl cellulose (trade name: Metholose SM), sodium croscarmellose, carmellose calcium, low-substituted hydroxypropyl cellulose, starch sodium glycolate, and partially alpha-derivatived starch.

The lubricant includes talc, crystalline cellulose, magnesium stearate, cornstarch, magnesium oxide.

The surfactant includes polyoxyethylene polyoxypropylene glycol (trade name: Pluronic), glycerin fatty acid ester, sucrose fatty acid ester, polyoxyethylene hydrogenated castor oil, Polysorbate 80, and cetanol.

Preferable examples of the preservative include p-hydroxybenzoic acid esters, chlorobutanol, benzyl alcohol, phenethyl alcohol, dehydroacetic acid, and sorbic acid.

Suitable examples of the antioxidant include sulfite and ascorbic acid.

These additives may be used alone or in combination with each other.

The solid dispersion can be prepared using a method known per se, and specifically, can be prepared by a solvent method such as a spray-drying method and a rotary evaporation method; a melting method such as a twin-screw extruder method; a mixing grinding method; an ultrasonication method using an ultrasonic molding machine.

More specifically, the solid dispersion can be prepared by the following solvent method:
(1) dissolving a lipid-rich plaque regressing substance in a suitable organic solvent,
(2) adding a hydrophilic polymer to this solution to prepare a suspension,
(3) suspending an additive such as an excipient, a disintegrant, a lubricant and a surfactant in this suspension or solution, if needed, and ,
(4) distilling off the organic solvent from this uniform suspension under reduced pressure or normal pressure by a conventional method such as a spray-drying method, and a rotary evaporation method.
   In addition, when a more uniform solid dispersion is desired, a uniform suspension is prepared by the above step (2) and is then subjected to the following sequential steps:
(5) dissolving the suspension prepared in the step (2) in a suitable organic solvent,
(6) suspending an additive such as an excipient, a disintegrant, a lubricant and a surfactant therein, if needed, and,
(7) distilling off the organic solvent under reduced pressure or normal pressure by a conventional method such as a spray-drying method and a rotary evaporation method.

The organic solvent used in the step (1) is not particularly limited as far as it can dissolve a hardly water-soluble or insoluble lipid-rich plaque regressing substance and a hydrophilic polymer. For example, alcohols such as methanol, ethanol, propanol, isopropyl alcohol, butanol, monomethoxyethanol, ethylene glycol monomethyl ether and the like; ethers such as diethylether, dibutyl ether, diisobutylether, dioxane, tetrahydrofuran, ethylene glycol and the like; aliphatic hydrocarbons such as n-hexane, cyclohexane, n-heptane; aromatic hydrocarbons such as benzene, toluene and xylene; nitriles such as acetonitrile and the like; organic acids such as acetic acid, propionic acid and the like; esters such as ethyl acetate and the like; aliphatic halogenated hydrocarbons such as dichloromethane, dichloroethane, chloroform and the like; ketones such as acetone, methylketone and the like; amides such as dimethylformamide, dimethyacetamide and the like; or a mixed solution of these at an appropriate ratio can be used. Among them, solvents having a low boiling point such as ketones and alcohols are preferable. Inter alia, acetone and ethanol are preferable.

Although operating conditions such as the treating temperature and the treating time vary depending on the starting compound and an organic solvent to be used, the treating temperature is usually 200°C or below.

In a melting method, a hardly water-soluble or insoluble lipid-rich plaque regressing substance is warmed to a temperature above the melting point to melt it, and a hydrophilic polymer and, if needed, an additive such as an excipient, a disintegrant, a lubricant and a surfactant are dissolved therein and then cooled rapidly to accomplish the production. For example, in a twin-screw extruder method, a hardly water-soluble or insoluble lipid-rich plaque regressing substance and a hydrophilic polymer and, if necessary, an additive such as an excipient, a disintegrant, a lubricant and a surfactant are mixed mechanically, warmed under high pressure to melt the hardly water-soluble or insoluble lipid-rich plaque regressing substance at a temperature below the melting point, and then cooled rapidly to accomplish the production.

In a mixing grinding method, a hardly water-soluble or insoluble lipid-rich plaque regressing substance and a hydrophilic polymer and, if necessary, an additive such as an excipient, a disintegrant, a lubricant and a surfactant are mixed mechanically and then ground with mixing to accomplish the production.

In an ultrasonication method, a hardly water-soluble or insoluble lipid-rich plaque regressing substance and a hydrophilic polymer and, if necessary, an additive such as an excipient, a disintegrant, a lubricant and a surfactant are mixed mechanically, and the mixture is charged in a mortar to pre-mold it, and then irradiated with an ultrasonic wave for example by using an ultrasonic molding machine to accomplish the production.

The amount of a hydrophilic polymer is not particularly limited, and may be any amount as far as it is such an amount that a hardly water-soluble or insoluble lipid-rich plaque regressing substance can be dispersed. For example, the preferable weight ratio between a hydrophilic polymer and a hardly water-soluble or insoluble lipid-rich plaque regressing substance is in the range of 0.01:1 to 100:1, preferably 0.02:1 to 50:1, more preferably 0.1:2 to 20:1, further preferably 0.3:1 to 10:1, more preferably 1:1 to 10:1, particularly preferably 3 to 5 (in particular 4):1.

The amount of an additive is not particularly limited, but when an additive is used, the preferable weight ratio between an additive such as an excipient, a disintegrant, a lubricant and a surfactant and a hardly water-soluble or insoluble lipid-rich plaque regressing substance is in the range of usually 0.1:1 to 20:1, preferably 0.3:1 to 10:1, more preferably 1:1 to 3:1.

An organic solvent used in the above step (5) is not particularly limited and any solvent may be used as far as it is a solvent which can dissolve a suspension in the step (2), such as chloroform and dichloromethane.

The solid dispersion can be used as it is as a pharmaceutical formulation for oral administration and may be formulated into a pharmaceutical formulation such as a particle, a fine particle, a granule, a tablet, a capsule and an injection by a conventional method.

A pharmaceutical formulation containing the solid dispersion describe above may contain the aforementioned additives, specifically, a coloring agent, a sweetener, a flavor, such as sucrose, lactose, starch, crystalline cellulose, synthetic ammonium silicate, magnesium stearate, talc and other diluents and lubricants in a pharmaceutical formulation for oral administration. Alternatively, the surface of a formulation may be coated to obtain a sustained-release formulation.

Usually, since a lipid-rich plaque regressing substance is hardly water-soluble or insoluble, when orally administered, the ratio at which it is absorbed actually into blood based on the dose give is low, resulting in a problematically low bioavailability.

However, various formulations prepared by converting the solid dispersion described above into the aforementioned various dosage forms have remarkably improved solubility, oral absorbability and/or absorbability into blood as compared with a crystal of a hardly water-soluble or insoluble lipid-rich plaque regressing substance itself.

Thus, the solid dispersion described above enables the solubilization of a hardly water-soluble or insoluble lipid-rich plaque regressing substance, thereby the bioavailability of a hardly water-soluble or insoluble lipid-rich plaque regressing substance is dramatically improved.

The content of a hardly water-soluble or insoluble lipid-rich plaque regressing substance in the solid dispersion varies depending on the dosage form, the administration method, a carrier and the like, and is usually 0.1 to 99% (w/w) of the total amount of the formulation.

The content of a hydrophilic polymer in the solid dispersion varies depending on the dosage form, the administration method, a carrier and the like, and is usually 1 to 99.9% (w/w) of the total amount of the formulation.

The content of an additive in the solid dispersion varies depending on the dosage form, the administration method and the like, and is usually 0 to 99% (w/w) of the total amount of the formulation.

The content of the solid dispersion in the pharmaceutical formulation of the present invention varies depending on the dosage form, the administration method, a carrier and the like, and is usually 0.1 to 100% (w/w) of the total amount of the formulation.

The content of an additive in the pharmaceutical formulation of the present invention varies depending on the dosage form, the administration method and the like, and is usually 0 to 99.9% (w/w) of the total amount of the formulation.

The dose of the concomitant formulation of the present invention varies depending on the kind of the compound of the present invention, age, body weight, symptom, dosage form, administration method, administration period and the like. For example, the daily dose for a hyperlipemia patient (adult, about 60 kg) is usually about 0.01 to about 100 mg/kg, preferably about 0.01 to about 100 mg/kg, more preferably about 0.1 to about 100 mg/kg, particularly about 0.1 to about 50 mg/kg, inter alia, about 1.5 to about 30 mg/kg of the compound of the present invention and administered intravenously once or in several portions. Of course, since a dose varies depending on various conditions as described above, an amount smaller than the aforementioned dose may be sufficient or a dose exceeding the aforementioned range may be necessary.

The dose of a concomitant drug can be set in any range as far as it does not cause problematic side effect. The daily dose of a concomitant drug is not limited particularly and varies depending on the severity of symptom, age, sex, body weight and susceptibility of a subject to be administered, timing and interval of administration, nature, preparation and kind of a pharmaceutical formulation, kind of an active ingredient, and the like. The daily oral dose per kg body weight in a mammal is usually about 0.001 to 2000 mg, preferably about 0.01 to 500 mg, further preferably about 0.1 to 100 mg of a concomitant drug, which is given usually in 1 to 4 portions.

Upon administration of the concomitant formulation of the present invention, the compound of the present invention and a concomitant drug may be administered at the same time, but after a concomitant drug is administered, the compound of the present invention may be administered. Alternatively, after the compound of the present invention is administered, a concomitant drug may be administered. When they are administered at a certain time interval, the interval varies depending on an active ingredient to be administered, a dosage form, an administration method and the like. For example, when a concomitant drug is administered in advance, the compound of the present invention is administered in 1 minute to 3 days, preferably 10 minutes, to 1 day, more preferably 15 minute to 1 hour after administration of a concomitant drug. For example, when the compound of the present invention is administered in advance, a concomitant drug is administered in 1 minute to 1 day, preferably 10 minutes to 6 hours, more preferably 15 minutes to 1 hour after administration of the compound of the present invention.

As a preferable administration method, for example, about 0.001 to 200 mg/kg of a concomitant drug which has been formulated into an oral formulation is orally administered and, after about 15 minutes, about 0.005 to 100 mg/kg of the compound of the present invention which has been formulated into an oral formulation is orally administered as the daily dose.

The following Examples, Preparation Examples and Experimental Examples further illustrate the present invention, but the present invention is not limited by them.

A ¹H-NMR spectrum was measured with a Varian Gemini 200 (200MHz) spectrometer using tetramethylsilane as an internal standard, and total δ values are represented in ppm. Unless otherwise is indicated, a numerical value shown for a mixed solvent is a volume mixing ratio of each solvent. Unless otherwise indicated, % means % by weight. In addition, the ratio of an eluted solvent for silica gel chromatography indicates a volume ratio, unless otherwise indicated. Herein, room temperature (normal temperature) represents a temperature of about 20°C to about 30°C.

Respective symbols in Examples represent the following meanings.

AcOEt: ethyl acetate, Me: methyl, Et: ethyl, THF: tetrahydrofuran, IPE: isopropyl ether, Et₂O: diethyl ether, decomp.: decomposition, s: singlet, d: doublet, t: triplet, q: quartet, dd: double doublet, dt: double triplet, m: multiplet, br: broad, J: coupling constant, Py: pyridyl, DBU: diazabicycloundecene, DMF: dimethylformamide, DPPA: diphenylphosphorylazide, NBS: N-bromosuccinimide, AIBM: azobisisobutyronitrile, hex: hexane, Ac: acetyl, Ph:phenyl, Ts:tosyl, mCPBA: m-chloroperbenzoic acid, ^{t}Bu: tert-butyl

### Referecne Example 1

### Synthesis of ethyl (2E)-3-[5-[7-chloro-3-[2-[[4-fluoro-2-(trifluoromethyl)phenyl]amino]-2-oxoethyl]-6-methyl-2-oxo-2H-chromen-4-yl]phenyl]-2-propenoate

### (a) Synthesis of (3-bromophenyl)(4-chloro-2-hydroxy-5-methylphenyl)methanone

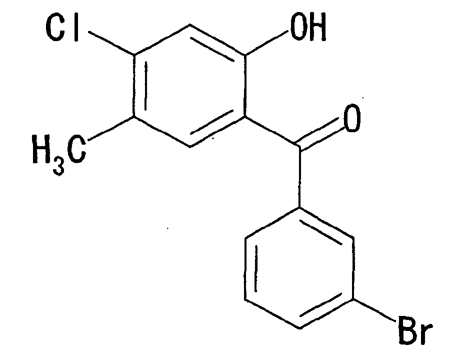

Aluminium chloride (102 g) was added to a solution of 3-chloro-4-methylanisole (97 g) in chlorobenzene (300 ml) under ice-cooling, and 3-bromobenzoyl chloride (136 g) was further added dropwise over 1 hour. After completion of addition, the mixture was stirred at room temperature for 30 minutes, and further heated at 120°C for 30 minutes. The reaction solution was ice-cooled, ethyl acetate (600 ml), methanol (100 ml) and 4N hydrochloric acid (400 ml) were added successively, and the mixture was stirred at room temperature for 30 minutes. The organic layer was washed successively with 1N hydrochloric acid and an aqueous saturated sodium chloride solution, dried over magnesium sulfate, and a solvent was distilled off under reduced pressure. The residue was purified by recrystallizing from ethyl acetate-hexane, to obtain the title compound (186 g, yield 89%).
mp: 115-127°C.

### (b) Synthesis of [4-(3-bromophenyl)-7-chloro-6-methyl-2-oxo-2H-chromen-3-yl]acetic acid

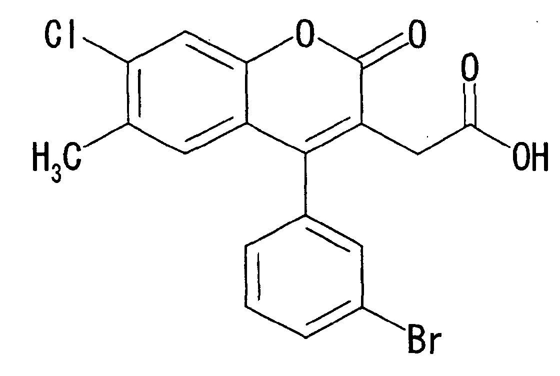

DBU (230 ml) was added to a suspension of (3-bromophenyl) (4-chloro-2-hydroxy-5-methylphenyl)methanone (186 g) in acetonitrile (400 ml), a solution of ethyl succinic chloride (157 g) in acetonitrile (250 ml) was added dropwise over 1 hour while warming to 40°C, and the mixture was stirred for 30 minutes. Water (450 ml) was added to the reaction solution, and the mixture was stirred for 30 minutes under ice-cooling. The resulting crystals were filtered, and washed with ethanol. The resulting crude crystals (182 g) of ethyl [4-(3-bromophenyl)-7-chloro-6-methyl-2-oxo-2H-chromen-3-yl]acetate was dissolved in acetic acid (1600 ml) and concentrated hydrochloric acid (600 ml), and the mixture was heated to reflux for 1 hour. The reaction solution was concentrated under reduced pressure, the resulting residue was washed with water, dried (diphosphorus pentaoxide), and purified by recrystallization from ethyl acetate to obtain the title compound (166 g, yield 71%).
mp: 270°C(decomp.).

### (c) Synthesis of 2-[4-(3-bromophenyl)-7-chloro-6-methyl-2-oxo-2H-chromen-3-yl]-N-[4-fluoro-2-(trifluoromethyl)phenyl]acetamide

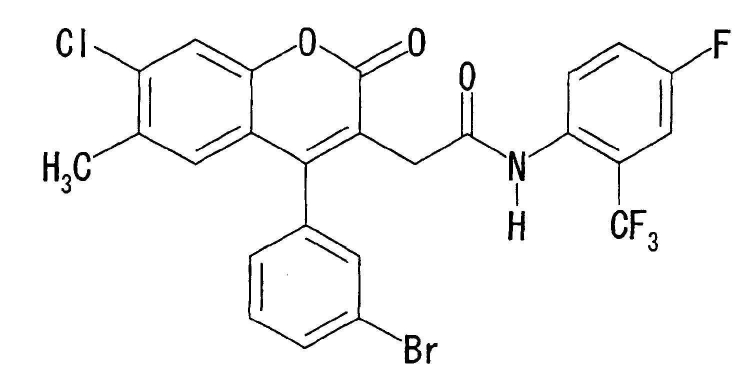

Dimethylformamide (5 drops) and oxalyl chloride (11 ml) were added to a solution of [4-(3-bromophenyl)-7-chloro-6-methyl-2-oxo-2H-chromen-3-yl]acetic acid (42 g) in THF (400 ml), and the mixture was stirred at room temperature for 30 minutes. The reaction solution was concentrated under reduced pressure to obtain a residue, which was dissolved in THF (400 ml). 4-Fluoro-2-(trifluoromethyl)aniline (14.7 ml) and sodium hydride (100 mg) were added, and the mixture was stirred at room temperature overnight. Water was added to the reaction solution, this was extracted with ethyl acetate, the extract was washed successively with 1N hydrochloric acid, an aqueous saturated sodium hydrogencarbonate solution and an aqueous saturated sodium chloride solution, dried over magnesium sulfate, and concentrated. The resulting residue was purified by recrystallization from ethyl acetate-THF to obtain the title compound (60 g, yield 77%).
mp: 222-223°C.

### (d) Synthesis of ethyl (2E)-3-[5-[7-chloro-3-[2-[[4-fluoro-2-(trifluoromethyl)phenyl]amino]-2-oxoethyl]-6-methyl-2-oxo-2H-chromen-4-yl]phenyl]-2-propenoate

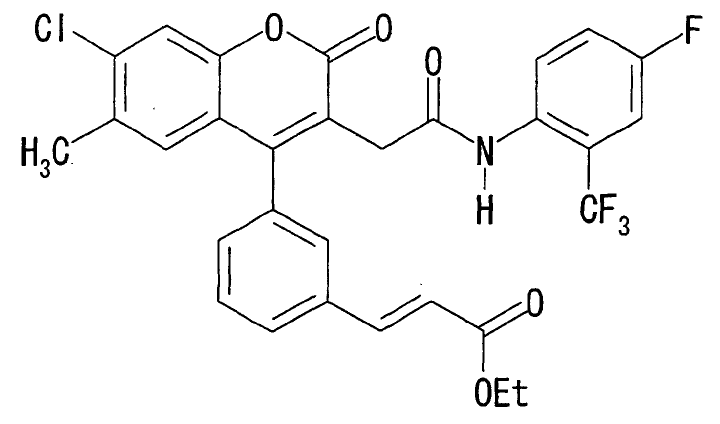

Ethyl acrylate (5.8 ml), triethylamine (7.9 ml), palladium (II) acetate (0.6 g) and triphenylphosphine (1.3 g) were added to a solution of 2-[4-(3-bromophenyl)-7-chloro-6-methyl-2-oxo-2H-chromen-3-yl]-N-[4-fluoro-2-(trifluoromethyl)phenyl]acetamide (30 g) in DMF (300 ml) under nitrogen atmosphere, and the mixture was heated at 100°C for 5 hours. After completion of the reaction, water was added, and this was extracted with ethyl acetate. The extract was washed with water, dried over magnesium sulfate, and concentrated. The resulting residue was purified by silica gel column chromatography (developing solvent: hexane-ethyl acetate=3:1), and recrystallized from ethyl acetate to obtain the title compound as colorless crystals (16.7 g, yield 55%).
mp: 193-196°C.

### Referecne Example 2

### Synthesis of (3-bromophenyl)(4-chloro-2-hydroxyphenyl)methanone

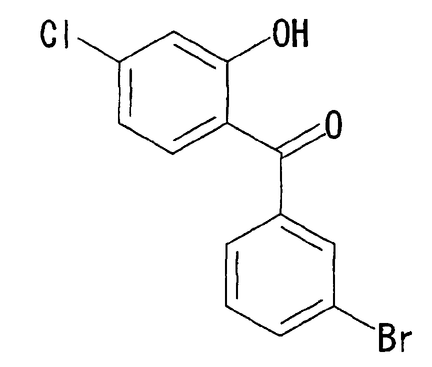

The title compound was prepared according to the method described in Tetrahedoron. Lett., vol. 42, p.4841 (2001).

Under ice-cooling, 1-ethyl-3-(3-dimethylaminopropyl)carbodimide hydrochloride (30.7 g) was added in portions to a mixed suspension of 4-chloro-2-hydroxybenzoic acid (13.8 g), N,O-dimethylhydroxylamine hydrochloride (15.6 g), 1-hydroxybenzotriazole (24.5 g) and triethylamine (22.3 ml) in N,N-dimethylformamide (20 ml) and dichloromethane (300 ml), and the mixture was stirred at room temperature for 6 hours. After completion of the reaction, the reaction solvent was concentrated and distilled off under reduced pressure, water was poured to the residue, and an organic material was extracted with ethyl acetate. The extract was washed with an aqueous saturated sodium chloride solution, dried over anhydrous magnesium sulfate, and the solvent was distilled off under reduced pressure. The resulting residue was purified by silica gel column chromatography (hexane:ethyl acetate=4:1) to obtain 4-chloro-2-hydroxy-N-methoxy-N-methylbenzamide (14.2 g, yield 82%).

Under dry nitrogen atmosphere, butyllithium (1.6M hexane solution, 12.5 ml) was added dropwise to butylmagnesium chloride (2M tetrahydrofuran solution, 5 ml) at 0°C, and the mixture was stirred for 30 minutes. To this was added dropwise a solution of 1,3-dibromobenzene (5.90 g) in toluene (15 ml). After completion of addition, the reaction solution was further stirred for 1.5 hours. Under ice-cooling, the suspension was added gradually to a solution of the aforementioned 4-chloro-2-hydroxy-N-methoxy-N-methylbenzamide (1.73 g) in toluene (15 ml), and the mixture was stirred for 1 hour. The reaction solution was poured into a 10% aqueous citric acid solution, and an organic material was extracted with ethyl acetate. The extract was dried over magnesium sulfate, and the solvent was distilled off under reduced pressure. The resulting residue was purified by silica gel column chromatography (hexane:ethyl acetate=19:1), and the resulting crystals were recrystallized from methanol to obtain the title compound (1.79 g, yield 71%).
mp: 105°C.
NMR (CDCl₃) δ: 6.89 (1H,dd,J = 8.4,2.2Hz), 7.11 (1H,d,J = 2.2Hz), 7.40 (1H,t,J = 8.0Hz), 7.49 (1H,d,J = 8.4Hz), 7.57 (1H,ddd,J = 8.0,1.4,1.0Hz), 7.74 (1H,ddd,J = 8.0,1.8,1.0Hz), 7.79 (1H,dd,J = 1.8,1.4Hz), 12.00 (1H,s).
IR(KBr): 3067, 1626, 1329, 1235, 1215 cm⁻¹.
Elemental analysis for C₁₃H₈BrClO₂
Calculated(%): C: 50.12, H: 2.59.
Found(%): C: 50.07, H: 2.53.

### Reference Examples 3 to 6

According to the same manner as that of Reference Example 1(C), compounds shown in [Table 1] (Reference Example 3: 2-[4-(3-bromophenyl)-7-chloro-6-methyl-2-oxo-2H-chromen-3-yl]-N-[2-(trifluoromethyl)phenyl]acetamide, Reference Example 4: 2-[4-(3-bromophenyl)-6-chloro-7-methyl-2-oxo-2H-chromen-3-yl]-N-[4-fluoro-2-(trifluoromethyl)phenyl]acetamide, Reference Example 5: 2-[4-(3-bromophenyl)-7-chloro-6-fluoro-2-oxo-2H-chromen-3-yl]-N-[4-fluoro-2-(trifluoromethyl)phenyl]acetamide, Reference Example 6: 2-[4-(3-bromophenyl)-7-chloro-2-oxo-2H-chromen-3-yl]-N-[4-fluoro-2-(trifluoromethyl)phenyl]acetamide) were obtained.

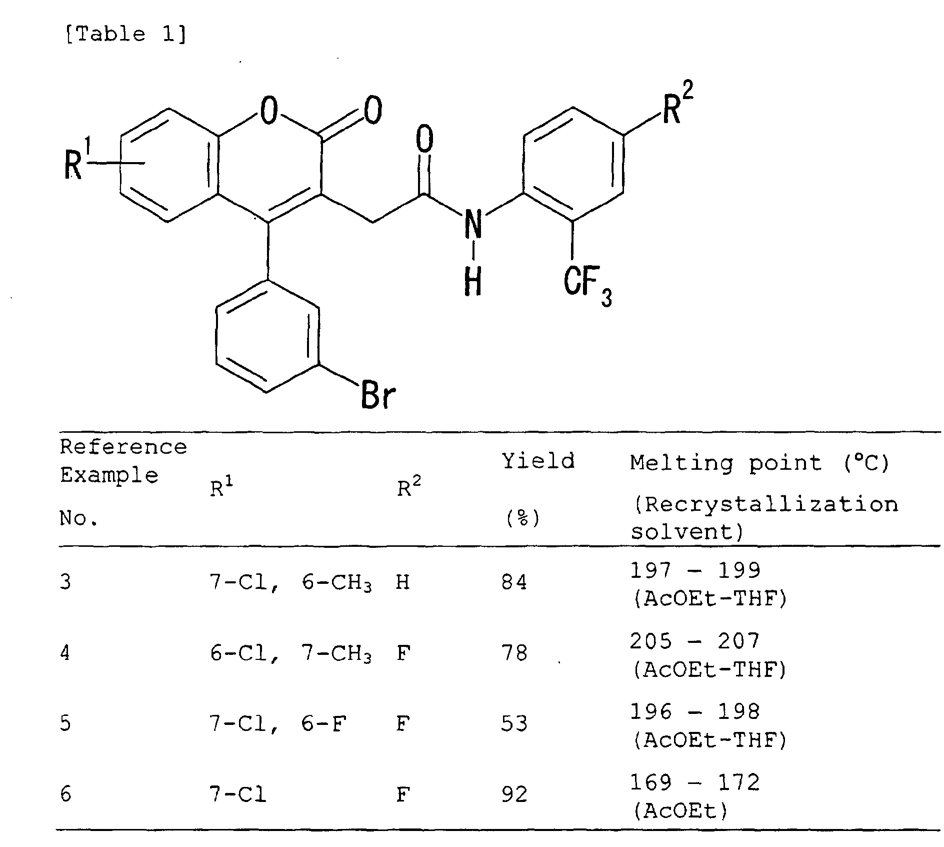

### Reference Example 7

### Synthesis of 2-[7-chloro-4-(3-formylphenyl)-6-methyl-2-oxo-2H-chromen-3-yl]-N-{4-chloro-2-(trifluoromethyl)phenyl]acetamide

### (a) Synthesis of [7-chloro-4-(3-formylphenyl)-6-methyl-2-oxo-2H-chromen-3-yl]acetic acid

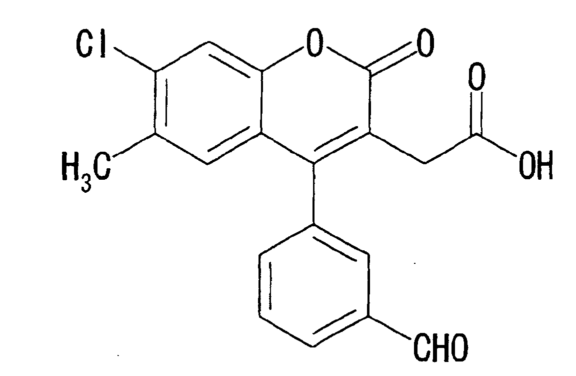

Under nitrogen atmosphere, butyllithium (1.6M hexane solution, 85 ml) was added dropwise to a solution of 2-(3-bromophenyl)-1,3-dioxolane (26.0 g) in THF (200 ml) at -78°C, the mixture was stirred at -78°C for 1 hour, a solution of 4-chloro-2-hydroxy-2N-methoxy-N,5-dimethylbenzamide (10.0 g) in THF (100 ml) was added dropwise, and the mixture was stirred at -78°C for 2 hours. 2N hydrochloric acid (200 ml) was added to the reaction solution, and extracted with ethyl acetate. The extract was concentrated under reduced pressure to obtain a residue, which was dissolved in THF (100 ml). 2N hydrochloric acid (150 ml) was added, and the mixture was stirred at room temperature overnight. The reaction solution was extracted with ethyl acetate, the extract was washed with water, dried over magnesium sulfate and concentrated. To 3-(4-chloro-2-hydroxy-5-methylbenzoyl)benzaldehyde (10 g) obtained as an oil were added acetonitrile (40 ml) and DBU (15 ml), a solution of ethyl succinate chloride (10.2 g) in acetonitrile (20 ml) was added dropwise over 1 hour while warming to 40°C, and the mixture was stirred for 30 minutes. Water (40 ml) was added to the reaction solution, extracted with ethyl acetate, the extract was washed successively with 1N-hydrochloric acid and water, dried over magnesium sulfate, and concentrated. The resulting residue was purified by silica gel column chromatography (developing solvent:hexane-ethyl acetate=3:1). The resulting crude crystals (2.8 g) of ethyl [7-chloro-4-(3-formylphenyl)-6-methyl-2-oxo-2H-chromen-3-yl]acetate were dissolved in acetic acid (150 ml) and concentrated hydrochloric acid (75 ml), and the solution was heated to reflux for 1 hour. The reaction solution was concentrated under reduced pressure, the resulting residue was washed with water, dried and recrystallized from ethyl acetate to obtain the title compound (2.5 g, yield 15%).
mp: 230-232°C.

### (b) Synthesis of 2-[7-chloro-4-(3-formylphenyl)-6-methyl-2-oxo-2H-chromen-3-yl]-N-[4-chloro-2-(trifluoromethyl)phenyl]acetamide

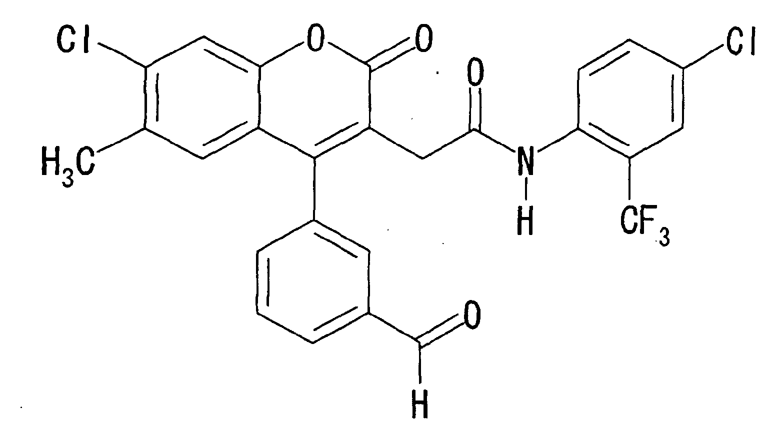

According to the same manner as that of Reference Example 1(c), the title compound (yield 50%) was obtained.
mp: 232-233°C.
NMR (CDCl₃) δ: 2.29 (3H, s), 3.37 (1H, d, J = 14.0 Hz), 3.51 (1H, d, 14.0 Hz), 6.79 (1H, s), 7.47 (2H, m), 7.58 (1H, s), 7.66 (1H, d, J = 7.4 Hz), 7.78 (1H, t, J = 7.4 Hz), 7.87 (1H, s), 8.07 (2H, m), 8.28 (1H, brs), 10.11 (1H, s).
Elemental analysis for C₂₆H₁₆Cl₂F₃NO₄
Calculated(%): C: 58.45, H: 3.02, N: 2.62.
Found(%): C: 58.41, H: 3.03, N: 2.39.

### Reference Example 8

### Synthesis of 2-[7-chloro-4-(3-formylphenyl)-6-methyl-2-oxo-2H-chromen-3-yl]-N-[4-fluoro-2-(trifluoromethyl)phenyl]acetamide

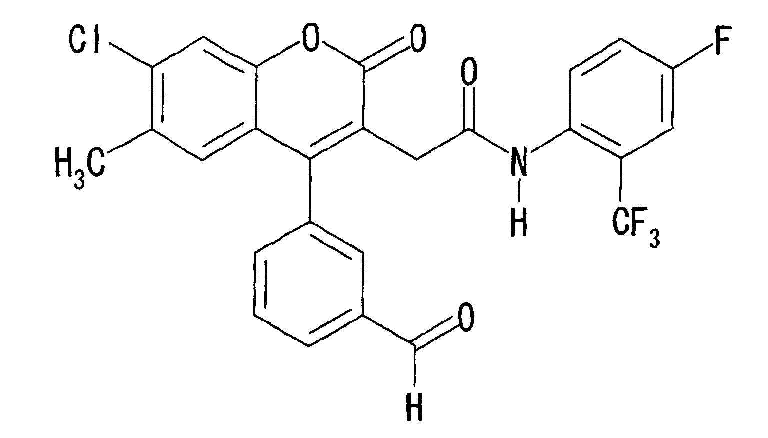

According to the same manner as that of Reference Example 7, the title compound was obtained (yield 68%).
mp: 214-215°C.
NMR (CDCl₃) δ: 2.29 (3H, s), 3.36 (1H, d, J = 14.0 Hz), 3.50 (1H, d, J = 14.0 Hz), 6,80 (1H, s), 7.31 (2H, m), 7.48 (1H, s), 7.68 (1H, m), 7.77 (1H, t, J = 7.7 7 Hz), 7.87 (1H, s), 7.98 (1H, m), 8.09 (1H, d, J = 7.6 Hz), 8.19 (1H, brs), 10.11 (1H, s).
Elemental analysis for C₂₆H₁₆ClF₄NO₄·0.3 H₂O
Calculated(%): C: 59.68, H: 3.20, N: 2.68.
Found(%): C: 59.45, H: 3.01, N: 2.63.

### Example 1

### Synthesis of 3-[7-chloro-3-[2-[[4-chloro-2-(trifluoromethyl)phenyl]amino]-2-oxoethyl]-6-methyl-2-oxo-2H-chromen-4-yl]benzoic acid

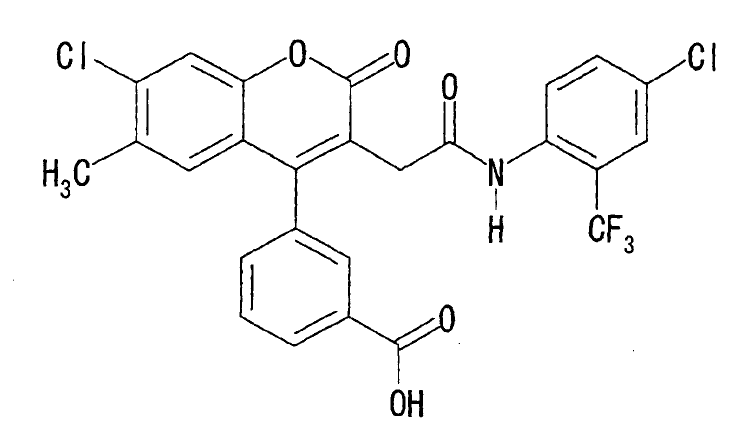

Sodium dihydrogenphosphate (450 mg) and 2-methyl-2-butene (1.8 ml) were added to a mixed suspension of 2-[7-chloro-4-(3-formylphenyl)-6-methyl-2-oxo-2H-chromen-3-yl]-N-[4-chloro-2-(trifluoromethyl)phenyl]acetamide (2.0 g) in t-butyl alcohol (30 ml), THF(10 ml) and water (8 ml), and the mixture was stirred at room temperature. Sodium chlorite (1.2 g) was gradually added to the reaction solution, and mixture was stirred at room temperature for 1 hour. After completion of the reaction, 1N hydrochloric acid was added, extracted with ethyl acetate, the extract was washed with water, dried over magnesium sulfate, and concentrated. The resulting residue was recrystallized from acetic acid to obtain the title compound (2.0 g, yield 92%).
mp: 270-272°C.
NMR (CDCl₃) δ: 2.28 (3H, s), 3.34 (1H, d, J = 14.8 Hz), 3.57 (1H, d, J = 14.8 Hz), 6.82 (1H, s), 7.4 - 7.7 (5H, m), 7.99 (2H, m), 8.23 (1H, d, J = 7.8 Hz), 8.38 (1H, brs).
Elemental analysis for C₂₆H₁₆Cl₂F₃NO₅
Calculated(%): C: 56.75, H: 2.93, N: 2.55.
Found(%): C: 56.46, H: 3.02, N: 2.58.

### Example 2

### Synthesis of 3-[7-chloro-3-]2-[[4-fluoro-2-(trifluoromethyl)phenyl]amino]-2-oxoethyl]-6-methyl-2-oxo-2H-chromen-4-yl]benzoic acid

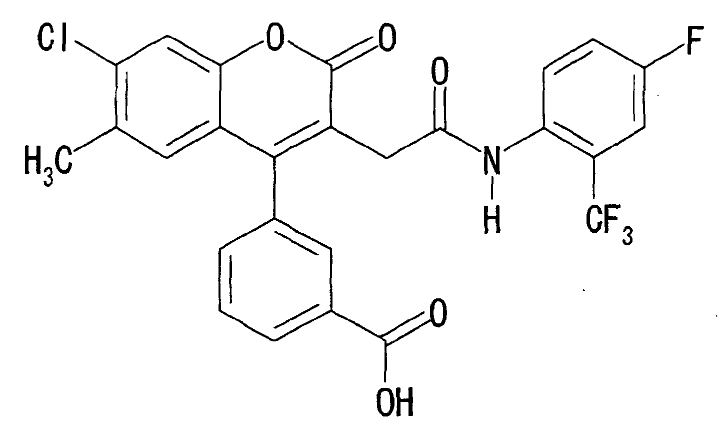

According to the same manner as that of Example 1, the title compound was obtained (yield 86%).
mp: 278-280°C
NMR (CDCl₃) δ: 2.28 (3H, s), 3.35 (1H, d, J = 16.0 Hz), 3.59 (1H, d, J = 16.0 Hz), 6.82. (1H, s), 7.29 (2H, m), 7.45 (1H, d, J = 9.2 Hz), 7.55 (2H, m), 7.75 (1H, m), 7.98 (1H, s), 8.22 (1H, d, J = 7.6 Hz), 8.77 (1H, brs).
Elemental analysis for C₂₆H₁₆ClF₄NO₅
Calculated(%): C: 58.49, H: 3.02, N: 2.62.
Found(%): C: 58.41, H: 3.25, N: 2.43.

### Example 3

### Synthesis of ethyl (2E)-3-[5-[7-chloro-3-[2-[[4-chloro-2-(trifluoromethyl)phenyl]amino]-2-oxoethyl]-6-methyl-2-oxo-2H-chromen-4-yl]-2-fluorophenyl]-2-propenoate

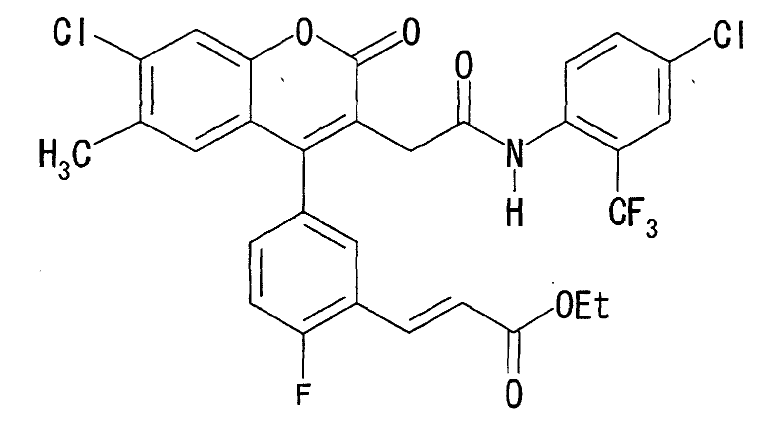

According to the same manner as that of Reference Example 1-(d), the title compound (yield 28%) was obtained.
mp: 160-162°C.
NMR (CDCl₃) 5: 1.33 (3H, t, J = 7.0Hz), 2.31 (3H, s), 3.45 (2H, s), 4.27 (2H, q, J = 7.0 Hz), 6.60 (1H, d, J = 16.4 Hz), 6.86 (1H, s), 7.3 - 7.4 (2H, m), 7.5 - 7.6 (4H, m), 7.74 (1H, d, J = 16.4 Hz), 8.07 (1H, d, J = 8.8 Hz), 8.30 (1H, brs).
Elemental analysis for C₃₀H₂₁Cl₂F₄NO₅
Calculated(%) : C: 57.89, H: 3.40, N: 2.25.
Found(%) : C: 57.95, H: 3.61, N: 2.10.

### Examples 4 to 7

According to the same manner as that of Example 1, compounds shown in [Table 2] (Example 4: ethyl (2E)-3-[3-[7-chloro-6-methyl-3-]2-[[2-(trifluoromethyl)phenyl]amino]-2-oxoethyl]-2-oxo-2H-chromen-4-yl)phenyl]-2-propenoate, Example 5: ethyl (2E)-3-[3-[6-chloro-3-[2-[[4-fluoro-2-(trifluoromethyl)phenyl]amino]-2-oxoethyl]-7-methyl-2-oxo-2H-chromen-4-yl]phenyl]-2-propenoate, Example 6: ethyl (2E)-3-[3-[7-chloro-5-fluoro-3-[2-[[4-fluoro-2-(trifluoromethyl)phenyl]amino]-2-oxoethyl]-2-oxo-2H-chromen-4-yl]phenyl]-2-propenoate, Example 7: ethyl (2E)-3-[3-[7-chloro-3-[2-[[4-fluoro-2-(trifluoromethyl)phenyl]amino]-2-oxoethyl]-2-oxo-2H-chromen-4-yl]phenyl]-2-propenoate) were obtained.

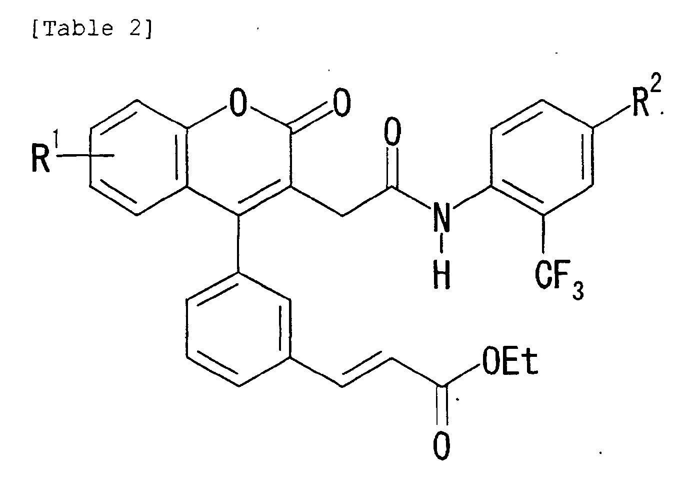

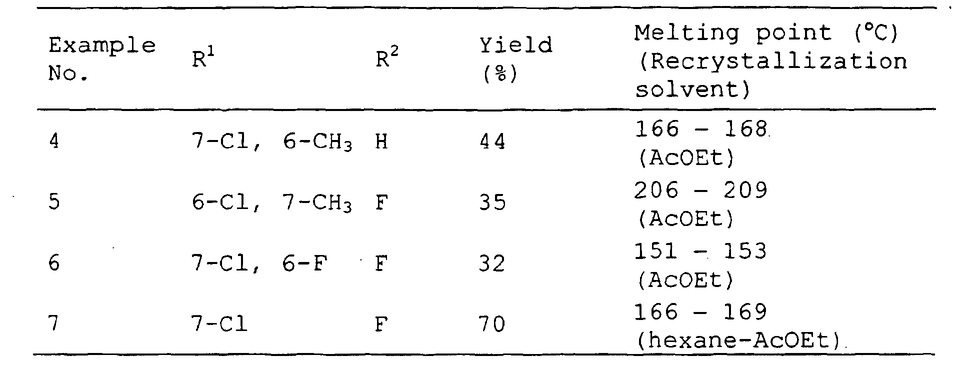

### Example 8

### Synthesis of ethyl 3-[5-[7-chloro-3-[2-[[4-fluoro-2-(trifluoromethyl)phenyl]amino]-2-oxoethyl]-6-methyl-2-oxo-2H-chromen-9-yl]phenyl]propionate

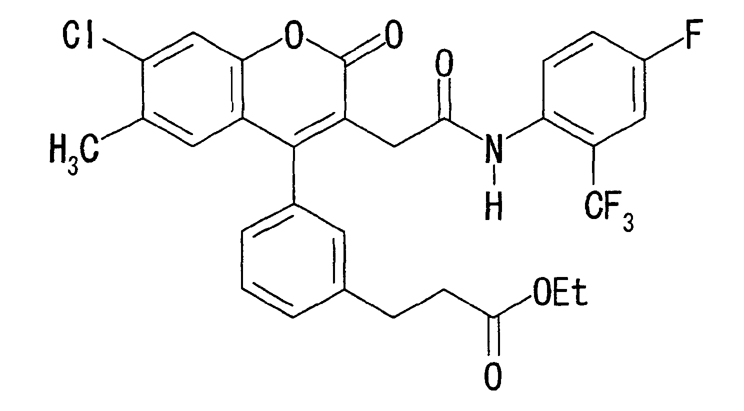

Raney nickel (about 1 g) was added to a mixed solution of ethyl 3-[5-[7-chloro-3-[2-[[4-fluoro-2-(trifluoromethyl)phenyl]amino]-2-oxoethyl]-6-methyl-2-oxo-2H-chromen-4-yl]phenyl]-2-propenoate (1.0 g) in THF (50 ml) and ethanol (50 ml), and the mixture was stirred for 1 day under hydrogen atmosphere. After completion of the reaction, the catalyst was filtered with Celite, and the filtrate was concentrated. The resulting residue was recrystallized from ethyl acetate to obtain the title compound (0.7 g, yield 69%).
mp: 121-123°C.
NMR (CDCl₃) δ: 1.21 (3H, t, J = 7.0 Hz), 2.30 (3H, s), 2.67 (2H, t, J = 7.5 Hz), 3.03 (2H, t, J = 7.5 Hz), 3.45 (2H, s), 4.11 (2H, q, J = 7.0 Hz), 6.91 (1H, s), 7 .2 - 7.5 (7H, m), 7.99 (1H, m), 8.18 (1H, brs).
Elemental analysis for C₃₀H₂₄ClF₄NO₅
Calculated(%): C: 61.08, H: 4.10, N: 2.37.
Found(%): C: 61.03, H: 4.16, N: 2.41.

### Examples 9 to 12

According to the same manner as that of Example 8, compounds shown in [Table 3] (Example 9: ethyl 3-[3-[7-chloro-6-methyl-3-[2-[[2-(trifluoromethyl)phenyl]amino]-2-oxoethyl]-2-oxo-2H-chromen-4-yl]phenyl]propionate, Example 10: ethyl 3-[3-[6-chloro-3-[2-[[4-fluoro-2-(trifluoromethyl)phenyl]amino]-2-oxoethyl]-7-methyl-2-oxo-2H-chromen-4-yl]phenyl]propionate, Example 11: ethyl 3-[3-[7-chloro-6-fluoro-3-[2-[[4-fluoro-2-(trifluoromethyl)phenyl]amino]-2-oxoethyl]-2-oxo-2H-chromen-4-yl]phenyl]propionate, Example 12: ethyl 3-[3-[7-chloro-3-[2-[[4-fluoro-2-(trifluoromethyl)phenyl]amino)-2-oxoethyl]-2-oxo-2H-chromen-4-yl]phenyl]propionate) were obtained.

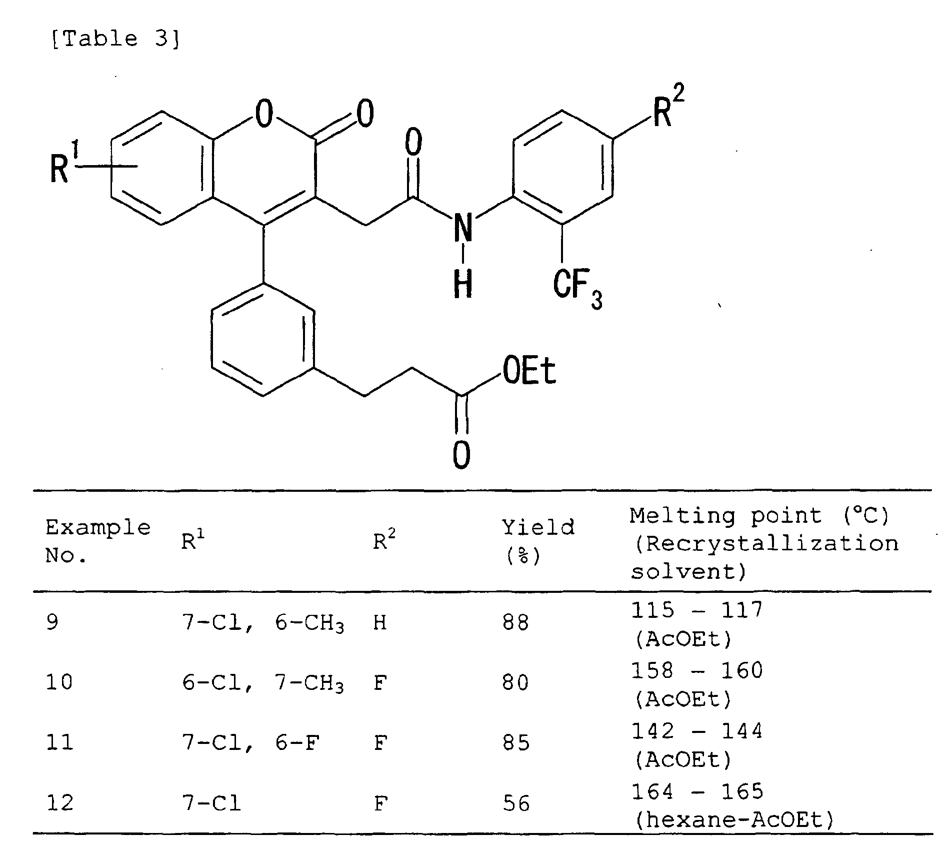

### Example 13

### Synthesis of ethyl 3-[3-[3-[2-[[4-fluoro-2-(trifluoromethyl)phenyl]amino]-2-oxoethyl]-2-oxo-2H-chromen-4-yl]phenyl]propionate

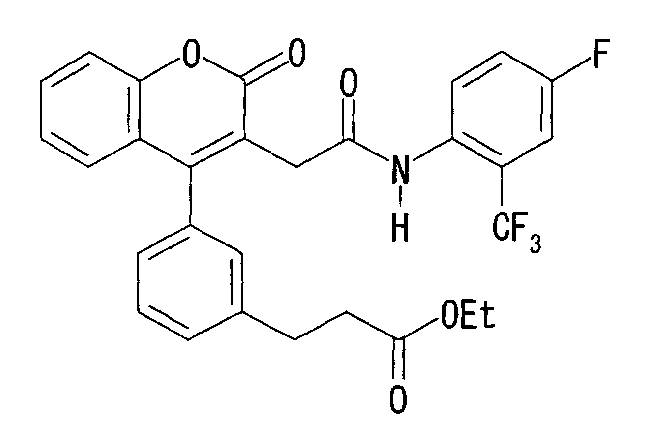

10% Palladium carbon (50% hydrous product, 0.30 g) was added to a mixed solution of ethyl (2E)-3-[3-[7-chloro-3-[2-[[4-fluoro-2-(trifluoromethyl)phenyl]amino]-2-oxoethyl]-2-oxo-2H-chromen-4-yl]phenyl]propenoate (2.54 g) in ethanol (50 ml) and N,N-dimethylformamide (20 ml), and the mixture was stirred at room temperature for 4 hours under hydrogen atmosphere. The reaction solution was filtered to remove the catalyst, the filtrate was concentrated under reduced pressure, and the resulting residue was purified by silica gel column chromatography (hexane:ethyl acetate:chloroform=4:1:2 to hexane:ethyl acetate=3:1) to obtain the title compound (1:29 g, yield 54%).
mp: 163-164°C.
NMR (CDCl₃) δ: 1.21 (3H, t, J = 7.2 Hz), 2.62-2.72 (2H, m) , 3.03 (2H, t, J = 7.5 Hz), 3.46 (1H, d, J = 14.4 Hz), 3.52 (1H, d, J = 14.4 Hz), 4.11 (2H, q, J = 7.2 Hz), 7.10 (1H, dd, J = 7.8, 1.5 Hz), 7.18-7.28 (4H, m), 7.32 (1H, dd, J = 8.4, 3.0 Hz), 7.35-7.38 (1H, m), 7.42-7.50 (2H, m), 7.52-7.58 (1H, m), 8.01 (1H, dd, J = 9.0, 5.1 Hz), 8.28 (1H, s). IR(KBr): 3256, 1723, 1713, 1665, 1526, 1431, 1321, 1123 cm⁻¹.
Elemental analysis for C₂₉H₂₃F₄NO₅
Calculated(%): C: 64.32, H: 4.28, N: 2.59.
Found(%): C: 64.32, H: 4.16, N: 2.30.

### Example 14

### Synthesis of 3-[5-[7-chloro-3-[2-[[4-fluoro-2-(trifluoromethyl)phenyl]amino]-2-oxoethyl]-6-methyl-2-oxo-2H-chromen-4-yl]phenyl]propionic acid

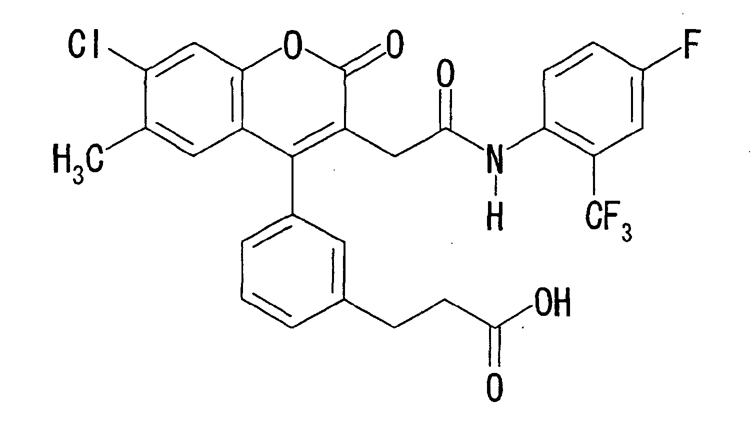

1N aqueous sodium hydroxide solution (5 ml) was added to a mixed solution of ethyl 3-[5-[3-[2-[[4-chloro-2-(trifluoromethyl)phenyl]amino]-2-oxoethyl]-6-methyl-2-oxo-2H-chromen-4-yl]phenyl]propionate. (580 mg) in THF (10 ml) and ethanol (5 ml), and the mixture was stirred overnight. The reaction solution was neutralized with 1N hydrochloric acid, extracted with ethyl acetate, washed with water, dried over magnesium sulfate, and concentrated. The resulting residue was recrystallized from ethyl acetate to obtain the title compound (500 mg, yield 91%).
mp: 212-214°C.
NMR (CDCl₃) δ: 2.29. (3H, s), 2.68 (2H, t, J = 7.2 Hz), 3.01 (2H, t, J = 7.2 Hz), 3.37 (1H, d, J = 13.5 Hz), 3.54 (1H, d, J = 13.5 Hz), 6.89 (1H, s), 7.1 - 7.5 (7H, m), 7.90 (1H, m), 8.46 (1H, brs).
Elemental analysis for C₂₈H₂₀ClF₄NO₅
Calculated(%): C: 59.85, H: 3.59, N: 2.49.
Found(%): C: 59.88, H: 3.88, N: 2.52.

### Examples 15 to 24

According to the same manner as that of Example 14, compounds shown in [Table 4] (Example 15: (2E)-3-[3-[7-chloro-6-methyl-2-oxo-3-(2-oxo-2-[[2-(trifluoromethyl)phenyl]amino]ethyl)-2H-chromen-4-yl]phenyl]-2-propenoic acid, Example 16: (2E)-3-[3-(6-chloro-3-(2-[[4-fluoro-2-(trifluoromethyl)phenyl]amino]-2-oxoethyl)-7-methyl-2-oxo-2H-chromen-4-yl]phenyl]-2-propenoic acid, Example 17: (2E)-3-[3-[7-chloro-6-fluoro-3-[2-[[4-fluoro-2-(trifluoromethyl)phenyl]amino]-2-oxoethyl]-2-oxo-2H-chromen-4-yl]phenyl]-2-propenoic acid, Example 18: (2E)-3-[3-[7-chloro-3-[2-[[4-fluoro-2-(trifluoromethyl)phenyl]amino]-2-oxoethyl]-2-oxo-2H-chromen-4-yl]phenyl]-2-propenoic acid, Example 19: (2E)-3-[5-[7-chloro-3-[2-[[4-chloro-2-(trifluoromethyl)phenyl]amino]-2-oxoethyl]-6-methyl-2-oxo-2H-chromen-4-yl]-2-fluorophenyl]-2-propenoic acid, Example 20: 3-[3-[7-chloro-6-methyl-2-oxo-3-(2-oxo-2-[[2-(trifluoromethyl)phenyl]amino]ethyl)-2H-chromen-4-yl]phenyl]propionic acid, Example 21: 3-[3-[6-chloro-3-(2-[[4-fluoro-2-(trifluoromethyl)phenyl]amino]-2-oxoethyl)-7-methyl-2-oxo-2H-chromen-4-yl]phenyl]propionic acid, Example 22: 3-[3-[7-chloro-6-fluoro-3-[2-[[4-fluoro-2-(trifluoromethyl)phenyl]amino]-2-oxoethyl]-2-oxo-2H-chromen-4-yl]phenyl]propionic acid, Example 23: 3-[3-[7-chloro-3-[2-[[4-fluoro-2-(trifluoromethyl)phenyl]amino]-2-oxoethyl]-2-oxo-2H-chromen-4-yl]phenyl]propionic acid, Example 24: 3-[3-[3-[2-[[4-fluoro-2-(trifluoromethyl)phenyl]amino]-2-oxoethyl]-2-oxo-2H-chromen-4-yl]phenyl]propionic acid) were obtained.

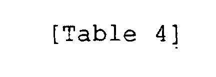

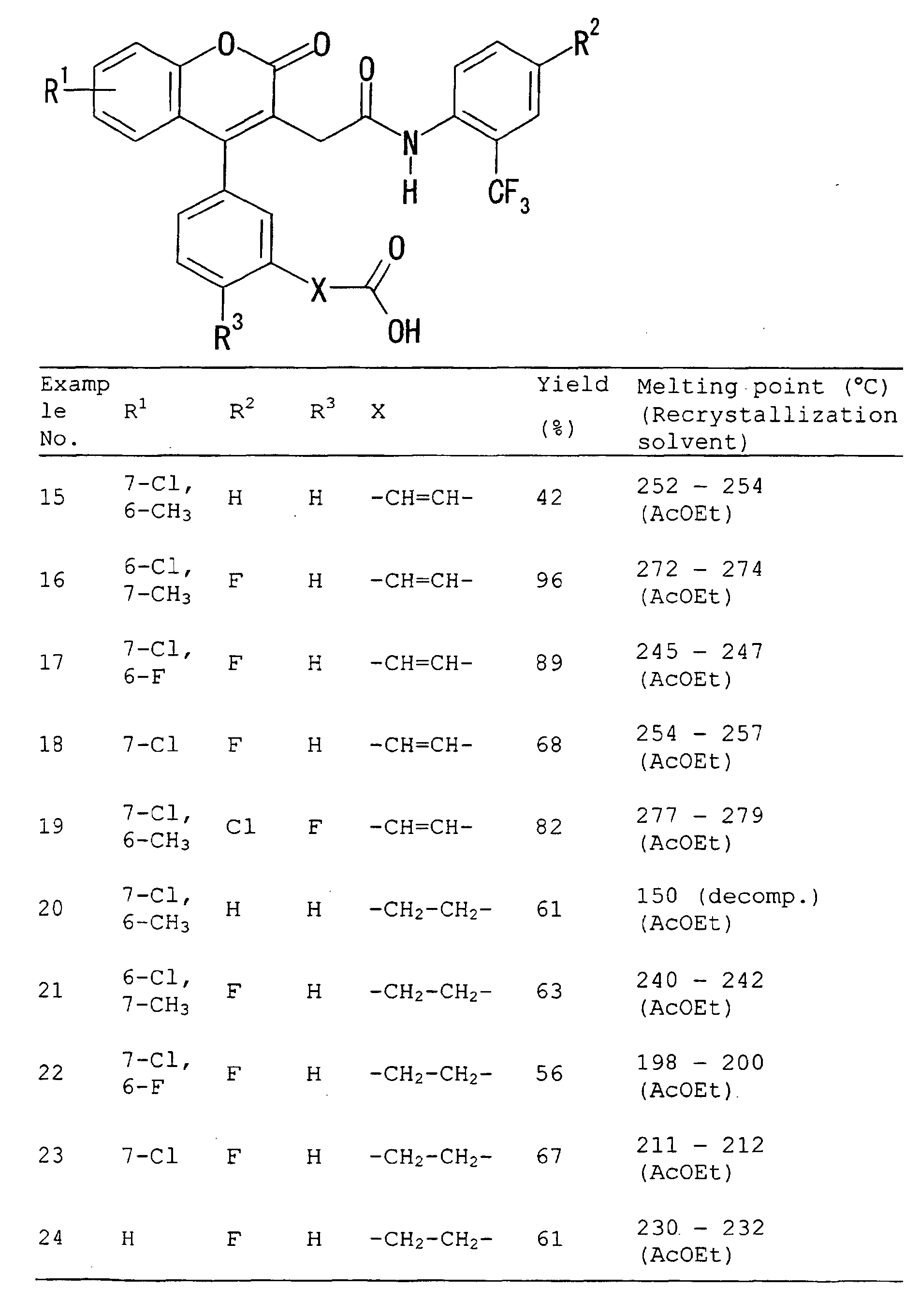

### Examples 25 to 40

According to the same manner as that of Example 3, compounds shown in [Table 5] were obtained.

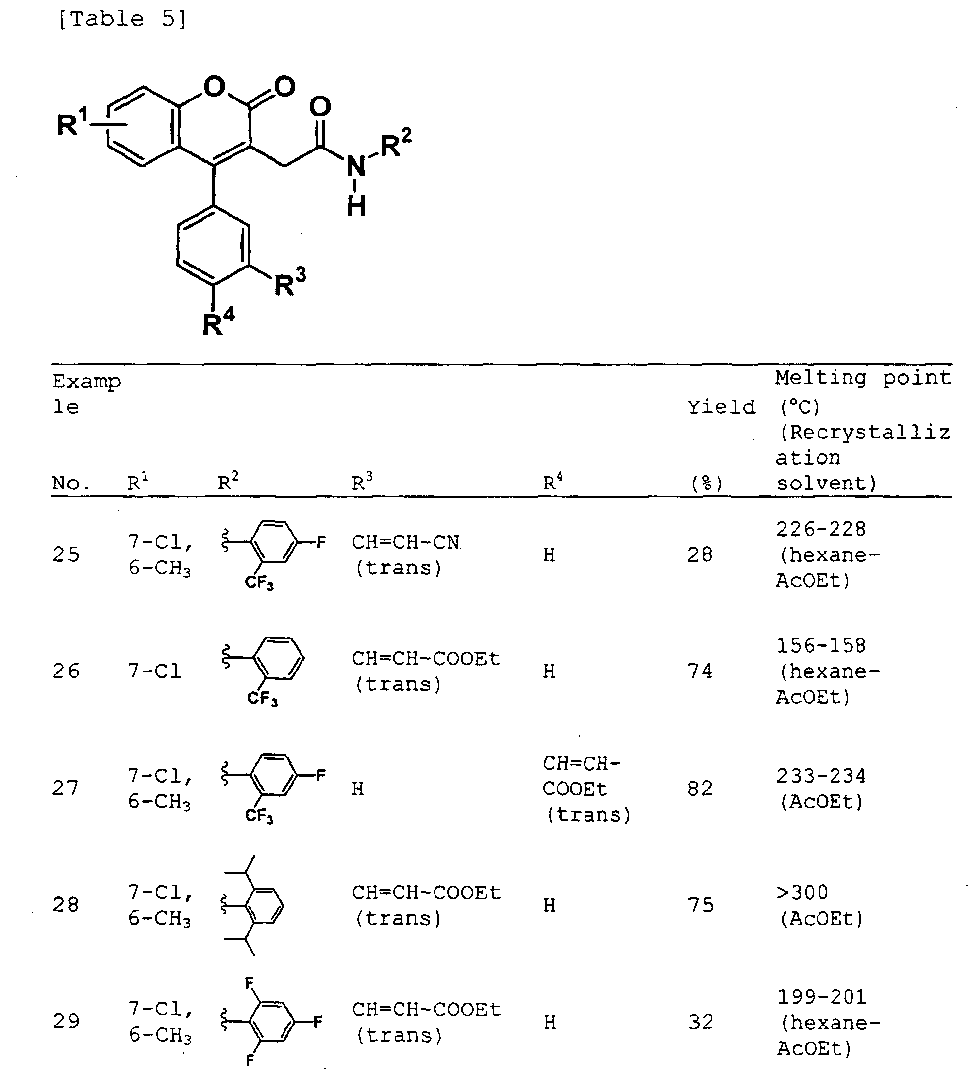

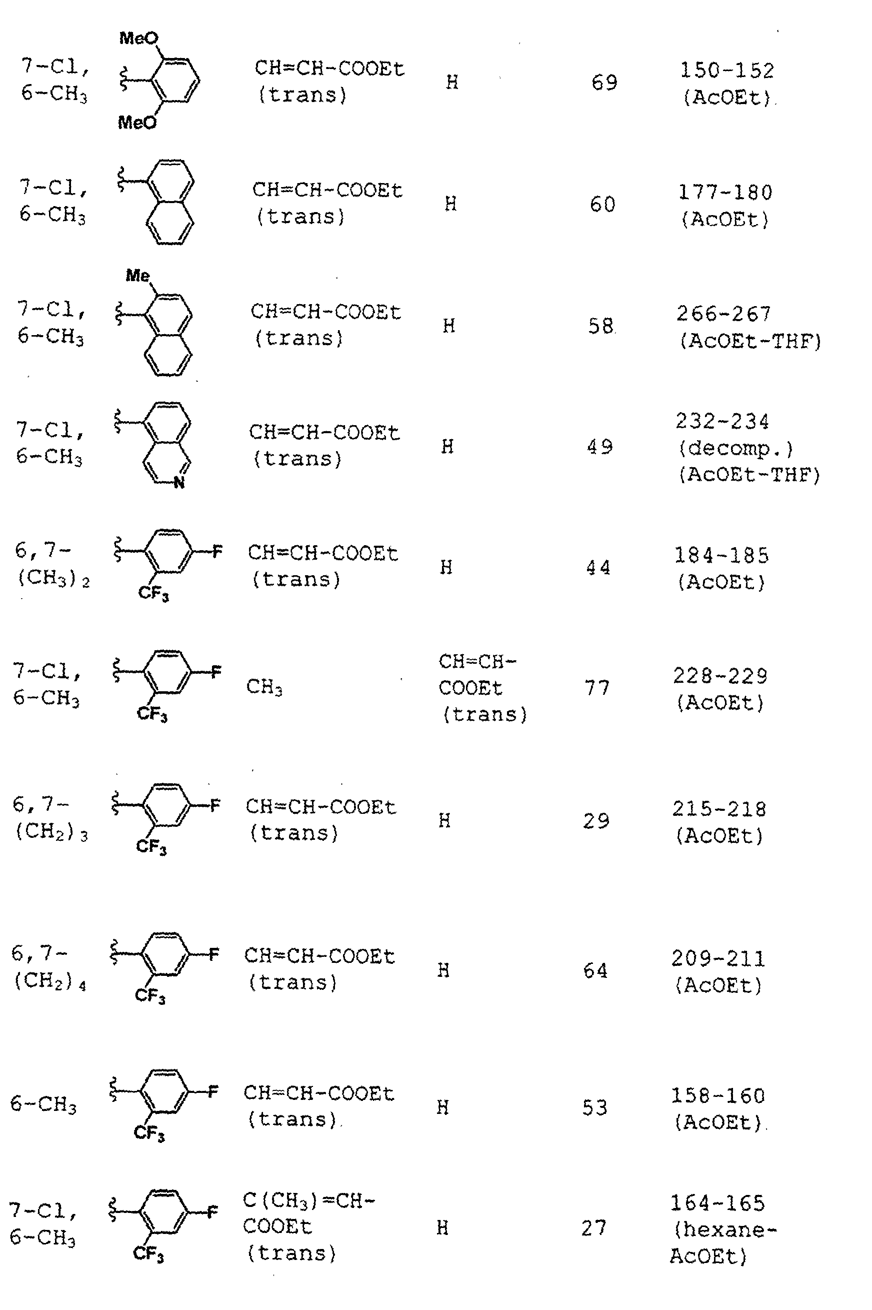

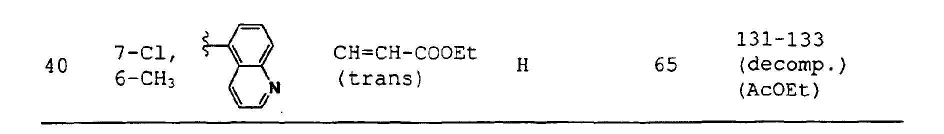

### Example 41

### 2-(7-chloro-6-methyl-2-oxo-4-{3-[(E)-2-(1H-tetrazol-5-yl)ethenyl]phenyl}-2H-chromen-3-yl)-N-[4-fluoro-2-(trifluoromethyl)phenyl]acetamide

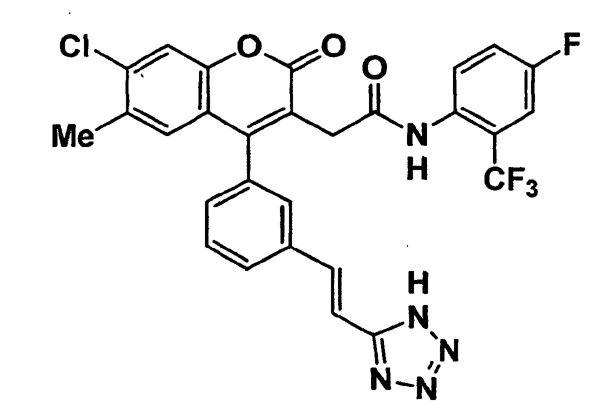

2-(7-chloro-4-{3-[(E)-2-cyanoethenyl]phenyl}-6-methyl-2-oxo-2H-chromen-3-yl)-N-[4-fluoro-2-(trifluoromethyl)phenyl]acetamide (1.24 g) and triethylamine hydrochloride (0.95 g) were suspended in toluene (10 ml), sodium azide (0.449 g) was added, and the mixture was stirred at 100°C for 2 hours under nitrogen atmosphere. Water was added to the reaction solution, acidified with 1N hydrochloric acid, and extracted with a mixed solvent of ethyl acetate-methanol. The extract was washed with an aqueous saturated sodium chloride solution, dried over magnesium sulfate, and the solvent was distilled off under reduced pressure. The resulting residue was purified by silica gel column chromatography (developing solvent:chloroform ∼1% chloroform/methanol ∼2%∼5%), and the resulting crystals were recrystallized from 2-propanol to obtain the title compound (0.832 g, yield 62%) as colorless crystals.
mp: 216-218°C
NMR (DMSO-d₆) δ: 2.26 (3H, s), 3.36 (2H, br s), 6.95 (1H, s), 7.35-7.76 (9H, m), 7.96 (1H, d, J = 8.0 Hz), 9.67 (1H, s), hidden (1H).
IR (KBr): 3119, 3044, 1688, 1321.
Elemental analysis for C₂₈H₁₈N₅O₃ClF₄
Calculated (%): C, 57.59; H, 3.11; N, 11.99.
Found (%): C, 57.33; H, 3.18; N, 11.92.

### Examples 42 to 44

According to the same manner as that of Example 41, compounds shown in [Table 6] were obtained.

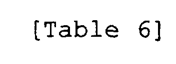

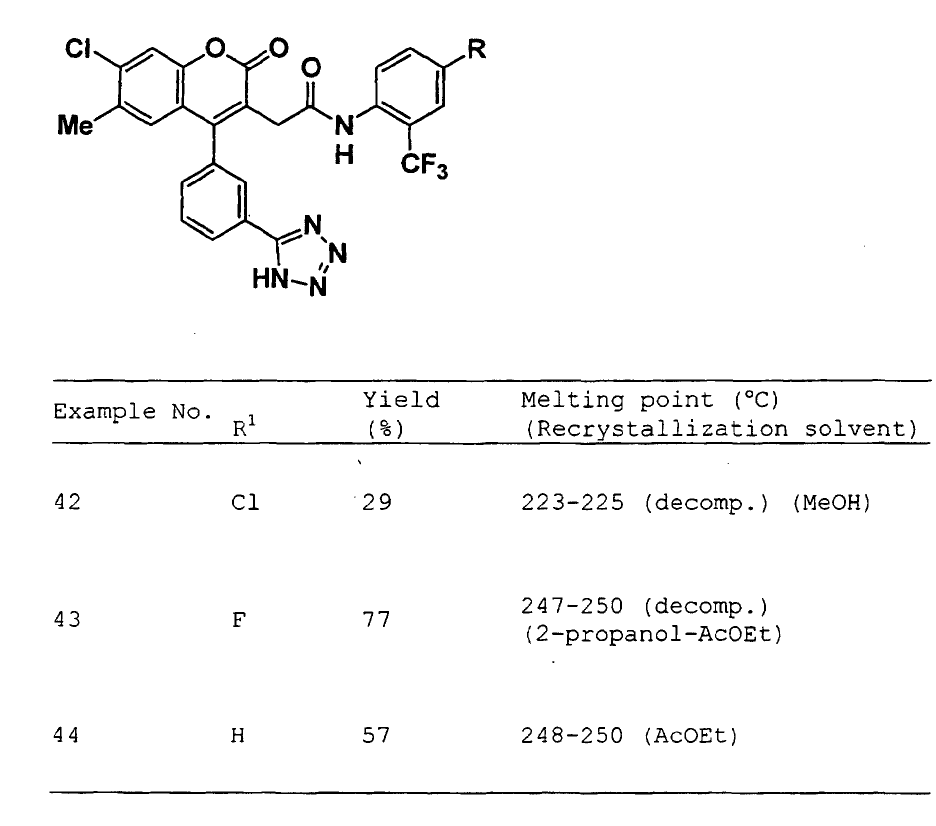

### Reference Example 9

### 2-[4-(4-bromophenyl)-7-chloro-6-methyl-2-oxo-2H-chromen-3-yl]-N-[4-fluoro-2-(trifluoromethyl)phenyl]acetamide

### (a) (4-bromophenyl)(4-chloro-2-hydroxy-5-methylphenyl)methanone

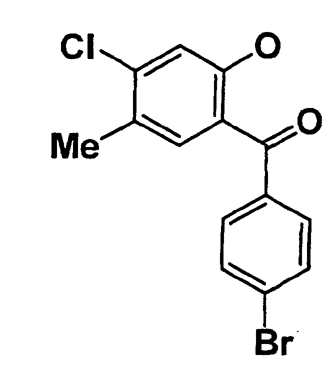

According to the same manner as that of Reference Example 1 (a), the title compound was obtained (yield:90%).
mp: 129-130°C (methanol).
NMR (CDCl₃) δ: 2.88 (3H, s), 7.12 (1H, s), 7.37 (1H, s), 7.54 (2H, d, J = 8.4 Hz), 7.68 (2H, d, J = 8.4 Hz), 11.76 (1H, s).
IR (KBr): 3088, 1630, 1586, 1333, 1003.
Elemental analysis for C₁₄H₁₀O₂BrCl
Calculated (%): C, 51.65; H, 3.10.
Found (%): C, 51.99; H, 2.95.

### (b) [4-(4-bromophenyl)-7-chloro-6-methyl-2-oxo-2H-chromen-3-yl]acetic acid

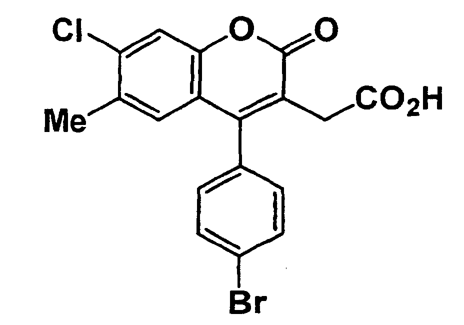

According to the same manner as that of Reference Example 1(b), the title compound was obtained (yield:85%).
mp: 238-239°C (2-propanol).
NMR (CDCl₃) δ: 2.29 (3H, s), 3.40 (2H, s), 6.83 (1H, s), 7.16 (2H, d, J = 8.4 Hz), 7.42 (1H, s), 7.72 (2H, d, J = 8.4 Hz), hidden (1H).
IR (KBr): 3011, 2959, 2930, 1721.
Elemental analysis for C₁₈H₁₂O₄BrCl
Calculated (%): C, 53.03; H, 2.97.
Found (%): C, 53.23; H, 2.91.

### (c) 2-[4-(4-bromophenyl)-7-chloro-6-methyl-2-oxo-2H-chromen-3-yl]-N-[4-fluoro-2-(trifluoromethyl)phenyl]acetamide

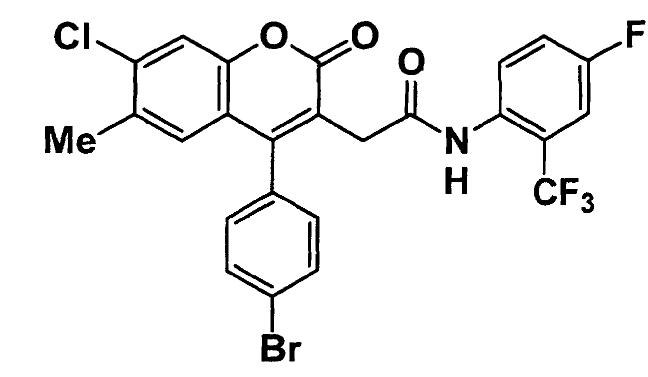

According to the same manner as that of Reference Example 1(b), the title compound was obtained (yield: 88%).
mp: 262-264°C (ethyl acetate-THF).
NMR (CDCl₃) δ: 2.29 (3H, s), 3.40 (2H, s), 6.83 (1H, s), 7.16 (2H, d, J = 8.4 Hz), 7.42 (1H, s), 7.72 (2H, d, J = 8.4 Hz), hidden (1H).
IR (KBr): 3241, 1717, 1659, 1535, 1186, 1127.
Elemental analysis for C₂₅H₁₅NO₃BrClF₄
Calculated (%): C, 52.80; H, 2.66; N, 2.46.
Found (%): C, 52.54; H, 2.62; N, 2.67.

### Example 45

### Methyl 4-(7-chloro-3-{2-[4-fluoro-2-(trifluoromethyl)anilino]-2-oxoethyl}-6-methyl-2-oxo-2H-chromen-4-yl)benzoate

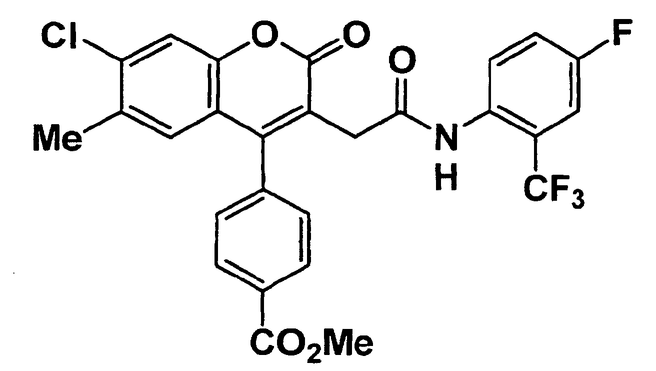

Palladium acetate (89.8 mg), 1,3-bis(diphenylphosphino)propane (206 mg) and triethylamine (1 ml) were added to a solution of 2-[4-(4-bromophenyl)-7-chloro-6-methyl-2-oxo-2H-chromen-3-yl]-N-[4-fluoro-2-(trifluoromethyl)phenyl]acetamide (1.17 g) in methanol (10 ml) and DMF (20 ml), and the mixture was stirred at 80°C for 2 days under carbon monoxide atmosphere (atmospheric pressure). The reaction solvent was concentrated to distill off under reduced pressure, water was added, and an organic material was extracted with a mixed solvent of chloroform and methanol. The extract was washed with an aqueous saturated sodium chloride solution and dried over magnesium sulfate, and the solvent was distilled off under reduced pressure. The resulting residue was purified by silica gel column chromatography (developing solvent: hexane-ethyl acetate-chloroform = 5:1:3∼4:1:1) to obtain the title compound (0.559 g, yield 49%).
mp: 202-204°C(hexane-ethyl acetate):
NMR (CDCl₃) δ: 2.28 (3H, s), 3.43 (2H, s), 3.98 (3H, s), 6.81 (1H, s), 7.21-7.27 (1H, m), 7.32 (1H, dd, J = 8.4, 3.0 Hz), 7.43-7.46 (3H, m), 7.97 (1H, dd, J = 9.0, 4.8 Hz), 8.17 (1H, s), 8.24 (2H, d, J = 8.7 Hz).
IR (KBr): 3258, 1719.
Elemental analysis for C₂₇H₁₈NO₅ClF₄
Calculated (%): C, 59.19; H, 3.31; N, 2.56.
Found (%): C, 59.09; H, 3.40; N, 2.60.

### Examples 46 to 48

According to the same manner as that of Example 45, compounds shown in [Table 7] were obtained.

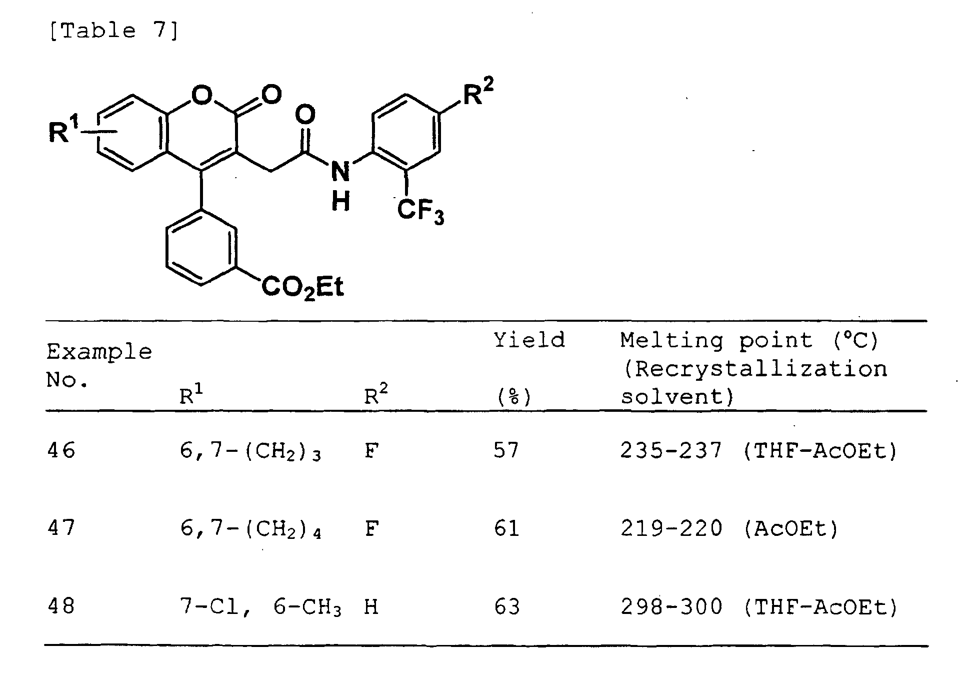

### Example 49

### Methyl {3-[7-chloro-3-(2-{[4-fluoro-2-(trifluoromethyl)phenyl]amino}-2-oxoethyl)-6-methyl-2-oxo-2H-chromen-4-yl]phenyl}acetate

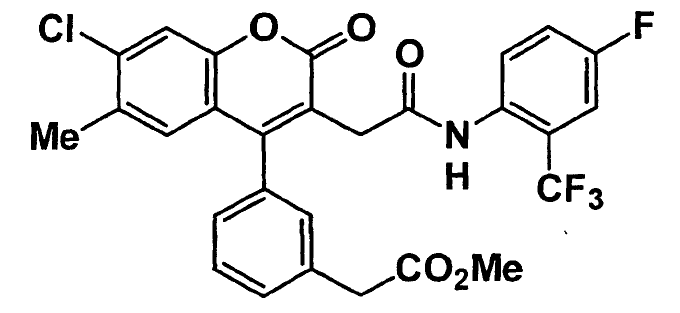

Oxalyl chloride (0.4 ml) was added to a mixed solution of 3-[7-chloro-3-(2-{[4-fluoro-2-(trifluoromethyl)phenyl]amino}-2-oxoethyl)-6-methyl-2-oxo-2H-chromen-4-yl]benzoic acid (2.0 g) in THF (100 ml) and DMF (3 drops), and the mixture was stirred at room temperature for 30 minutes. The reaction solution was concentrated under reduced pressure, the resulting residue was dissolved in THF (50 ml), and the solution was added dropwise to a solution of diazomethane in diethyl ether (50 ml) prepared from N-methyl-N'-nitroso-N-nitroguanidine (3.0 g). The mixture was stirred at room temperature for 2 hours, and the reaction solution was concentrated under reduced pressure. The resulting residue was dissolved in methanol (100 ml), and silver oxide (1.2 g) was added thereto. The mixture was then heated to reflux for 2 hours. Insolubles were filtered with Celite, and the filtrate was concentrated under reduced pressure. The resulting residue was purified by silica gel column chromatography (developing solvent: hexane-ethyl acetate = 3:1) to obtain the title compound (1 g). The resulting crude crystals were used in the next step without purification.
NMR (CDCl₃) δ: 2.30 (3H, s), 3.42 (1H, d, J = 10 Hz) , 3.47 (1H, d, J = 10 Hz), 3.70 (3H, s), 3.72 (3H, s), 6.94 (1H, s), 7.25-7.33 (4H, m), 7.43-7.52 (4H, m); 7.97 (1H, m), 8.16 (1H, brs).

### Example 50

### Methyl {3-[7-chloro-3-(2-{[4-fluoro-2-(trifluoromethyl)phenyl]amino}-2-oxoethyl)-6-methyl-2-oxo-2H-chromen-4-yl]phenyl}butanoate

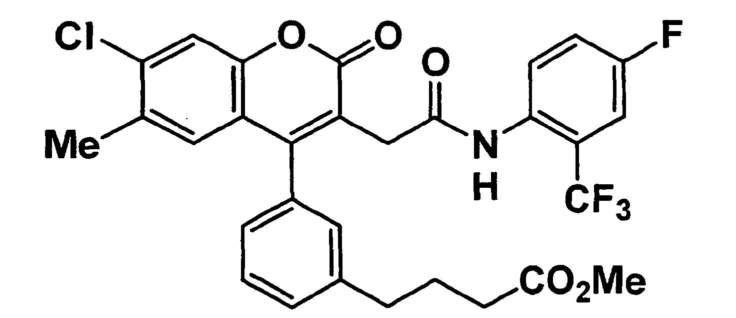

According to the same manner as that of Example 49, the title compound was obtained.
NMR (CDCl₃) δ: 2.00 (2H, m), 2.30 (3H, s), 2.36 (2H, t, J = 7.6 Hz), 2.74 (2H, t, J = 7.7 Hz), 3.46 (2H, s), 3.64 (3H, s), 6.92 (1H, s), 7.15-7.37 (5H, m), 7.48 (2H, m), 8.00 (1H, m), 8.19 (1H, brs).

### Reference Example 10

### 2-(7-chloro-6-methyl-2-oxo-4-{3-[(E)-3-oxo-1-propenyl]phenyl}-2H-chromen-3-yl)-N-[4-fluoro-2-(trifluoromethyl)phenyl]acetamide

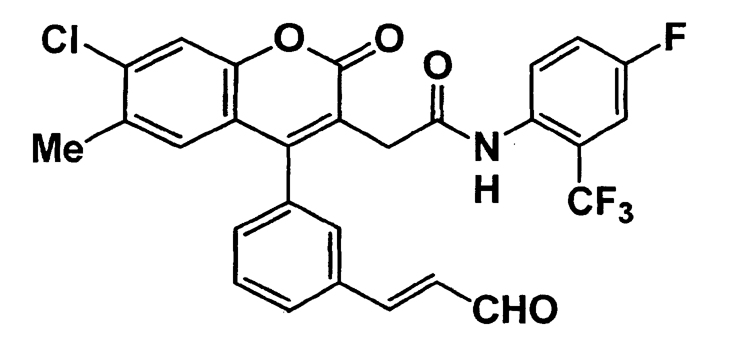

Acrolein diethylacetal (3.12 g), palladium acetate (0.225 g), tris (2-methylphenyl)phosphine (0.609 g) and sodium acetate (1.97 g) were added to a solution of 2-[4-(3-bromophenyl)-7-chloro-6-methyl-2-oxo-2H-chromen-3-yl]-N-[4-fluoro-2-(trifluoromethyl)phenyl]acetamide. (11.4. g) in DMF (60 ml), and the mixture was stirred at 120°C for 4 hours under nitrogen atmosphere. The reaction solvent was distilled off under reduced pressure, ethyl acetate and water were added thereto, and insolubles were removed by Celite filtration. The organic layer of the filtrate was washed with an aqueous saturated sodium chloride solution, dried over magnesium sulfate, and the solvent was distilled off under reduced pressure. The resulting residue was purified by silica gel column chromatography (developing solvent: hexane/ethyl acetate/chloroform = 5:1:3∼4:1:2) to obtain pale yellow crystals. The resulting crystals were dissolved in THF (100 ml), 1N hydrochloric acid (20 ml) was added, and the mixture was stirred at room temperature for 20 minutes. The reaction solvent was distilled off under reduced pressure, water was added, and an organic material was extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent: hexane-ethyl acetate-chloroform = 4:1:3∼3:1:2∼2:1:2) to obtain the title compound (2.70 g, yield 25%) as colorless crystals.
mp: 232-235°C (ethyl acetate).
NMR (CDCl₃) δ: 2.30 (3H, s), 3.42 (1H, d, J = 13.8 Hz), 3.47 (1H, d, J = 13. 8 Hz), 6.78 (1H, dd, J = 15. 9, 7. 5 Hz), 6.85 (1H, s), 7.21-7.27 (1H, m), 7.31 (1H, dd, J = 8. 7, 3.0 Hz), 7.42 (1H, d, J = 7.8 Hz), 7.46 (1H, s), 7.53 (1H, d, J = 15.9 Hz), 7.58 (1H, s), 7.64 (1H, t, J = 7.8 Hz), 7.76 (1H, d, J = 7.8 Hz) , 7.95 (1H, dd, J = 8.7, 4.8 Hz) , 8.23 (1H, s), 9.72 (1H, d, J = 7.5 Hz).
IR (KBr): 3231, 1719, 1680, 1657, 1134.
Elemental analysis for C₂₈H₁₈NO₄ClF₄
Calculated (%): C, 61.83; H, 3.34; N, 2.58.
Found (%): C, 61.81; H, 3.35; N, 2.50.

### Example 51

### Ethyl (2E,4E)-5-[3-(7-chloro-3-{2-[4-fluoro-2-(trifluoromethyl)anilino]-2-oxoethyl}-6-methyl-2-oxo-2H-chromen-4-yl)phenyl]-2,4-pentadienoate

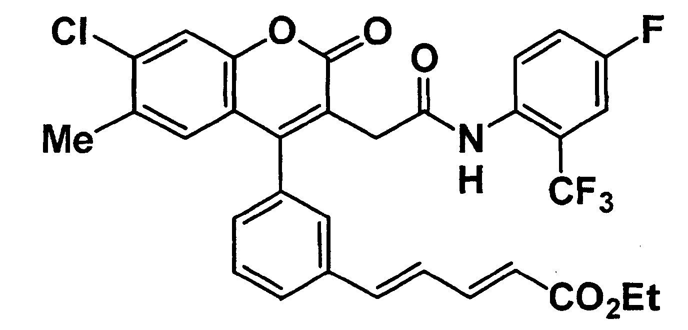

Under ice-cooling, sodium hydride (60%, oily)(0.132 g) was added in portions to a solution of triethyl phosphonoacetate (0.927 g) in THF (15 ml), and the mixture was stirred for 20 minutes under nitrogen atmosphere. To the reaction solution was added 2-(7-chloro-6-methyl-2-oxo-4-{3-[(E)-3-oxo-1-propenyl]phenyl)-2H-chromen-3-yl)-N-[4-fluoro-2-(trifluoromethyl)phenyl]acetamide (1.50 g), and the mixture was warmed to room temperature and stirred for 1 hour. Water was added to the reaction solution, and extracted with a mixed solvent of ethyl acetate-THF. The extract was washed with an aqueous saturated sodium chloride solution, dried over magnesium sulfate, and the solvent was distilled off under reduced pressure. The resulting residue was purified by silica gel column chromatography (developing solvent: hexane-ethyl acetate-chloroform = 4:1:3) to obtain the title compound (1.59g, yield 95%) as colorless crystals.
mp: 216-218°C (ethyl acetate).
NMR (CDCl₃) δ: 1.32 (3H, t, J = 7.2 Hz), 2.30 (3H, s), 3.46 (2H, s), 4.23 (2H, q, J = 7.2 Hz), 5.98 (1H, d, J = 15.4 Hz), 6.90-6.95 (3H, m), 7.19-7.66 (8H, m), 7.99 (1H, dd, J = 9.2, 5.0 Hz), 8.17 (1H, s).
IR (KBr): 3258, 2984, 1717, 1663, 1532, 1433, 1319, 1175, 1132.
Elemental analysis for C₃₂H₂₄NO₅ClF₄
Calculated (%): C, 62.60; H, 3.94; N, 2.28.
Found (%): C, 62.71; H, 3.90; N, 2.26.

### Example 52

### Ethyl (2E)-3-{3-[7-chloro-3-(2-{[9-fluoro-2-(trifluoromethyl)phenyl]amino}-2-oxoethyl-6-methyl-2-oxo-2H-chromen-4-yl]phenyl}-2-methylacrylate

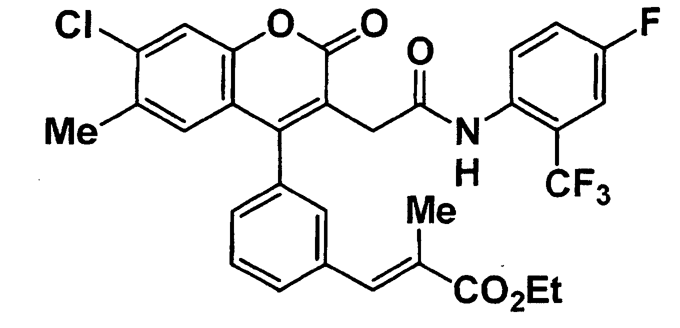

According to the same manner as that or Example 51, the title compound was obtained.
mp: 172-174°C (hexane-ethyl acetate).
NMR (CDCl₃) δ: 1.34 (3H, t, J = 7.2 Hz), 2.11 (3H, d, J = 1.5 Hz), 2.29 (3H, s), 3.42 (1H, d, J = 13.8 Hz), 3.52 (1H, d, J = 13.8 Hz), 4.27 (2H, q, J = 7.2 Hz), 6.87 (1H, s), 7.19-7.26 (1H, m), 7.29-7.32 (3H, m), 7.45 (1H, s), 7.55-7.62 (2H, m), 7.70 (1H, d, J = 1.5 Hz), 7.97 (1H, dd, J = 9.0, 5.2 Hz), 8.13 (1H, s).

### Examples 53 to 61

According to the same manner as that of Example 13, compounds shown in [Table 8] were obtained.

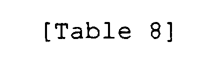

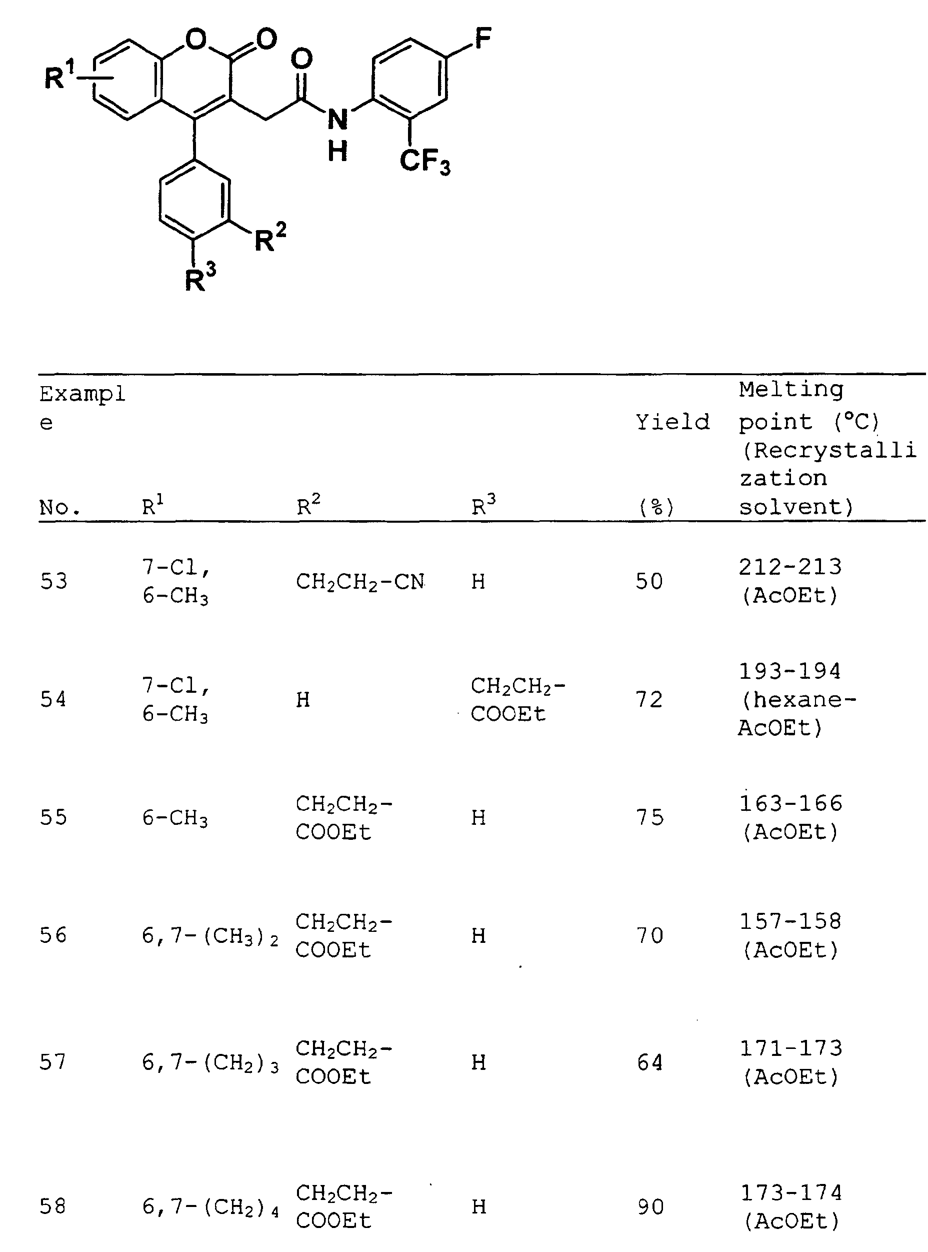

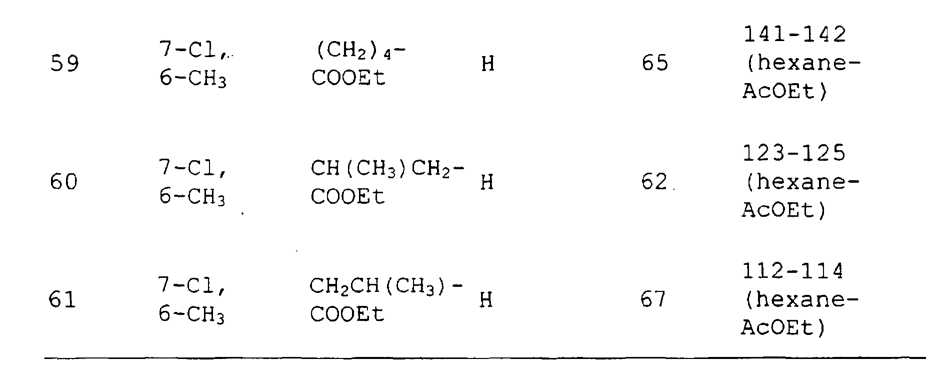

### Example 62

### Ethyl ({3-[7-chloro-3-(2-{[4-fluoro-2-(trifluoromethyl)phenyl]amino}-2-oxoethyl)-6-methyl-2-oxo-2H-chromen-4-yl]benzyl}amino)acetate

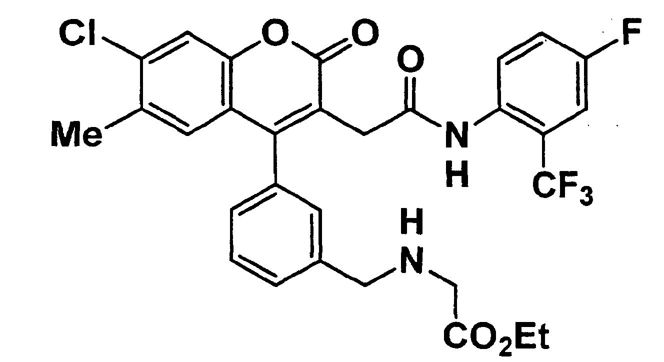

Glycine ethyl ester hydrochloride (0.307 g), molecular sieves (4A, beads) and triethylamine (0.307 ml) were added to a solution of 2-[7-chloro-4-(3-formylphenyl)-6-methyl-2-oxo-2H-chromen-3-yl]-N-[4-fluoro-2-(trifluoromethyl)phenyl]acetamide (1.04 g) in dichloromethane (30 ml), and the mixture was vigorously stirred at room temperature for 24 hours. The reaction solution was filtered with Celite, the filtrate was concentrated, water was added to the residue, and this was extracted with a mixed solvent of ethyl acetate-THF. The extract was washed with an aqueous saturated sodium chloride solution, dried over magnesium sulfate, and the solvent was distilled off under reduced pressure. The resulting residue was suspended in a mixed solvent of ethanol (10 ml) and THF (30 ml), 10% palladium-carbon (50% hydrous product) was added, and the mixture was stirred at room temperature for 20 minutes under hydrogen atmosphere (atmospheric pressure). After completion of the reaction, the catalyst was filtered, and the filtrate was concentrated under reduced pressure. Water was added to the residue, and this was extracted with a mixed solvent of ethyl acetate-THF. The extract was washed with an aqueous saturated sodium chloride solution, dried over sodium sulfate, and the solvent was distilled off under reduced pressure. The resulting residue was purified by silica gel column chromatography (developing solvent: hexane-ethyl acetate-chloroform = 2:1:2∼2:2:1∼only ethyl acetate) and reversed phase HPLC (mobile phase: water-acetonitrile containing 0.1%TFA) to obtain the title compound (0.49g, yield 41%) as colorless crystals.
mp: 153-155°C (hexane-ethyl acetate).
NMR (CDCl₃) δ: 1.26 (3H, t, J = 7.2 Hz), 2.29 (3H, s), 3.44 (4H, s), 3.90 (2H, s), 4.17 (2H, q, J = 7.2 Hz), 6.91 (1H, s), 7.19-7.32 (4H, m), 7.44 (1H, s), 7.48-7.54 (2H, m), 7.99 (1H, dd, J = 9.0, 4.8 Hz), 8.24 (1H, s), hidden (1H).
IR (KBr): 3252, 1717, 1663, 1528, 1433, 1319, 1173, 1128.
Elemental analysis for C₃₀H₂₅N₂O₅ClF₄
Calculated (%): C, 59.56; H, 4.17; N, 4.63.
Found (%): C, 59.37; H, 4.16; N, 4.61.

### Example 63

### Ethyl ({3-[7-chloro-3-(2-{[4-fluoro-2-(trifluoromethyl)phenyl]amino}-2-oxoethyl)-6-methyl-2-oxo-2H-chromen-4-yl]benzyl}thio) acetate

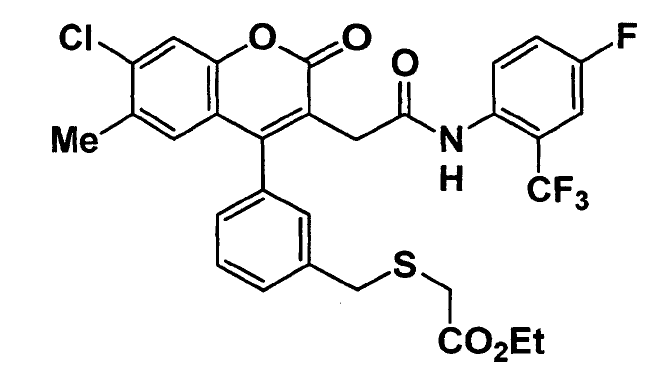

Under ice-cooling, sodium borohydride (0.145 g) was added in portions to a solution of 2-[7-chloro-4-(3-formylphenyl)-6-methyl-2-oxo-2H-chromen-3-yl]-N-[4-fluoro-2-(trifluoromethyl)phenyl]acetamide (3.96 g) in 1,2-dimethoxyethane (35 ml) and THF (35 ml), and the mixture was stirred for 30 minutes under nitrogen atmosphere. The reaction solution was treated with 1N hydrochloric acid, and extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent: hexane/ethyl acetate/chloroform = 2:1:2∼1:1:1) to obtain colorless crystals (3.61 g). The resulting crystals (1.56 g) were mixed with thionyl chloride (5 ml), and the mixture was stirred at room temperature for 2 hours. Pyridine (5 drops) was added to the reaction solution, followed by further stirring for 30 minutes. After completion of the reaction, excessive thionyl chloride was distilled off by concentration. The resulting residue was dissolved in DMF (10 ml), ethyl thioglycolate (0.658 ml) and cesium fluoride (0.775 g) were added, and the mixture was heated and stirred at 80°C for 2 hours. Water was added to the reaction solution, and extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent: hexane-ethyl acetate-chloroform = 5:1:3∼4:1:2) to obtain the title compound (1.64 g, yield 80%) as pale yellow crystals.
mp: 106-108°C (hexane-ethyl acetate)
NMR (CDCl₃) δ: 1.25 (3H, t, J = 7.2 Hz), 2.29 (3H, s), 3.09 (1H, d, J = 14.7 Hz), 3.15 (1H, d, J = 14.7 Hz), 3.43 (1H, d, J = 14.1 Hz), 3.51 (1H, d, J = 14.1 Hz), 3.90 (2H, s), 4.13 (2H, q, J = 7.2 Hz), 6.91 (1H, s), 7.19-7.32 (4H, m), 7.44 (1H, s), 7.51-7.53 (2H, m), 7.97 (1H, dd, J = 8.7, 5.1 Hz), 8.20 (1H, s).
IR (KBr): 3265, 3029, 1721, 1607, 1522, 1433, 1321, 1171, 1134.
Elemental analysis for C₃₀H₂₄NO₅ClF₄S
Calculated (%): C, 57.93; H, 3.89; N, 2.25.
Found (%): C, 57.92; H, 3.97; N, 2.23.

### Example 64

### Ethyl ({3-[7-chloro-3-(2-{[4-fluoro-2-(trifluoromethyl)phenyl]amino}-2-oxoethyl)-6-methyl-2-oxo-2H-chromen-4-yl]benzyl}sulfonyl)acetate

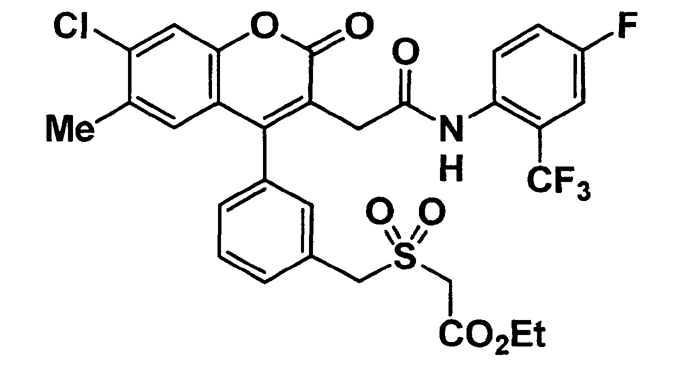

Under ice-cooling, m-chloroperbenzoic acid (0.705 g) was added in portions to a solution of ethyl {3-[7-chloro-3-(2-{[4-fluoro-2-(trifluoromethyl)phenyl]amino}-2-oxoethyl)-6-methyl-2-oxo-2H-chromen-4-yl]benzyl}thio)acetate (0.732 g) in dichloromethane (35 ml). The mixture was warmed to room temperature and then stirred for 18 hours. The reaction solution was treated with an aqueous sodium thiosulfate solution, and extracted with a mixed solvent of ethyl acetate-THE. The extract was washed successively with an aqueous saturated sodium bicarbonate solution and an aqueous saturated sodium chloride solution, dried over magnesium sulfate, and the solvent was distilled off under reduced pressure to obtain the title compound (0.66 g, yield 86%) as pale yellow crystals.
mp: 230-231°C(ethyl acetate)
NMR (CDCl₃) δ: 1.29 (3H, t, J = 7.2 Hz), 2.29 (3H, s), 3.42 (1H, d, J = 13.5 Hz) , 3.51 (1H, d, J = 13.5 Hz), 3.82 (1H, d, J = 15.0 Hz), 4.06 (1H, d, J = 15.0 Hz), 4.16-4.28 (2H, m), 4.51 (1H, d, J = 14.1 Hz), 4.73 (1H, d, J = 14.1 Hz), 6.89 (1H, s), 7.18-7.24 (1H, m), 7.31 (1H, dd, J = 8.4, 3.0 Hz), 7.41 (1H, dt, J = 7.5, 1.5 Hz), 7.45 (1H, s), 7.48 (1H,. t, J = 1.5 Hz), 7.63 (1H, t, J = 7.5 Hz), 7.68 (1H, dt, J = 7.5, 1.5 Hz), 7.89 (1H, dd, J = 9.0, 4.8 Hz), 8.31 (1H, s)
IR (KBr): 3264, 1732, 1663, 1532, 1433, 1319, 1177, 1127.
Elemental analysis for C₃₀H₂₄NO₇ClF₄S· 0.5H₂O
Calculated (%): C, 54.34; H, 3.80; N, 2.11.
Found (%): C, 54.54; H, 3.75; N, 2.11.

### Examples 65 to 97

According to the same manner as that of Example 14, compound shown in [Table 9] were obtained.

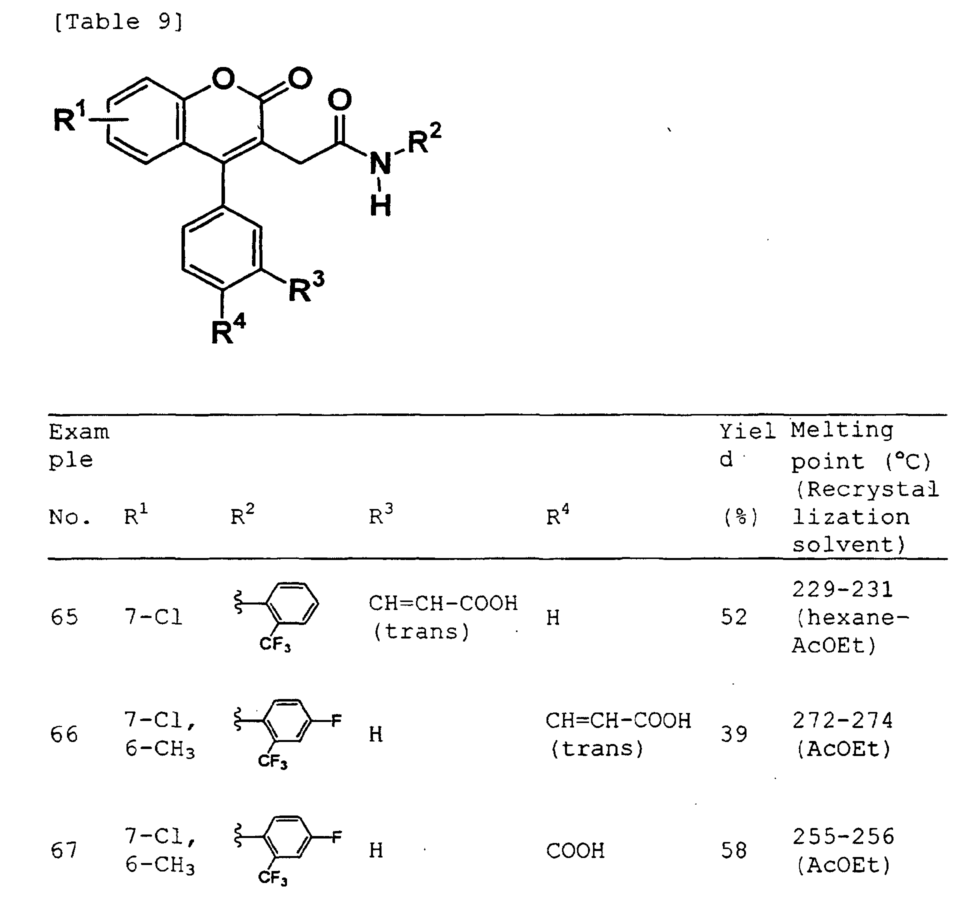

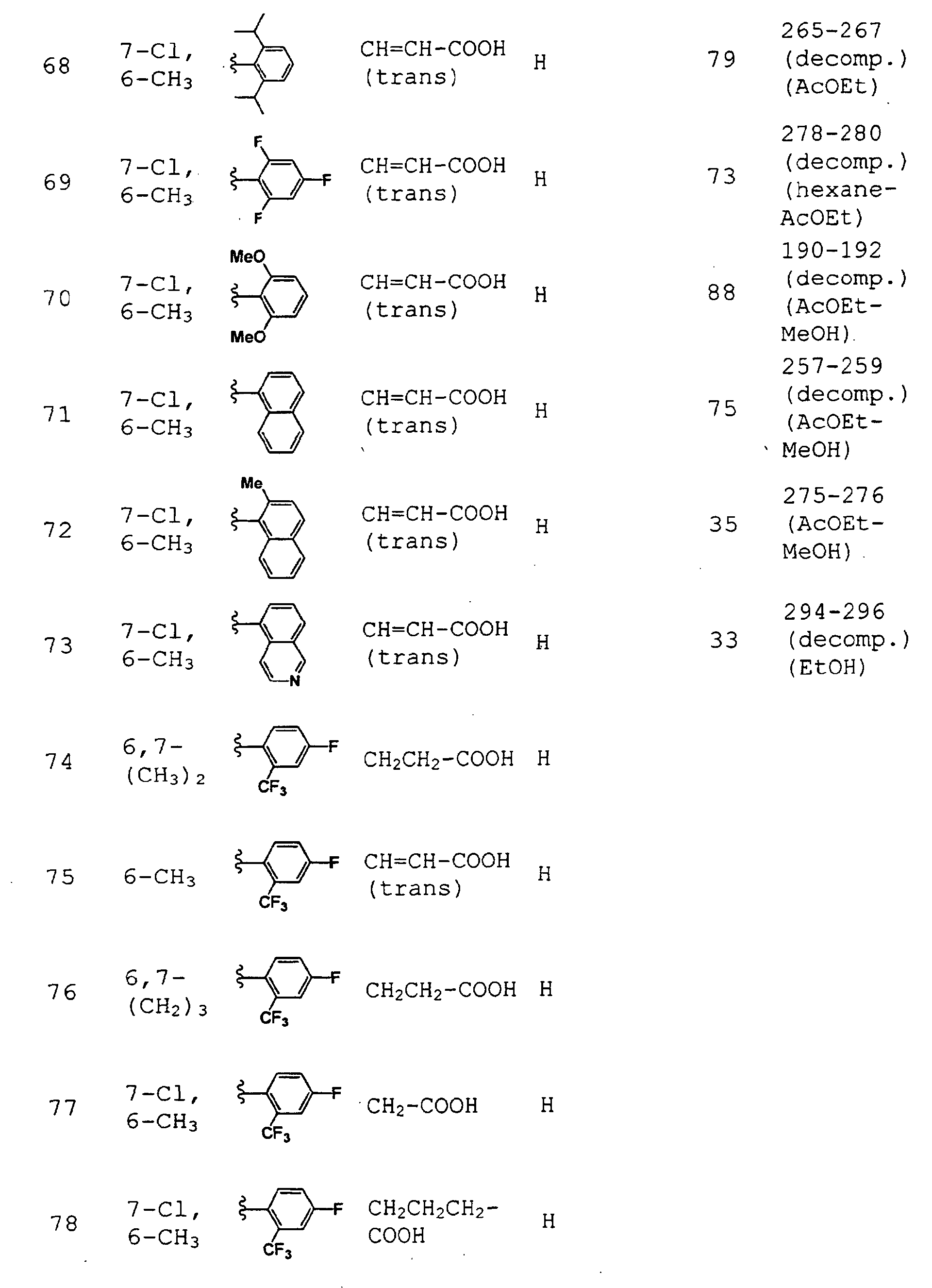

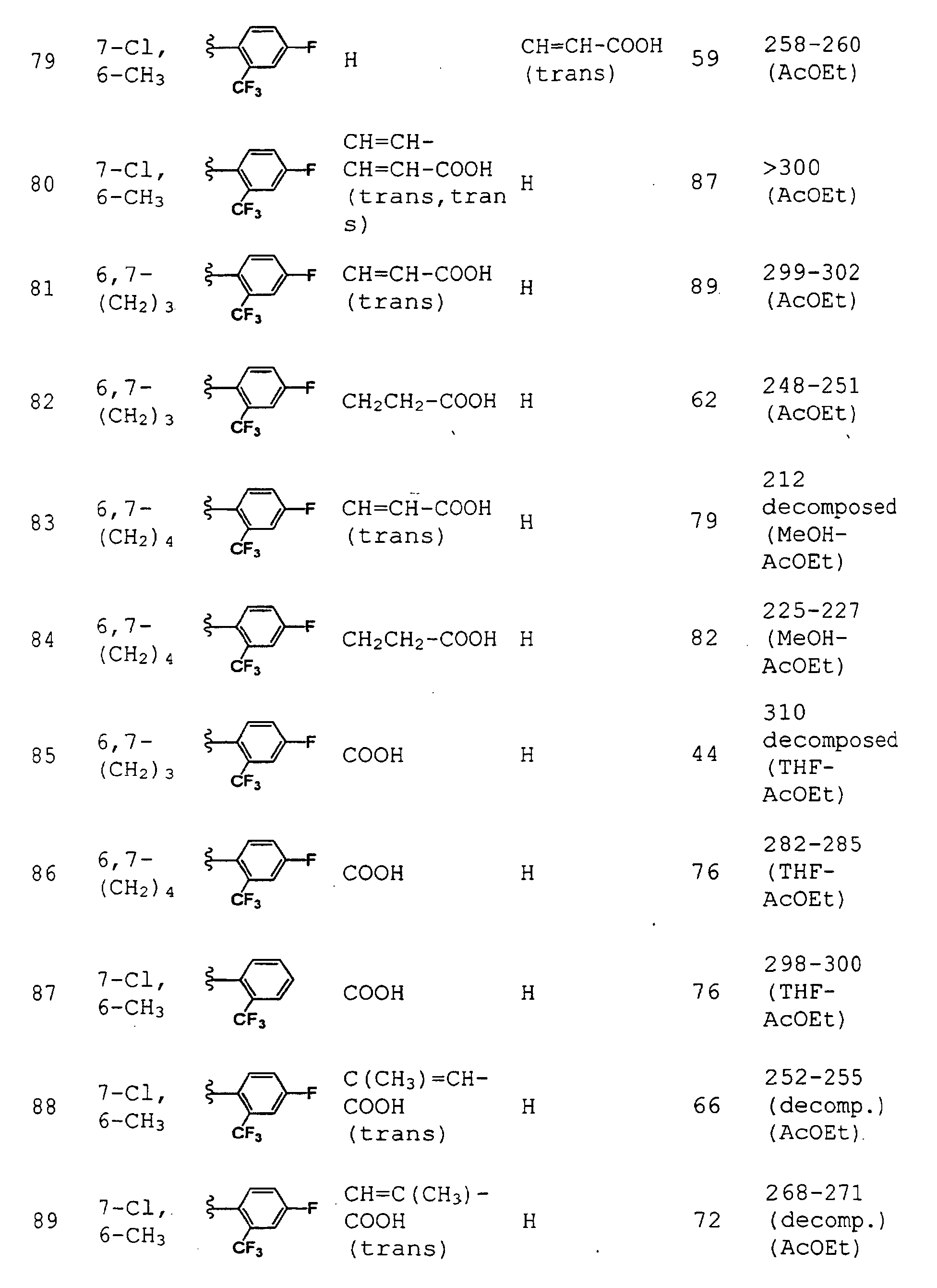

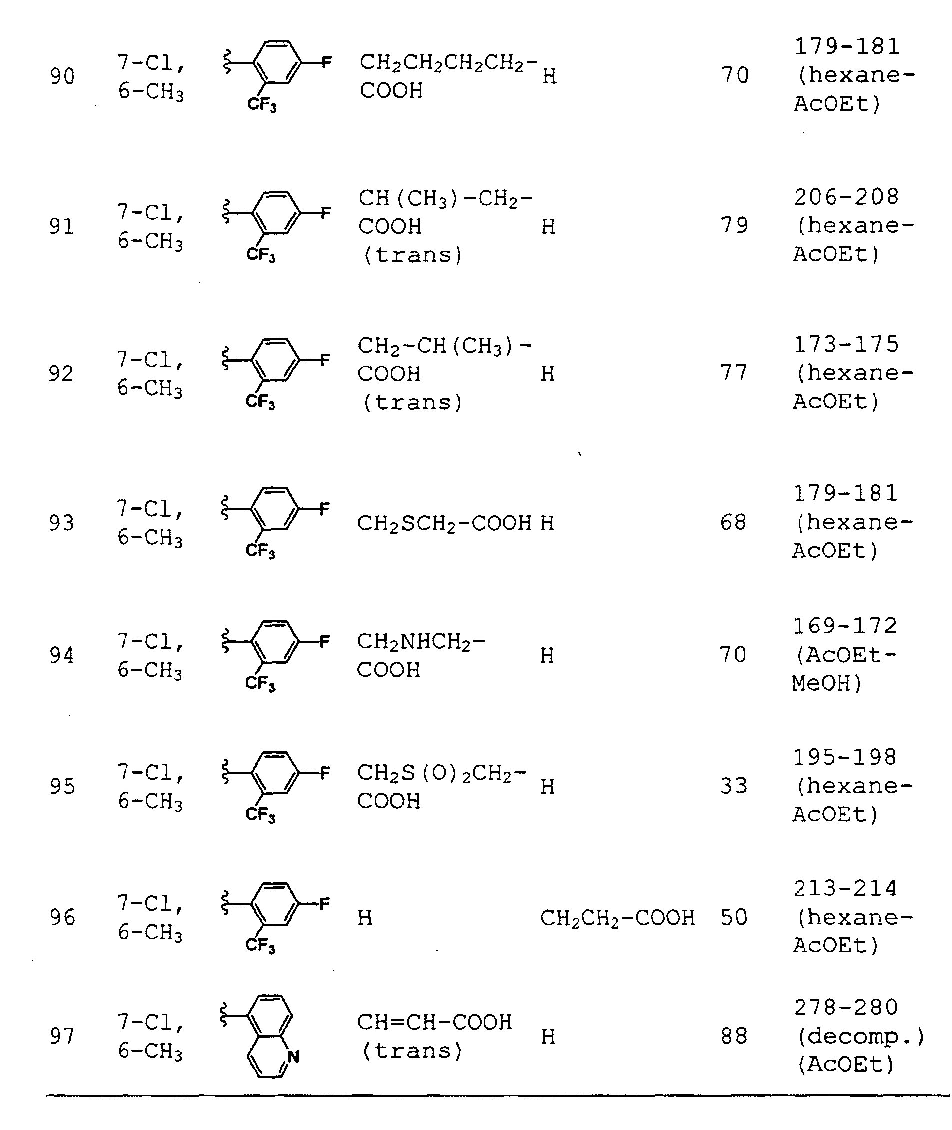

### Example 98

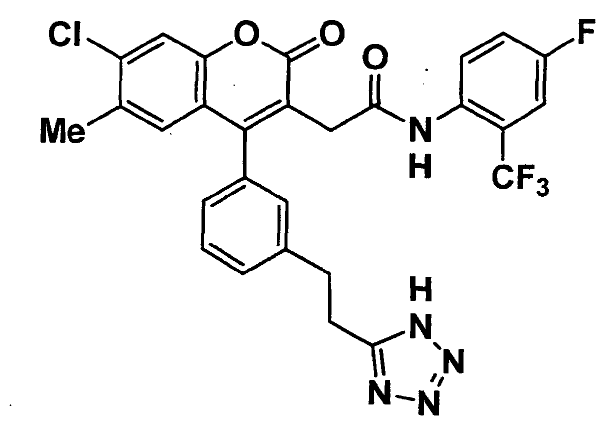

According to the same manner as that of Example 41, the title compound was obtained.
mp: 244-246°C (ethyl acetate).
NMR (CDCl₃) δ: 2.30 (3H, s), 3.05 (1H, d, J = 13.5 Hz), 3.10-3.30 (3H, m), 3.42-3.52 (1H, m), 3.95 (1H, d, J = 13.5 Hz), 6.84 (1H, s), 6.93-7.01 (2H, m), 7.11 (1H, s), 7.23-7.31 (2H, m), 7.38 (1H, dd, J = 8.7, 3.0 Hz), 7.51 (1H, s), 7.72 (1H, dd, J = 8.7, 4.8 Hz), 9.20 (1H, s).
Elemental analysis for C₂₈H₂₀N₅O₃ClF₄
Calculated (%): C, 57.40; H, 3.44; N, 11.95.
Found (%): C, 57.18; H, 3.48; N, 11.74.

### Reference Example 11

### 2-(4-{3-[amino(hydroxyimino)methyl]phenyl}-7-chloro-6-methyl-2-oxo-2H-chromen-3-yl]-N-[4-fluoro-2-(trifluoromethyl)phenyl]acetamide

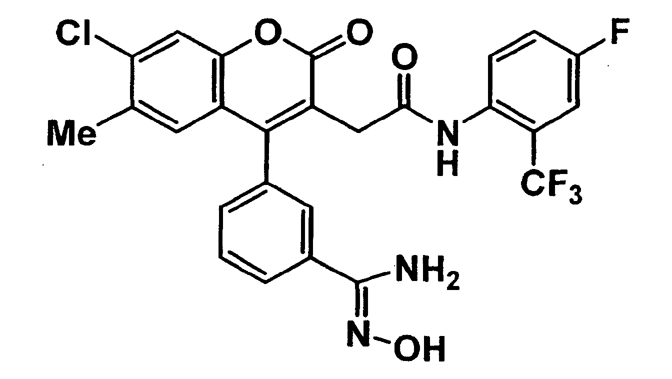

Tetrakis(triphenylphosphine)palladium (0) (1.16 g) and zinc cyanate (2.47 g) were added to a solution of 2-[4-(3-bromophenyl)-7-chloro-6-methyl-2-oxo-2H-chromen-3-yl]-N-[4-fluoro-2-(trifluoromethyl)phenyl]acetamide (11.4 g) in DMF (50 ml), and the mixture was stirred at 80°C for 6 hours under nitrogen atmosphere. The reaction solution was treated with a 28% aqueous ammonia solution, and extracted with a mixed solvent of toluene-THF. The extract was washed with an aqueous saturated sodium chloride sodium, dried over sodium sulfate, and the solvent was distilled off under reduced pressure. The resulting residue was purified by silica gel column chromatography (developing solvent: hexane-ethyl acetate-chloroform=3:1:3∼3:1:2∼2:1:1) to obtain colorless crystals (8.48 g). Hydroxylamine hydrochloride (3.47 g) was suspended in DMSO (20 ml), and triethylamine (6.97 ml) was added. Precipitated triethylamine hydrochloride was filtered and washed with THF. THF in the filtrate was distilled off under reduced pressure, crystals (5.15 g) obtained in the above were added, and the mixture was stirred at 75°C for 7 hours. After completion of the reaction, water was added, and this was extracted with ethyl acetate. The extract was washed with an aqueous saturated sodium chloride solution, dried over sodium sulfate, and the solvent was distilled off under reduced pressure to obtain the title compound (5.08 g).
mp: 220-222°C(decomp)(ethyl acetate)
NMR (CDCl₃) δ: 2.31 (3H, s), 3.17 (1H, d, J = 13.5 Hz), 3.69 (1H, d, J = 13.5 Hz), 5.41 (2H, s), 7.03 (1H, s), 7.21-7.27 (1H, m), 7.31-7.37 (2H, m), 7.47 (1H, s), 7.58 (1H, t, J = 7.8 Hz), 7.66 (1H, t, J = 1.8 Hz), 7.86-7.93 (2H, m), 8.79 (1H, s).
IR (KBr): 3272, 3050, 1779, 1746.

### Example 99

### 2-{7-chloro-6-methyl-2-oxo-4-[3-(5-oxo-4,5-dihydro-1,2,4-oxadiazol-3-yl)phenyl]-2H-chromen-3-yl}-N-[4-fluoro-2-(trifluoromethyl)phenyl]acetamide

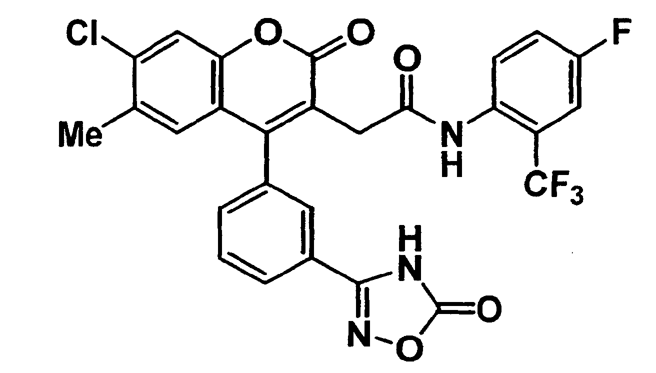

2-(4-{3-[amino(hydroxyimino)methyl)phenyl}-7-chloro-6-methyl-2-oxo-2H-chromen-3-yl)-N-[4-fluoro-2-(trifluoromethyl)phenyl]acetamide (0.822 g) was suspended in THF (10 ml), 1,1'-carbonyldiimidazole (0.36 g) and DBU (0.897 ml) were added, and the mixture was stirred at room temperature for 3 hours. After completion of the reaction, water was added. The reaction mixture was adjusted to pH 2 with 1N hydrochloric acid and then extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent: hexane-ethyl acetate-chloroform = 1:2:1∼ethyl acetate-chloroform = 1:1) to obtain the title compound (0.302 g, yield 350).
mp: 285-287°C (2-prpoanol-ethyl acetate).
NMR (DMSO-d₆) δ: 2.26 (3H, s), 3.33-3.42 (2H, m), 6.91 (1H, s), 7.39 (1H, dd, J = 8.7, 5.4 Hz), 7.48-7.62 (3H, m), 7.71 (1H, s), 7.78-7.83 (2H, m), 8.00 (1H, d, J = 7.8 Hz), 9.65 (1H, s), hidden (1H).
IR (KBr): 3272, 3050, 1779, 1746.
Elemental analysis for C₂₇H₁₆N₃O₅ClF₄
Calculated (%): C, 56.51; H, 2.81; N, 7.32.
Found (%): C, 56.49; H, 2.95; N, 7.10.

### Examples 100 to 101

According to the same manner as that of Example 99, compounds shown in [Table 10] were obtained.

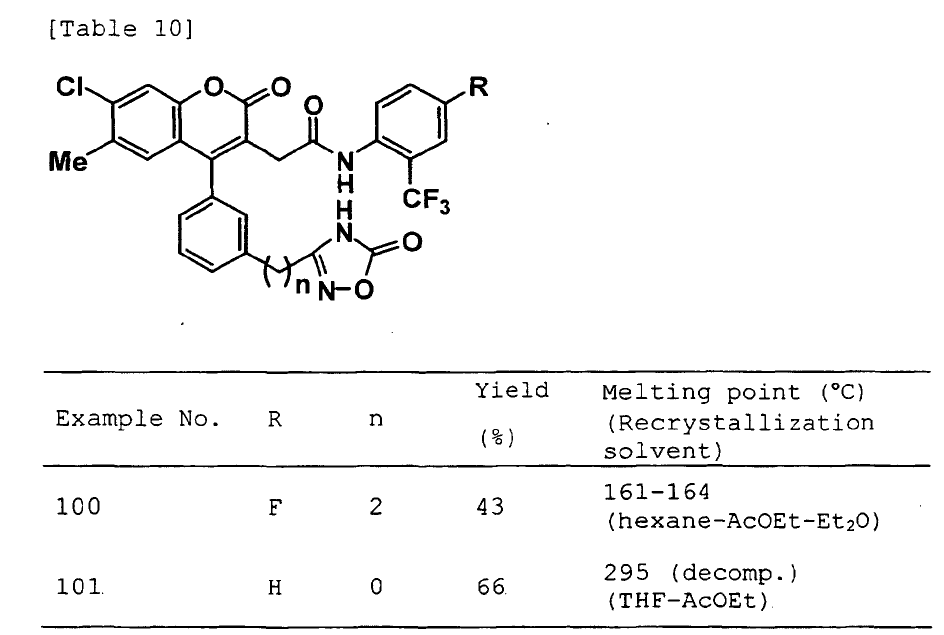

### Example 102

### 2-{7-chloro-6-methyl-2-oxo-4-[3-(5-thioxo-4,5-dihydro-1,2,4-oxadiazol-3-yl)phenyl]-2H-chromen-3-yl}-N-[4-fluoro-2-(trifluoromethyl)phenyl]acetamide

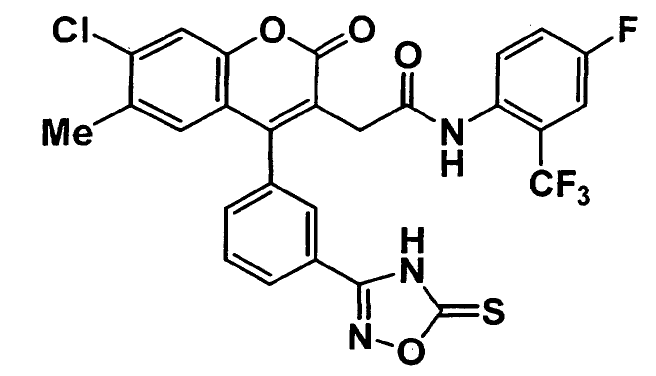

2-(4-{3-[Amino(hydroxyimino)methyl]phenyl}-7-chloro-6-methyl-2-oxo-2H-chromen-3-yl)-[4-fluoro-2-(trifluoromethyl)phenyl]acetamide (0.822 g) was suspended in THF (10 ml), 1,1'-thiocarbonyldiimidazole (0.365 g) and DBU (0.897 ml) were added, and the mixture was stirred at room temperature for 3 hours. After completion of the reaction, water was added. The reaction mixture was adjusted to pH 2 with 1N hydrochloric acid and then extracted with a mixed solvent of ethyl acetate-THF. The extract was washed with an aqueous saturated sodium chloride solution, dried over magnesium sulfate, and the solvent was distilled off under reduced pressure. The resulting residue was purified by silica gel column chromatography (developing solvent: ethyl acetate/chloroform = 1:1) to obtain the title compound (0.463 g, yield 52%).
mp: 178-180°C(hexane-ethyl acetate).
NMR (CDCl₃) δ: 2.35 (3H, s), 3.06 (1H, d, J = 13.5 Hz), 3.89 (1H, d, J = 13.5 Hz), 7.11 (1H, s), 7.24-7.36 (2H, m), 7.50 (1H, s), 7.58 (1H, d, J = 7.8 Hz), 7.73-7.78 (2H, m), 7.97 (1H, dd, J = 9.0, 5.1 Hz), 8.09 (1H, t, J = 7.8 Hz), 9.18 (1H, s), hidden (1H).
IR (KBr): 3248, 1705, 1493, 1433, 1321.
Elemental analysis for C₂₇H₁₆N₃O₄ClF₄S·0.5H₂O
Calculated (%): C, 54.14; H, 2.86; N, 7.02.
Found (%): C, 54.35; H, 2.87; N, 6.68.

### Example 103

### 2-{7-chloro-6-methyl-2-oxo-4-[3-(5-thioxo-4,5-dihydro-1,2,4-oxadiazol-3-yl)phenyl]-2H-chromen-3-yl}-N-[2-(trifluoromethyl)phenyl]acetamide

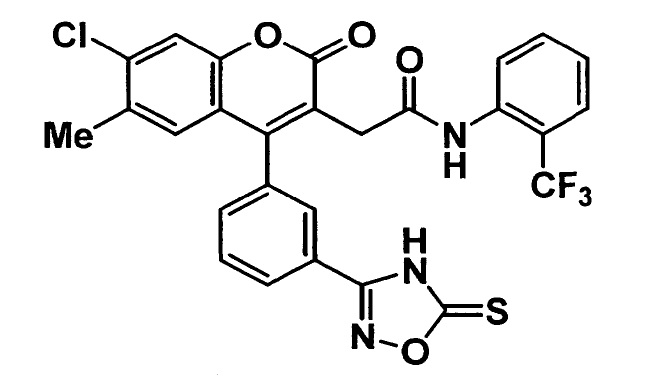

According to the same manner as that of Example 102, the title compound (yield 33%) was obtained.
mp: 202°C(decomp.)(THF-ethyl acetate).
NMR (DMSO-d₆) δ: 2.27 (3H, s), 3.38 (2H, br), 6.93 (1H, s), 7.38 (1H, d, J = 8.2 Hz), 7.45 (1H, d, J = 7.6 Hz), 7.58-7.75 (4H, m), 7.81 (1H, d, J = 7.6 Hz), 7.87 (1H, br d, J = 1.4 Hz), 8.10 (1H, d, J = 8.0 Hz), 9.62 (1H, s).

### Example 104

### 2-{7-chloro-6-methyl-2-oxo-4-[3-(5-oxo-4,5-dihydro-1,2,4-thiadiazol-3-yl)phenyl]-2H-chromen-3-yl}-N-[4-fluoro-2-(trifluoromethyl)phenyl]acetamide

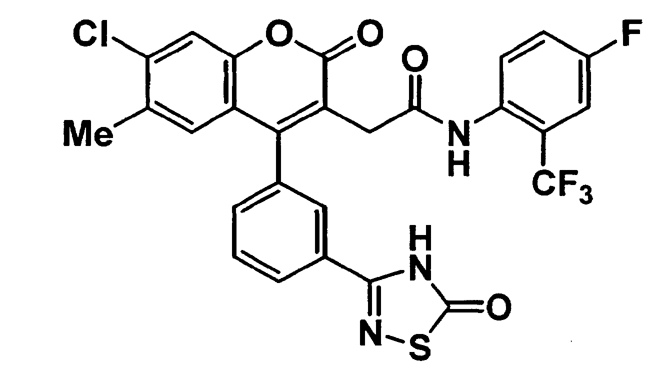

2-(4-{3-[Amino(hydroxyimino)methyl]phenyl}-7-choloro-6-methyl-2-oxo-2H-chromen-3-yl)-N-[4-fluoro-2-(trifluoromethyl)phenyl]acetamide (0.822 g) was suspended in THF (10 ml), 1,1'-thiocarbonyldiimidazole (0.365 g) was added, and the mixture was stirred at room temperature for 3 hours. After completion of the reaction, water was added, and extracted with a mixed solvent of ethyl acetate-THF. The extract was washed with an aqueous saturated sodium chloride solution, dried over magnesium sulfate, and the solvent was distilled off under reduced pressure. The resulting residue was suspended in THF (15 ml), boron trifluoride diethyl ether complex (0.76 ml) was added, and the mixture was stirred at room temperature for 12 hours. After completion of the reaction, water was added, acidified with 1N hydrochloric acid and extracted with a mixed solvent of ethyl acetate-THF. The extract was washed with an aqueous saturated sodium chloride solution, dried over magnesium sulfate, and the solvent was distilled off under reduced pressure. The resulting residue was recrystallized from chloroform-methanol to obtain the title compound (0.801 g, yield 68%)
mp: 269-271°C(decomp.).
NMR (DMSO-d₆) δ: 2.26 (3H, s), 3.28-3.42 (2H,m), 6.91 (1H, s), 7.38 (1H, dd, J = 9.0, 5.1 Hz), 7.48-7.55 (2H, m), 7.60 (1H, dd, J = 9.0, 3.0 Hz), 7.72 (1H, s), 7.76 (1H, t, J = 7.8 Hz), 7.92 (1H, s), 8.12 (1H, t, J = 7.8 Hz), 9.64 (1H, s), hidden (1H).
IR (KBr): 3264, 1746, 1686, 1659, 1541, 1327, 1109.
Elemental analysis for C₂₇H₁₆N₃O₄ClF₄S·0.5H₂O
Calculated (%): C, 54.14; H, 2.86; N, 7.02.
Found (%) : C, 53.85; H, 2.81; N, 6.91.

### Example 105

### 2-{7-chloro-6-methyl-2-oxo-4-[3-{2-(5-oxo-4,5-dihydro-1,2,4-thiadiazol-3-yl)ethyl}phenyl]-2H-chromen-3-yl}-N-[2-(trifluoromethyl) phenyl] acetamide

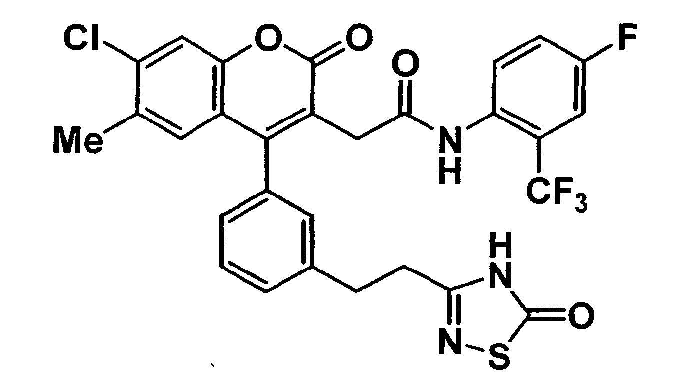

According to the same manner as that of Example 104, the title compound (yield 23%) was obtained.
mp: 181°C (decomp.)(hexane-ethyl acetate).
NMR (DMSO-d₆) δ: 2.29 (3H, s), 2.76-3.13 (5H, m), 3.90 (1H, d, J = 13.5 Hz), 6.86 (1H, s), 7.04-7.09 (2H, m), 7.17 (1H, d, J = 7.8 Hz), 7.24-7.29 (1H, m), 7.35 (1H, dd, J = 8.4, 3.0 Hz), 7.42 (1H, t, J = 7.8 Hz), 7.49 (1H, s), 7.76 (1H, dd, J = 9.0,. 5.1 Hz), 9.07 (1H, s), 10.96 (1H, s).

### Reference Example 12

### Ethyl [6-bromomethyl-4-(3-bromophenyl)-7-chloro-2-oxo-2H-chromen-3-yl]acetate

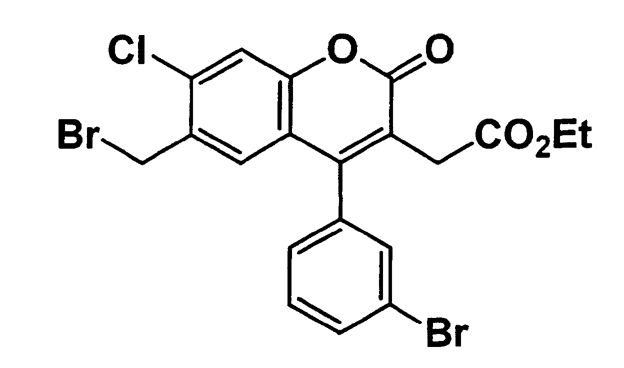

NBS (4.9 g) and AIBN (190 mg) were added to a suspension of ethyl [4-(3-bromophenyl)-7-chloro-6-methyl-2-oxo-2H-chromen-3-yl]acetate (10 g) in t-butyl acetate (70 ml), and the mixture was stirred at 80°C for 2 hours. Ethyl acetate was added to the reaction solution, this was washed successively with an aqueous saturated sodium hydrogencarbonate solution and water, dried over magnesium sulfate (MgSO₄), and the solvent was distilled off under reduced pressure. The resulting residue was washed with isopropyl ether to obtain crude crystals (9.8 g:83%) of the title compound. The resulting crude crystals were used in the next step without further purification.
NMR (CDCl₃) δ: 1.25 (3H, t, J = 7.4 Hz), 3.66 (2H, s), 4.16 (2H, q, J = 7.4 Hz), 4.49 (2H, m), 7.04 (1H, s), 7.27 (2H, m), 7.44 (2H, m), 7.72 (1H, m).

### Reference Example 13

### [6-Bromomethyl-4-(3-bromophenyl)-7-chloro-2-oxo-2H-chromen-3-yl]acetic acid

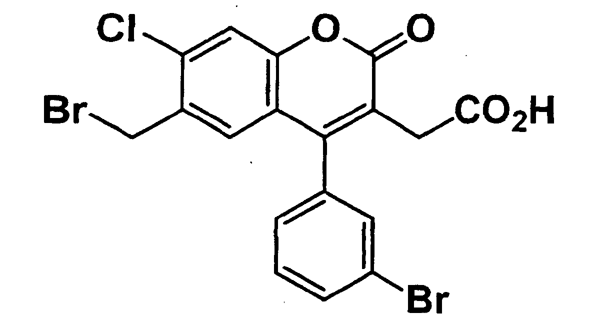

Acetic acid (150 ml) and concentrated hydrochloric acid (75 ml) were added to ethyl [6-bromomethyl-4-(3-bromophenyl)-7-chloro-2-oxo-2H-chromen-3-yl]acetate, and the mixture was heated to reflux for 2 hours. The reaction solution was concentrated under reduced pressure. The resulting residue was washed with water and dried to obtain crude crystals (8.5 g:92%) of the title compound. The resulting crude crystals were used in the next step without further purification.
NMR (CDCl₃) δ: 3.42 (2H, m), 4.61 (2H, m), 7.07 (1H, s), 7.27 (2H, m), 7.49 (2H, m), 7.71 (1H, m).

### Reference Example 14

### 2-{4-(3-bromophenyl)-7-chloro-2-oxo-6-[(4-phenylpiperazin-1-yl)methyl]-2H-chromen-3-yl}-N-[4-fluoro-2-(trifluoromethyl)phenyl]acetamide

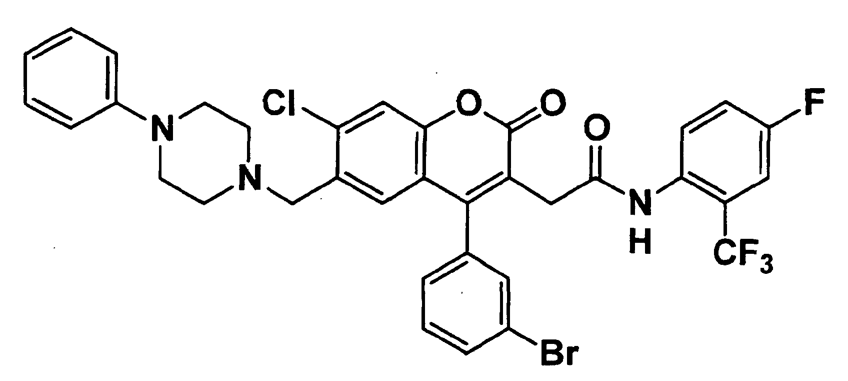

Oxalyl chloride (1.2 ml) was added to a mixed solution of [6-bromomethyl-4-(3-bromophenyl)-7-chloro-2-oxo-2H-chromen-3-yl]acetic acid (5.5 g) in THF (100 ml) and DMF (3 drops), and the mixture was stirred at room temperature for 30 minutes. The reaction solution was concentrated under reduced pressure, and the resulting residue was dissolved in THF (100 ml). 2-Amino-5-fluorobenzotrifluoride (1.6 ml) was added, and the mixture was stirred overnight. Water was added to the reaction solution, and extracted with ethyl acetate. The extract was washed with water, dried over magnesium sulfate, and the solvent was distilled off under reduced pressure. The resulting residue was dissolved in THF (100 ml), phenylpiperazine (2.0 ml) was added, and the mixture was heated to reflux overnight. Water was added to the reaction solution, and extracted with ethyl acetate. The extract was washed with water, dried over magnesium sulfate; and the solvent was distilled off under reduced pressure to obtain crude crystals (3.2 g, yield 39%) of the title compound. The resulting crude crystals were used in the next step without further purification.
NMR (CDCl₃) δ: 2.60 (4H, m), 3.09 (4H, m), 3.48 (2H, m), 3.60 (2H, s), 6.90 (4H, m), 7.2-7.5 (6H, m), 7.65 (1H, m), 7.97 (1H, m), 8.14 (1H, brs).

### Example 106

### Buthyl (2E)-3-(3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}phenyl)acrylate

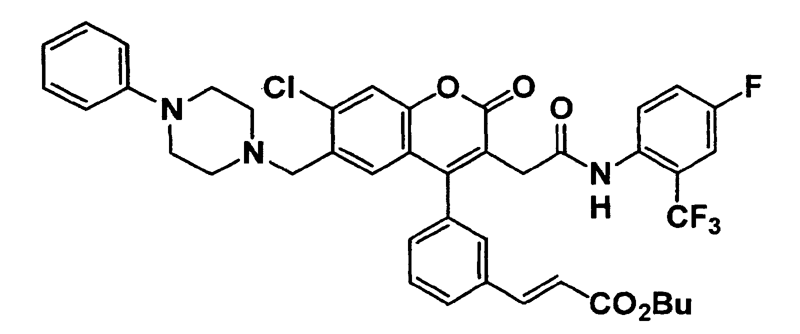

Palladium acetate (1.2 g), triphenylphosphine (2.9 g), triethylamine (3.8 ml) and butyl acrylate (2.9 ml) were added to a solution of 2-{4-(3-bromophenyl)-7-chloro-2-oxo-6-[(4-phenylpiperazin-1-yl)methyl]-2H-chromen-3-yl}-N-[4-fluoro-2-(trifluoromethyl)phenyl]acetamide (13.3 g) in DMF (140 ml), and the mixture was stirred at 100°C for 8 hours. Water was added to the reaction solution, and extracted with ethyl acetate. The extract was dried over magnesium sulfate, and the solvent was distilled off under reduced pressure. The resulting residue was purified by silica gel column chromatography (developing solvent: hexane-ethyl acetate = 3:1) to obtain the title compound (5.7g, yield 40%).
NMR (CDCl₃) δ: 0.94 (2H, t, J = 7.2 Hz) , 1.41 (2H, m), 1.66 (2H, m), 2.56 (4H, m), 3.01 (4H, m), 3.49 (2H, m), 3.58 (2H, m), 4.49 (2H, t, J = 7.2 Hz), 6.48 (1H, d, J = 15.9 Hz), 6.86 (3H, m), 7.2-7.7 (11H, m), 7.97 (1H, m), 8.18 (1H, brs).

### Example 107

### Buthyl (2E)-3-(3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}phenyl)acrylate

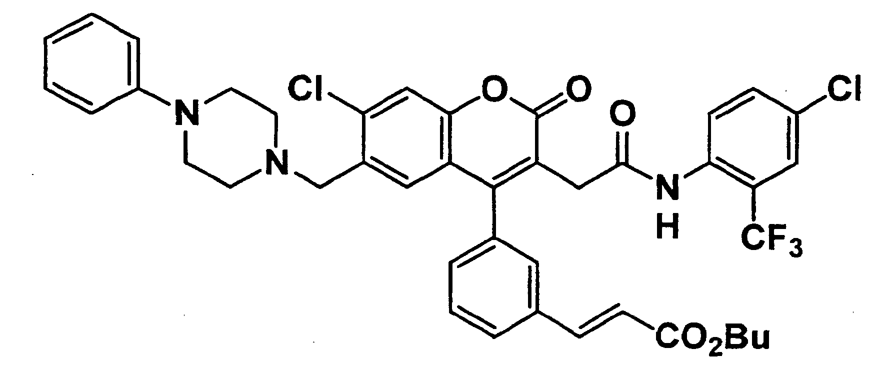

According to the same manner as that of Example 106, the title compound (yield 37%) was obtained.
NMR (CDCl₃) δ: 0.95 (2H, t, J = 6.8 Hz), 1.43 (2H, m), 1.67 (2H, m), 2.55 (4H, m), 3.01 (4H, m), 3.49 (2H, m), 3.59 (2H, m), 4.19 (2H, t, J = 6.6 Hz), 6.50 (1H, d, J = 16.0 Hz), 7.00 (3H, m), 7.2-7.7 (11H, m), 8.09 (1H, d, J = 8.8 Hz), 8.27 (1H, brs).

### Example 108

### Examples 108 to 109

According to the same manner as that of Example 45, compounds shown in [Table 11] were obtained.

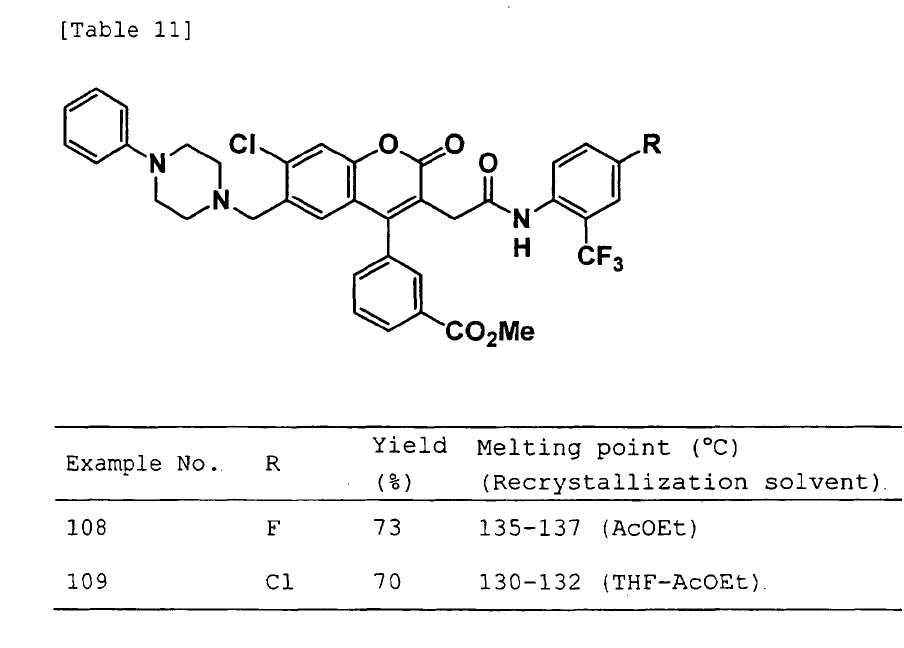

### Examples 110 to 113

According to the same manner as that of Example 14, compounds shown in [Table 12] were obtained.

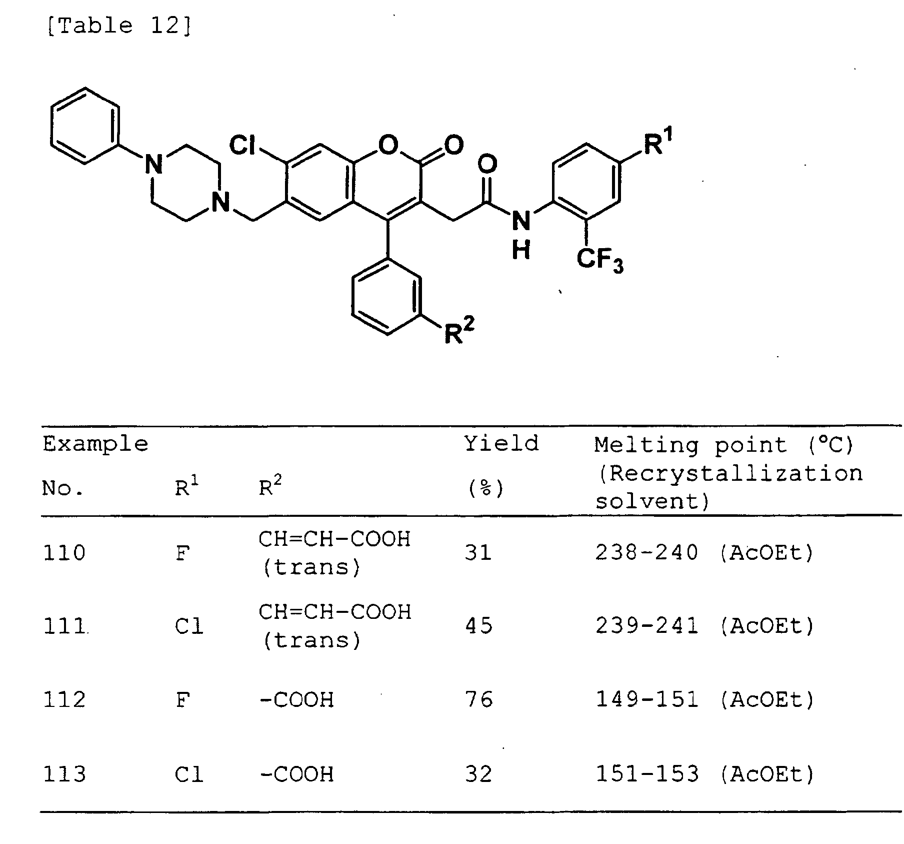

### Examples 114 to 115

According to the same manner as that of Example 13, compounds shown in [Table 13] were obtained.

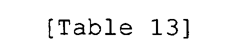

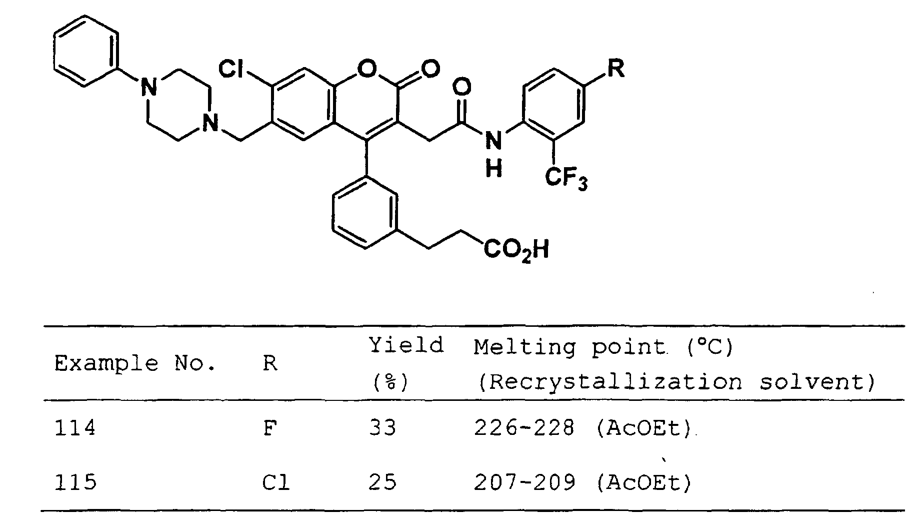

### Reference Example 15

### [4-(3-Bromophenyl)-7-chloro-3-(2-{[4-fluoro-2-(trifluoromethyl)phenyl]amino}-2-oxoethyl)-2-oxo-2H-chromen-6-yl]methyl acetate

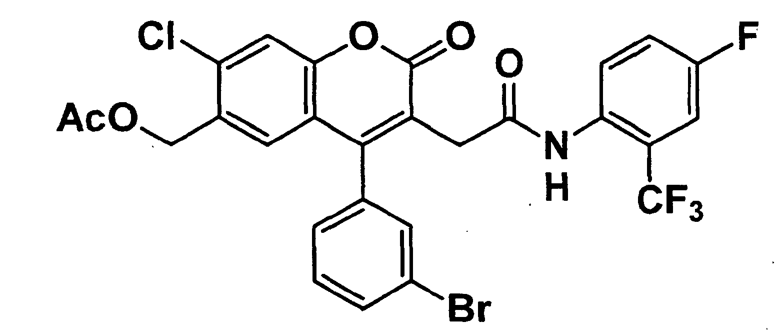

Oxalyl chloride (1.8 ml) was added to a mixed solution of [6-bromomethyl-4-(3-bromophenyl)-7-chloro-2-oxo-2H-chromen-3-yl]acetic acid (5.0 g) in THF (100 ml) and DMF (3 drops), and the mixture was stirred at room temperature for 30 minutes. The reaction solution was concentrated under reduced pressure, and the resulting residue was dissolved in THF (100 ml). 2-Amino-5-fluorobenzotrifluoride (2.2 ml) was added, and the mixture was stirred overnight. Water was added to the reaction solution, and extracted with ethyl acetate. The extract was washed with water, dried over magnesium sulfate, and the solvent was distilled off under reduced pressure. The resulting residue was dissolved in DMF (50 ml), sodium acetate (1.0 g) was added, and the mixture was stirred at 60°C for 1 hour. Water was added to the reaction solution, and extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent:ethyl acetate-hexane = 1:4), and then recrystallized from ethyl acetate to obtain the title compound (2.3 g).
NMR (CDCl₃) δ: 2.04 (3H, s), 3.42 (1H, d, J = 21 Hz), 3.54 (1H, d, J = 21 Hz), 5.08 (1H, d, J = 20 Hz), 5.16 (1H, d, J = 20 Hz), 7.07 (1H, s), 7.1-7.8 (7H, m), 7.9-8.1 (2H, m).

### Example 116

### Ethyl (2E)-3-{3-[6-[(acetyloxy)methyl]-7-chloro-3-(2-{[4-fluoro-2-(trifluoromethyl)phenyl]amino}-2-oxoethyl)-2-oxo-2H-chromen-4-yl]phenyl}acrylate

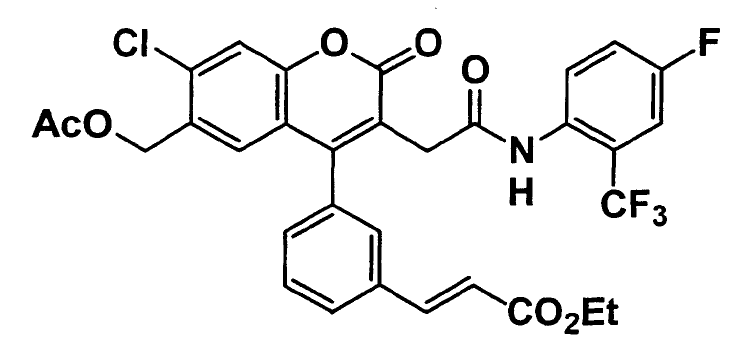

According to the same manner as that of Reference Example 1-(d), the title compound was obtained.
NMR (CDCl₃) δ: 1.33 (3H, t, J = 7 Hz), 1.98 (3H, s), 3.4-3.5 (2H, m), 4.26 (2H, q, J = 7 Hz), 5.09 (2H, s), 6.50 (1H, d, J = 16 Hz), 7.06 (1H, s), 7.2-7.7 (8H, m), 7.9-8.0 (1H, m), 8.11 (1H, brs).

### Example 117

### (2E)-3-{3-[7-chloro-3-(2-{[4-fluoro-2-(trifluoromethyl)phenyl]amino}-2-oxoethyl)-6-(hydroxymethyl)-2-oxo-2H-chromen-4-yl]phenyl}acrylic acid

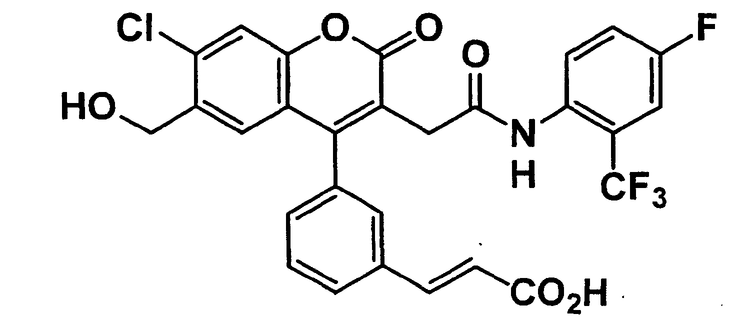

Ethyl (2E)-3-{3-[6-[(acetyloxy)methyl]-7-chloro-3-(2-{[4-fluoro-2-(trifluoromethyl)phenyl]amino}-2-oxoethyl)-2-oxo-2H-chromen-4-yl]phenyl}acrylate (600 mg) was suspended in methanol (18 ml) and DBU (0.21 ml) was added thereto under ice-cooling. The mixture was stirred at room temperature for 1 hour. 1N Hydrochloric acid was added to the reaction solution, produced precipitates were collected, and dissolved in a mixed solvent of ethyl acetate. This solution was washed with an aqueous saturated sodium chloride solution, dried over magnesium sulfate, and the solvent was distilled off under reduced pressure. The resulting residue was dissolved in THF (7.5 ml), methanol and 1N aqueous sodium hydroxide solution (2.5 ml) were added, and the mixture was stirred overnight. 1N Hydrochloric acid was added to the reaction solution, and extracted with ethyl acetate. The extract was washed with an aqueous saturated sodium chloride solution, dried over magnesium sulfate, and the solvent was distilled off under reduced pressure. The resulting residue was purified by silica gel column chromatography (developing solvent: dichloromethane-methanol = 95:5), and treated with THF-diisopropyl ether to obtain the title compound (85 mg) as white powders.
NMR (CDCl₃) δ: 3.44 (1H, d, J = 11 Hz), 3.50 (1H, d, J = 11 Hz), 4.65 (2H, s), 6.48 (1H, d, J = 7 Hz), 7.2-7.7 (9H, m), 7.8-8.0 (1H, m), 8.42 (1H, brs).

### Example 118

### (2E)-3-{3-[7-chloro-3-(2-{[4-fluoro-2-(trifluoromethyl)phenyl]amino}-2-oxoethyl)-6-methyl-2-oxo-2H-chromen-4-yl]phenyl)-N- (methylsulfonyl) acrylamide

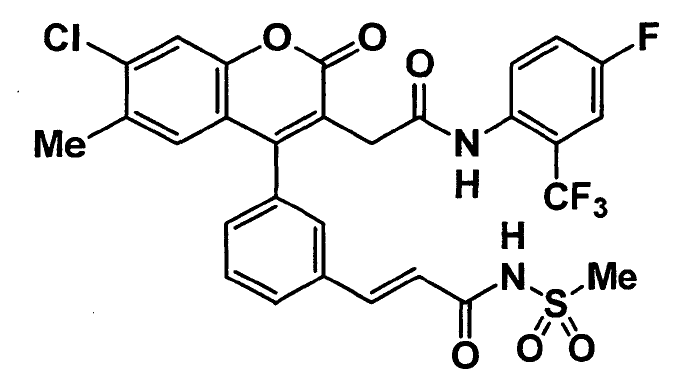

(2E)-3-{3-[7-Chloro-3-(2-{[4-fluoro-2-(trifluoromethyl)phenyl]amino}-2-oxoethyl)-6-methyl-2-oxo-2H-chromen-4-yl]phenyl}acrylic acid (200 mg) was suspended in DMF (2 ml) and carbonyldiimidazole (116 mg) was added thereto. The mixture was stirred at room temperature for 1 hour. Methanesulfonamide (68 mg) and DBU (82 mg) were added to the reaction solution, and the mixture was stirred at 100°C for 3 hours. 1N Hydrochloric acid was added, produced precipitates were collected, washed with water, and dissolved in a mixed solvent of THF and ethyl acetate. This solution was washed with an aqueous saturated sodium chloride solution, dried over magnesium sulfate, and the solvent was distilled off under reduced pressure. The resulting residue was purified by silica gel column chromatography (developing solvent:ethyl acetate-hexane = 2:1), and recrystallized from THF-hexane to obtain the title compound (86 mg, yield 38%) as colorless crystals.
NMR (CDCl₃) δ: 2.30 (3H, s), 3.30 (3H, s), 3.33 (1H, d, J = 14 Hz), 3.58 (1H, d, J = 14 Hz), 6.56 (1H, d, J = 16 Hz), 6.91 (1H, s), 7.2-7.4 (3H, m), 7.42 (1H, s), 7.5-7.8 (5H, m), 7.80 (1H, d, J = 16 Hz), 8.44 (1H, brs).

### Example 119

### (2E)-N-(butylsulfonyl)-3-{3-[7-chloro-3-(2-{[4-fluoro-2-(trifluoromethyl)phenyl]amino}-2-oxoethyl)-6-methyl-2-oxo-2H-chromen-4-yl]phenyl}acrylamide

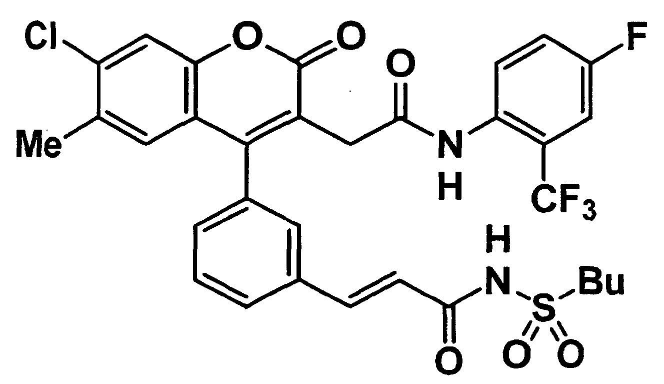

According to the same manner as that of Example 118, the title compound (yield 44%) was obtained.
NMR (CDCl₃) δ: 0.94 (3H, t, J = 7 Hz), 1.3-1.6 (2H, m), 1.7-1.9 (2H, m), 2.30 (3H, s), 3.37 (1H, d, J = 14 Hz), 3.4-3.5 (2H, m), 3.53 (1H, d, J = 14 Hz), 6.56 (1H, d, J = 16 Hz), 6.89 (1H, s), 7.2-7.9 (9H, m), 8.34 (1H, brs), 8.58 (1H, brs).

### Example 120

### 3-[7-Chloro-3-(2-{[4-chloro-2-(trifluoromethyl)phenyl]amino}-2-oxoethyl)-6-methyl-2-oxo-2H-chromen-4-yl]-N-ethylbenzamide

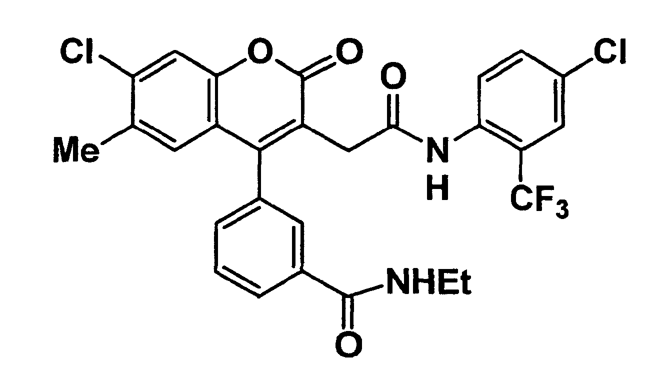

Oxalyl chloride (35 µl) was added to a mixed solution of 3-[7-chloro-3-(2-{[9-chloro-2-(trifluoromethyl)phenyl]amino}-2-oxoethyl)-6-methyl-2-oxo-2H-chromen-4-yl]benzoic acid (150 mg) in THF (5 ml) and DMF (1 drop), the mixture was stirred at room temperature for 30 minutes, and the solvent was then distilled off under reduced pressure. The resulting residue was dissolved in THF (10 ml), and the solution was added dropwise to a mixed solution of a 70% aqueous ethylamine solution (1 ml) and THF (5 ml). The mixture was stirred at room temperature for 30 minutes. 1N Hydrochloric acid was added to the reaction solution and the mixture was extracted with ethyl acetate. The extract was washed with an aqueous saturated sodium hydrogencarbonate solution and water, dried over magnesium sulfate, and the solvent was distilled off under reduced pressure. The resulting crude crystals were recrystallized from ethyl acetate to obtain the title compound (136 mg: yield 86%) as colorless crystals. mp: 220-222°C.

### Examples 121 to 148

According to the same manner as that of Example 120, compounds shown in [Table 14] were obtained.

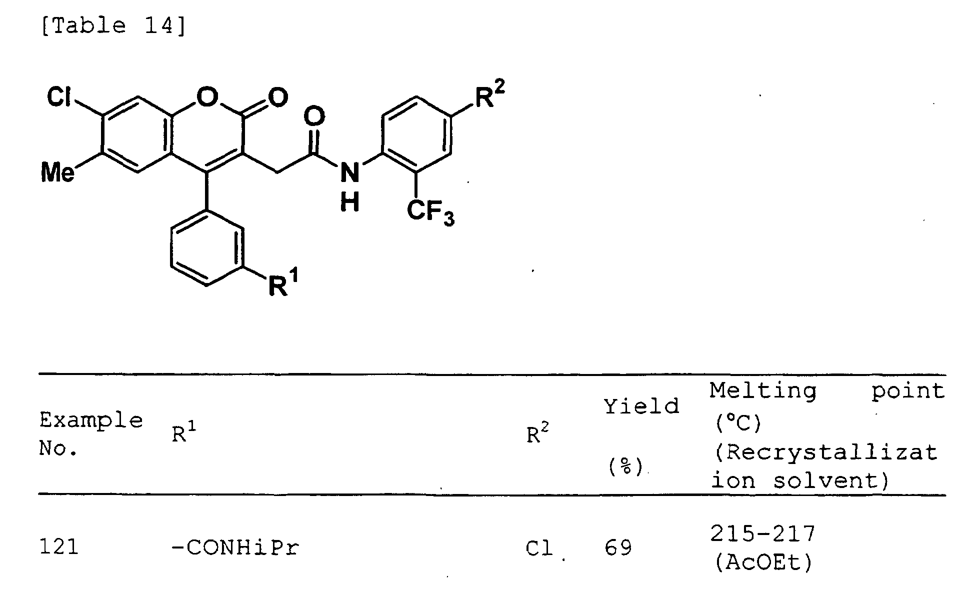

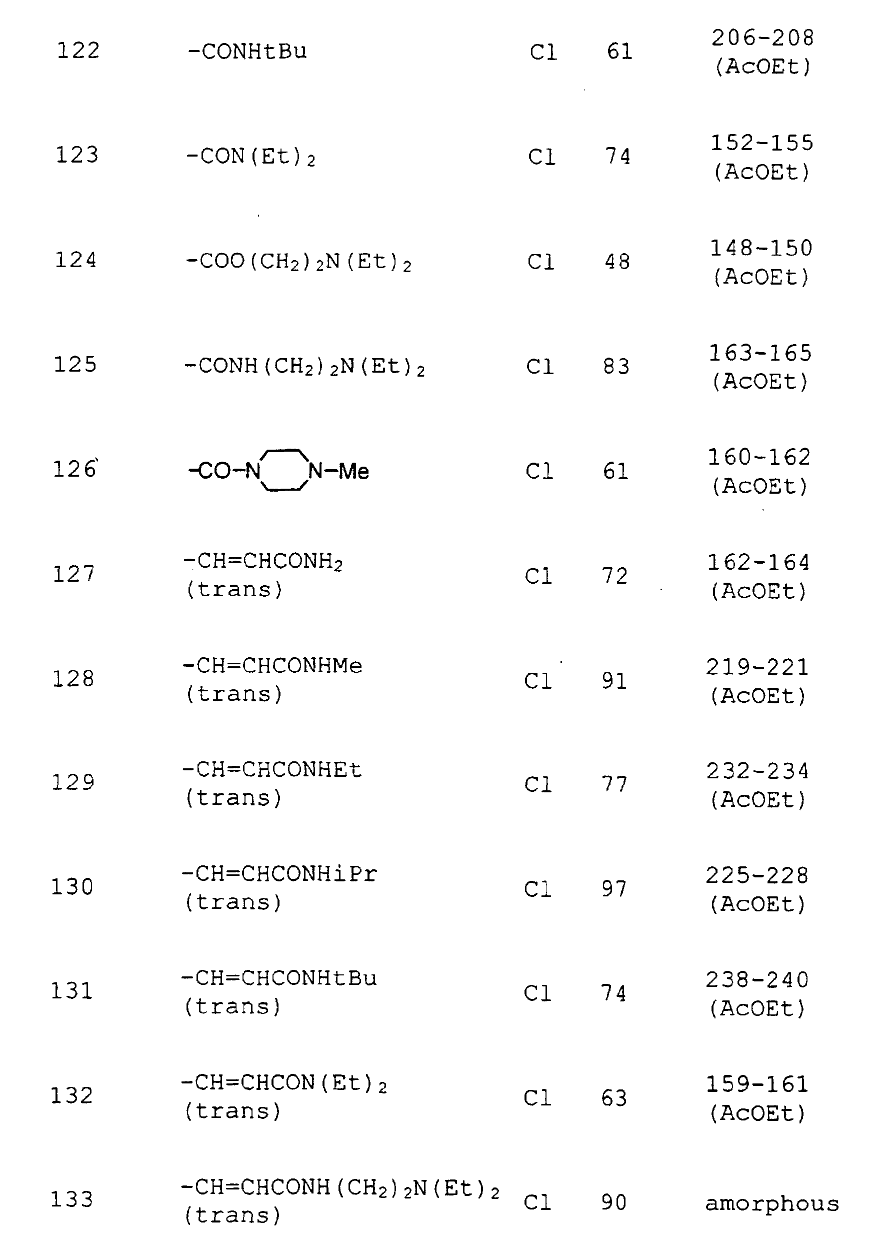

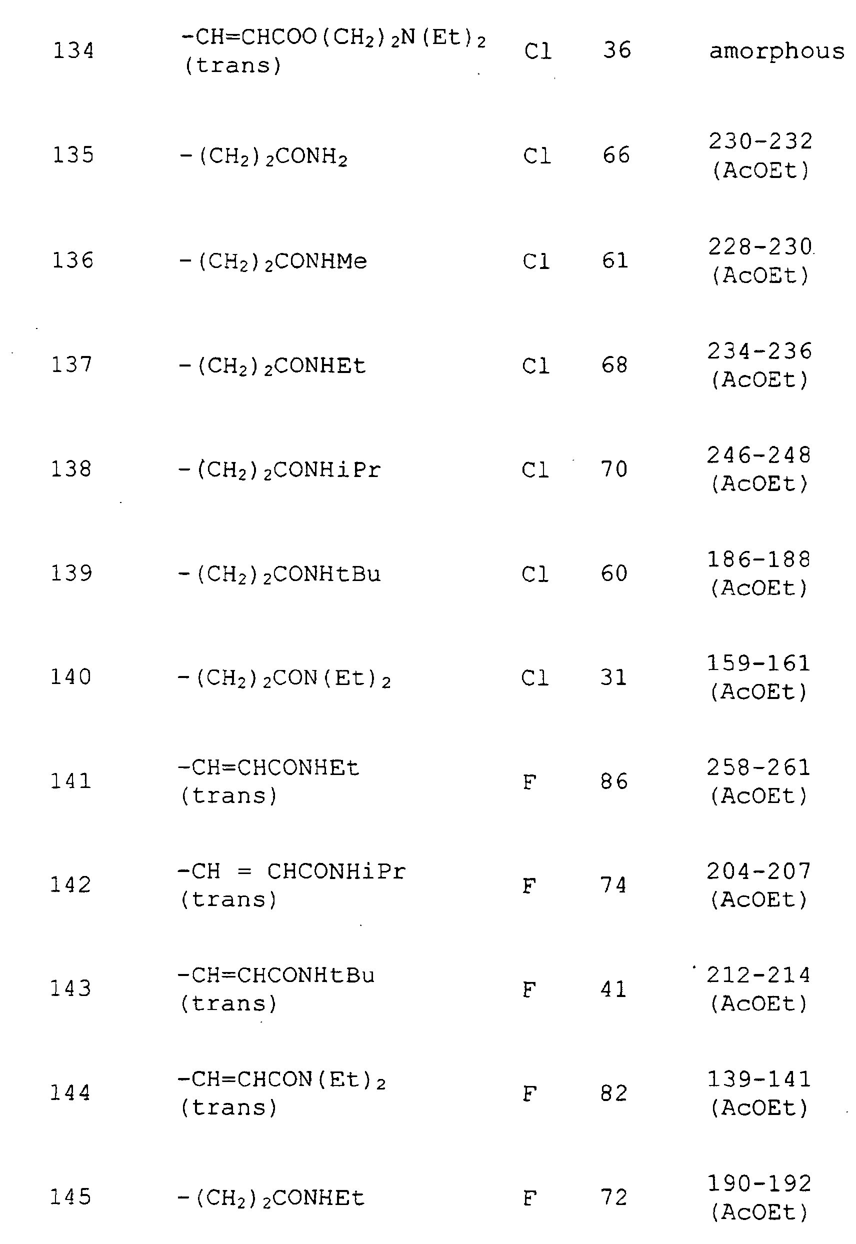

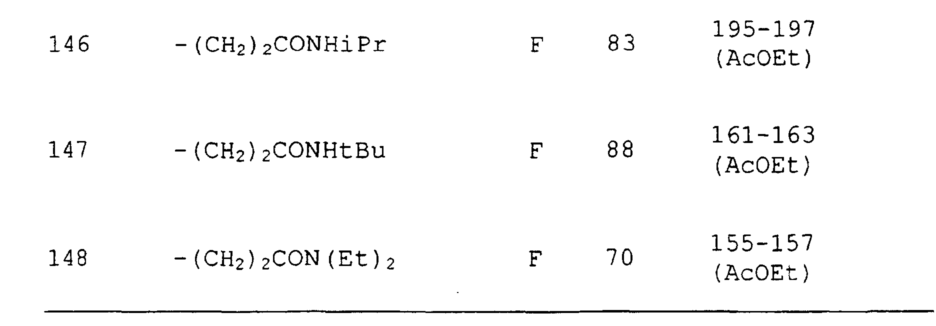

In the following Formulation Examples and Experimental Examples, Compounds A to E mean the following compounds.
Compound A: 3-[3-[7-chloro-3-(2-[[4-fluoro-2-(trifluoromethyl)phenyl]amino]-2-oxoethyl)-6-methyl-2-oxo-2H-chromen-4-yl]phenyl]propionic acid
Compound B: (2E)-3-[3-[7-chloro-6-methyl-2-oxo-3-(2-oxo-2-[[2-(trifluoromethyl)phenyl]amino]ethyl)-2H-chromen-4-yl]phenyl]-2-propenoic acid
Compound C: 3-[3-[7-chloro-6-methyl-2-oxo-3-(2-oxo-2-[[2-(trifluoromethyl)phenyl]amino]ethyl)-2H-chromen-4-yl]phenyl]propionic acid
Compound D: (2E)-3-[3-[6-chloro-3-(2-[[4-fluoro-2-(trifluoromethyl)phenyl]amino]-2-oxoethyl)-7-methyl-2-oxo-2H-chromen-4-yl]phenyl]-propenoic acid
Compound E: 3-[3-[6-chloro-3-(2-[[4-fluoro-2-(trifluoromethyl)phenyl]amino]-2-oxoethyl)-7-methyl-2-oxo-2H-chromen-4-yl]phenyl]propionic acid

### Formulation Example

A lipid-rich plaque regressing agent or an ACAT inhibitor containing the compound [I] or a salt thereof of the present invention as an active ingredient can be produced, for example, by the following formulations.

In the following formulations, as ingredients (additives) other than an active ingredient, products listed in Japanese Pharmacopoeia, Japanese Pharmaceutical Codex or Japanese Pharmaceutical Excipients can be used.

### 1. Capsule

| | | |
|---|---|---|
| (1) | Compound A | 10 mg |
| (2) | Lactose | 90 mg |
| (3) | Microcrystalline Cellulose | 70 mg |
| (4) | Magnesium stearate | 10 mg |
| | One capsule | 180 mg |

(1), (2), (3) and 1/2 of (4) are mixed and then granulated. To this is added the remainder of (4) and the entire is encapsulated into a gelatin capsule.

### 2. Tablet

| | | |
|---|---|---|
| (1) | Compound A | 10 mg |
| (2) | Lactose | 35 mg |
| (3) | Cornstarch | 150 mg |
| (4) | Microcrystalline Cellulose | 30 mg |
| (5) | Magnesium stearate | 5 mg |
| | One capsule | 230 mg |

(1), (2), (3) , 2/3 of (4) and 1/2 of (5) are mixed and granulated. To this granule, the remainders of (4) and (5) are added and compressed into a tablet.

### 3. Injection formulation

| | | |
|---|---|---|
| (1) | Compound A | 10 mg |
| (2) | Inositol | 100 mg |
| (3) | Benzyl alcohol | 20 mg |
| | One ampoule | 130 mg |

(1), (2) and (3) are dissolved in distilled water for injection to make the total volume 2 ml, and charged in an ampoule. All steps are performed aseptically.

### 4. Tablet

According to the following composition, a mixture of Compound A (175 g), D-mannitol (175 g), cornstarch (118.65 g) and sodium croscarmellose (105 g) is mixed sufficiently with a vertical granulator (FM-VG-10 type manufactured by POWREX CORPORATION) and then kneaded with a solution of hydroxypropyl cellulose (19.25 g) in water (kneading condition: 400rpm, 10 minutes). A white kneaded material is dried in a fluidized drier (FD-3S manufactured by POWREX CORPORATION) at a ventilating temperature of 60°C for 30 minutes, and then sieved through a 1.5 mmφ punching screen using a power mill (P-3 type manufactured by Showa kagaku kikai kousakusho) to obtain a granule. This granule (525.14 g), sodium croscarmellose (31 g) and magnesium stearate (1.86 g) are added and mixed for 5 minutes with a mixing machine (TM-15 type manufactured by Showa kagaku kikai kousakusho) to obtain a granule for tableting. This granule is compressed in 180 mg aliquots with a tableting machine (Correct 19K manufactured by Kikusui Seisakusho Ltd.) using a 8.0 mmφ edged plain mallet under a pressure of 0.7 ton/cm² to obtain 2,350 tablets.

| | |
|---|---|
| Compound A | 50 mg |
| D-mannitol | 50 mg |
| Cornstarch | 33.9 mg |
| Sodium croscarmellose | 40 mg |
| Hydroxypropyl cellulose | 5.5 mg |
| Magnesium stearate | 0.6 mg |
| Total | 180.0 mg (per tablet) |

### 1. Capsule

| | | |
|---|---|---|
| (1) | Compound B | 10 mg |
| (2) | Lactose | 90 mg |
| (3) | Microcrystalline cellulose | 70 mg |
| (4) | Magnesium stearate | 10 mg |
| | One capsule | 180 mg |

(1), (2), (3) and 1/2 of (4) are mixed and then granulated. To this is added the remainder of (4) and the entire is encapsulated into a gelatin capsule.

### 2. Tablet

| | | |
|---|---|---|
| (1) | Compound B | 10 mg |
| (2) | Lactose | 35 mg |
| (3) | Cornstarch | 150 mg |
| (4) | Microcrystalline cellulose | 30 mg |
| (5) | Magnesium stearate | 5 mg |
| | One tablet | 230 mg |

(1), (2), (3), 2/3 of (4) and 1/2 of (5) are mixed and then granulated. To this granule, the Remainders of (4) and (5) are added and compressed into a tablet.

### 3. Injection formulation

| | | |
|---|---|---|
| (1) | Compound B | 10 mg |
| (2) | Inositol | 100 mg |
| (3) | Benzyl alcohol | 20 mg |
| | One ampoule | 130 mg |

(1), (2) and (3) are dissolved in distilled water for injection to make the total volume 2 ml, and charged in an ampoule. All steps are performed aseptically.

### 4. Tablet

According to the following composition, a mixture of Compound B (175 g), D-mannitol (175 g), cornstarch (118.65 g) and sodium croscarmellose (105 g) is mixed sufficiently with a vertical granulator (FM-VG-10 type manufactured by POWREX CORPORATION) and then kneaded with a solution of hydroxypropyl cellulose (19.25 g) in water (kneading condition: 400rpm, 10 minutes). A white kneaded material is dried in a fluidized drier (FD-3S manufactured by POWREX CORPORATION) at a ventilating temperature of 60°C for 30 minutes, and then sieved through a 1.5 mmφ punching screen using a power mill (P-3 type manufactured by Showa kagaku kikai kousakusho) to obtain a granule. This granule (525.14 g), sodium croscarmellose (31 g) and magnesium stearate (1.86 g) are added and mixed for 5 minutes with a mixing machine (TM-15 type manufactured by Showa kagaku kikai kousakusho) to obtain a granule for tableting. This granule is compressed in 180 mg aliquots with a tableting machine (Correct 19K manufactured by Kikusui Seisakusho Ltd.) using a 8.0 mmφ edged plain mallet under a pressure of 0.7 ton/cm² to obtain 2,350 tablets.

| | |
|---|---|
| Compound B | 50 mg |
| D-mannitol | 50 mg |
| Cornstarch | 33.9 mg |
| Sodium croscarmellose | 40 mg |
| Hydroxypropyl cellulose | 5.5 mg |
| Magnesium stearate | 0.6 mg |
| Total | 180.0 mg (per tablet) |

### 1. Capsule

| | | |
|---|---|---|
| (1) | Compound C | 10 mg |
| (2) | Lactose | 90 mg |
| (3) | Microcrystalline cellulose | 70 mg |
| (4) | Magnesium stearate | 10 mg |
| | One capsule | 180 mg |

(1), (2), (3) and 1/2 of (4) are mixed and then granulated. To this is added the remainder of (4) and the entire is encapsulated into a gelatin capsule.

### 2. Tablet

| | | |
|---|---|---|
| (1) | Compound C | 10 mg |
| (2) | Lactose | 35 mg |
| (3) | Cornstarch | 150 mg |
| (4) | Microcrystalline cellulose | 30 mg |
| (5) | Magnesium stearate | 5 mg |
| | One tablet | 230 mg |

(1), (2), (3), 2/3 of (4) and 1/2 of (5) are mixed and then granulated. To this granule, the Remainders of (4) and (5) are added and compressed into a tablet.

### 3. Injection formulation

| | | |
|---|---|---|
| (1) | Compound C | 10 mg |
| (2) | Inositol | 100 mg |
| (3) | Benzyl alcohol | 20 mg |
| | One ampoule | 130 mg |

(1), (2) and (3) are dissolved in distilled water for injection to make the total volume 2 ml, and charged in an ampoule. All steps are performed aseptically.

### 4. Tablet

According to the following composition, a mixture of Compound C (175 g), D-mannitol (175 g), cornstarch (118.65 g) and sodium croscarmellose (105 g) is mixed sufficiently with a vertical granulator (FM-VG-10 type manufactured by POWREX CORPORATION) and then kneaded with a solution of hydroxypropyl cellulose (19.25 g) in water (kneading condition: 400rpm, 10 minutes). A white kneaded material is dried in a fluidized drier (FD-3S manufactured by POWREX CORPORATION) at a ventilating temperature of 60°C for 30 minutes, and then sieved through a 1.5 mmφ punching screen using a power mill (P-3 type manufactured by Showa kagaku kikai kousakusho) to obtain a granule. This granule (525.14 g), sodium croscarmellose (31 g) and magnesium stearate (1.86 g) are added and mixed for 5 minutes with a mixing machine (TM-15 type manufactured by Showa kagaku kikai kousakusho) to obtain a granule for tableting. This granule is compressed in 180 mg aliquots with a tableting machine (Correct 19K manufactured by Kikusui Seisakusho Ltd.) using a 8.0 mmφ edged plain mallet under a pressure of 0.7 ton/cm² to obtain 2,350 tablets.

| | |
|---|---|
| Compound C | 50 mg |
| D-mannitol | 50 mg |
| Cornstarch | 33.9 mg |
| Sodium croscarmellose | 40 mg |
| Hydroxypropyl cellulose | 5.5 mg |
| Magnesium stearate | 0.6 mg |
| Total | 180.0 mg (per tablet) |

### 1. Capsule

| | | |
|---|---|---|
| (1) | Compound D | 10 mg |
| (2) | Lactose | 90 mg |
| (3) | Microcrystalline cellulose | 70 mg |
| (4) | Magnesium stearate | 10 mg |
| | One capsule | 180 mg |

(1), (2), (3) and 1/2 of (4) are mixed and then granulated. To this is added the remainder of (4) and the entire is encapsulated into a gelatin capsule.

### 2. Tablet

| | | |
|---|---|---|
| (1) | Compound D | 10 mg |
| (2) | Lactose | 35 mg |
| (3) | Cornstarch | 150 mg |
| (4) | Microcrystalline cellulose | 30 mg |
| (5) | Magnesium stearate | 5 mg |
| | One tablet | 230 mg |

(1), (2), (3), 2/3 of (4) and 1/2 of (5) are mixed and then granulated. To this granule, the Remainders of (4) and (5) are added and compressed into a tablet.

### 3. Injection formulation

| | | |
|---|---|---|
| (1) | Compound D | 10 mg |
| (2) | Inositol | 100 mg |
| (3) | Benzyl alcohol | 20 mg |
| | One ampoule | 130 mg |

(1), (2) and (3) are dissolved in distilled water for injection to make the total volume 2 ml, and charged in an ampoule. All steps are performed aseptically.

### 4. Tablet

According to the following composition, a mixture of Compound D (175 g), D-mannitol (175 g), cornstarch (118.65 g) and sodium croscarmellose (105 g) is mixed sufficiently with a vertical granulator (FM-VG-10 type manufactured by POWREX CORPORATION) and then kneaded with a solution of hydroxypropyl cellulose (19.25 g) in water (kneading condition: 400rpm, 10 minutes). A white kneaded material is dried in a fluidized drier (FD-3S manufactured by POWREX CORPORATION) at a ventilating temperature of 60°C for 30 minutes, and then sieved through a 1.5 mmφ punching screen using a power mill (P-3 type manufactured by Showa kagaku kikai kousakusho) to obtain a granule. This granule (525.14 g), sodium croscarmellose (31 g) and magnesium stearate (1.86 g) are added and mixed for 5 minutes with a mixing machine (TM-15 type manufactured by Showa kagaku kikai kousakusho) to obtain a granule for tableting. This granule is compressed in 180 mg aliquots with a tableting machine (Correct 19K manufactured by Kikusui Seisakusho Ltd.) using a 8.0 mmφ edged plain mallet under a pressure of 0.7 ton/cm² to obtain 2,350 tablets.

| | |
|---|---|
| Compound D | 50 mg |
| D-mannitol | 50 mg |
| Cornstarch | 33.9 mg |
| Sodium croscarmellose | 40 mg |
| Hydroxypropyl cellulose | 5.5 mg |
| Magnesium stearate | 0.6 mg |
| Total | 180.0 mg (per tablet) |

### 1. Capsule

| | | |
|---|---|---|
| (1) | Compound E | 10 mg |
| (2) | Lactose | 90 mg |
| (3) | Microcrystalline cellulose | 70 mg |
| (4) | Magnesium stearate | 10 mg |
| | One capsule | 180 mg |

(1), (2), (3) and 1/2 of (4) are mixed and then granulated. To this is added the remainder of (4) and the entire is encapsulated into a gelatin capsule.

### 2. Tablet

| | | |
|---|---|---|
| (1) | Compound E | 10 mg |
| (2) | Lactose | 35 mg |
| (3) | Cornstarch | 150 mg |
| (4) | Microcrystalline cellulose | 30 mg |
| (5) | Magnesium stearate | 5 mg |
| | One tablet | 230 mg |

(1), (2), (3), 2/3 of (4) and 1/2 of (5) are mixed and then granulated. To this granule, the Remainders of (4) and (5) are added and compressed into a tablet.

### 3. Injection formulation

| | | |
|---|---|---|
| (1) | Compound E | 10 mg |
| (2) | Inositol | 100 mg |
| (3) | Benzyl alcohol | 20 mg |
| | One ampoule | 130 mg |

(1), (2) and (3) are dissolved in distilled water for injection to make the total volume 2 ml, and charged in an ampoule. All steps are performed aseptically.

### 4. Tablet

According to the following composition, a mixture of Compound E (175 g), D-mannitol (175 g), cornstarch (118.65 g) and sodium croscarmellose (105 g) is mixed sufficiently with a vertical granulator (FM-VG-10 type manufactured by POWREX CORPORATION) and then kneaded with a solution of hydroxypropyl cellulose (19.25 g) in water (kneading condition: 400rpm, 10 minutes). A white kneaded material is dried in a fluidized drier (FD-3S manufactured by POWREX CORPORATION) at a ventilating temperature of 60°C for 30 minutes, and then sieved through a 1.5 mmφ punching screen using a power mill (P-3 type manufactured by Showa kagaku kikai kousakusho) to obtain a granule. This granule (525.14 g), sodium croscarmellose (31 g) and magnesium stearate (1.86 g) are added and mixed for 5 minutes with a mixing machine (TM-15 type manufactured by Showa kagaku kikai kousakusho) to obtain a granule for tableting. This granule is compressed in 180 mg aliquots with a tableting machine (Correct 19K manufactured by Kikusui Seisakusho Ltd.) using a 8.0 mmφ edged plain mallet under a pressure of 0.7 ton/cm² to obtain 2,350 tablets.

| | |
|---|---|
| Compound E | 50 mg |
| D-mannitol | 50 mg |
| Cornstarch | 33.9 mg |
| Sodium croscarmellose | 40 mg |
| Hydroxypropyl cellulose | 5.5 mg |
| Magnesium stearate | 0.6 mg |
| Total | 180.0 mg (per tablet) |

### Experimental Example

The following Experimental Example will illustrate ACAT inhibitory activity of the compound [I] of the present invention or a salt thereof.

### Experimental Example 1 (ACAT inhibitory activity)

### [Preparation of mouse abdominal macrophage microsome ACAT]

According to the method of Hakamada et al. (Experimental Medicine Suppliment vol.14, No.12, Circulation Research Protocol, p, 49-52, 1996), an abdominal macrophage was taken from a thioglycolate-stimulated C57BL6J mouse and cultured for 24 hours in a RPMI 1640-25mM HEPES (pH7.0) medium containing rabbit β-very low density lipoprotein (β-VLDL, 150 µg cholesterol/ml) which had been prepared by the method of Ishii et al. (Ishii I et al., Arterioscler, Thromb., 12, 1139-1145, 1992). The abdominal macrophage was then collected by centrifugation (4°C, 1.000rpm, 5 minutes) and sonicated. The sonicated liquid was centrifuged (4°C, 5000rpm, 15 minutes) and then ultracentrifuged (4°C, 50, 000rpm, 90 minutes) to prepare a microsome. The microsome thus obtained was used for measuring ACAT inhibitory activity of a test compound as mouse abdominal macrophage microsome ACAT.

### [Method for measuring ACAT inhibitory activity]

A mixture of a test compound, cholesterol-albumin-containing-Tris-HCL buffer (pH 7.5) and mouse abdominal macrophage microsome ACAT was pre-incubated at 37°C for 10 minutes, and ³H-oleyl-CoA was added to react at 37°C for 20 minutes. A stopping solution composed of chloroform-methyl alcohol-distilled water (2:2:1 v/v) was added, and produced cholesteryl ester (CE) was extracted with shaking. The extract was subjected to silica gel thin chromatography (petroleum ether:diethyl ether:acetic acid = 9:1:0.1 v/v), and the resulting ³H-CE fraction was measured with a scintillation counter.

ACAT inhibiting rate was calculated from the proportion based on ACAT activity without a test compound, and an IC₅₀ value was calculated as the concentration (µM) of a test compound showing ACAT inhibiting rate 50%. The results are shown in [Table 15].

**[Table 15]**

| Compound No. (Example No.) | Enzyme inhibition (IC₅₀, µM) |
|---|---|
| 78 | 0.43 |
| 110 | 0.54 |
| 111 | 0.61 |
| 112 | 0.54 |
| 113 | 1.22 |
| 114 | 0.55 |
| 115 | 0.42 |

As apparent from the above results, the compound of the present invention has excellent ACAT inhibitory activity, and useful as a novel arteriosclerosis treating agent that results in inhibiting formation of and regressing an arteriosclerotic lesion. In addition, since it is believed that the subtype of macrophage-derived ACAT and that of liver-derived ACAT are the same in a human, the compound of the present invention may be also useful as a hyperlipemia treating agent.

### Industrial applicability

Since the compound [I] of the present invention, a salt thereof and a prodrug thereof have excellent lipid-rich plaque regressing activity or/and ACAT inhibitory activity, they are useful for preventing or treating acute myocardial infarction, acute coronary syndrome such as unstable angina, peripheral artery occlusion, hyperlipemia, cerebral infarction, cerebral apoplexy, arteriosclerosis, Alzheimer's disease, multiple risk syndrome and metabolism syndrome, etc. in a mammal (e.g. mouse, rat, rabbit, dog, cat, cow, pig, monkey, human etc.) or preventing or treating restenosis after PTCA or after stent placement.

## Claims

1. A compound represented by the formula [I]: 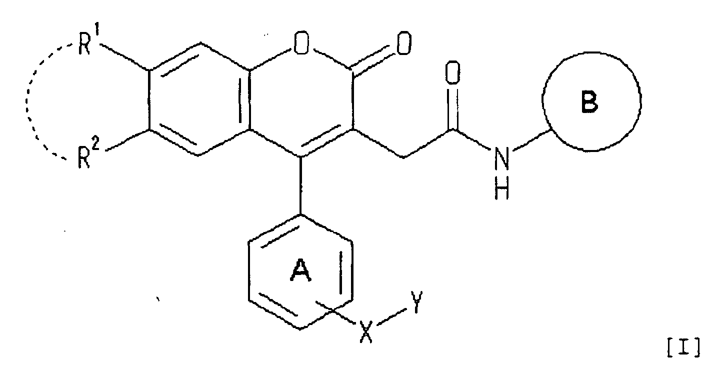 wherein R¹ and R² are each a hydrogen atom, a halogen atom, an optionally substituted linear hydrocarbon group, or a hydroxyl group which may be substituted with an optionally substituted linear hydrocarbon group, or R¹ and R² may be taken together with the adjacent carbon atoms to form an optionally substituted cyclic hydrocarbon, or a dihydrofuran ring which may be substituted with an oxo group; ring A is an optionally further substituted benzene ring; B is an optionally substituted aromatic ring; X is a bond or a spacer whose main chain consists of 1 to 6 atoms; Y is an optionally esterified carboxyl group, an optionally substituted carbamoyl group, a cyano group, or an optionally substituted heterocyclic group bearing a hydrogen atom capable of being deprotonated; provided that 3-[3-[7-chloro-3-(2-[[4-chloro-2-(trifluoromethyl)phenyl]amino]-2-oxoethyl)-6-methyl-2-oxo-2H-chromen-4-yl]phenyl]-2-propionic acid, ethyl 3-[3-[7-chloro-3-(2-[[4-chloro-2-(trifluoromethyl)phenyl]amino]-2-oxoethyl)-6-methyl-2-oxo-2H-chromen-4-yl]phenyl]-2-propionate, methyl (2E)-3-[3-[7-chloro-3-(2-[[4-chloro-2-(trifluoromethyl)phenyl]amino]-2-oxoethyl)-6-methyl-2-oxo-2H-chromen-4-yl]phenyl]-2-propenoate, (2E)-3-[3- [7-chloro-3-(2-[[4-chloro-2-(trifluoromethyl)phenyl]amino]-2-oxoethyl)-6-methyl-2-oxo-2H-chromen-4-yl]phenyl]-2-propenoic acid, ethtyl (2E)-3-[3-[7-chloro-3-(2-[[4-chloro-2-(trifluoromethyl)phenyl]amino]-2-oxoethyl)-6-methyl-2-oxo-2H-chromen-4-yl]phenyl]-2-propenoate, ethyl (2E)-3-[3-[7-chloro-3-(2-[[4-fluoro-2-(trifluoromethyl)phenyl]amino]-2-oxoethyl)-6-methyl-2-oxo-2H-chromen-4-yl]phenyl]-2-propenoate and (2E)-3-[3-[7-chloro-3-(2-[[4-fluoro-2-(trifluromethyl)phenyl]amino]-2-oxoethyl)-6-methyl-2-oxo-2H-chromen-4-yl]phenyl]-2-propenoic acid are excluded, or a salt thereof.

2. The compound according to claim 1, whererin the formula [I] is the formula [I']: 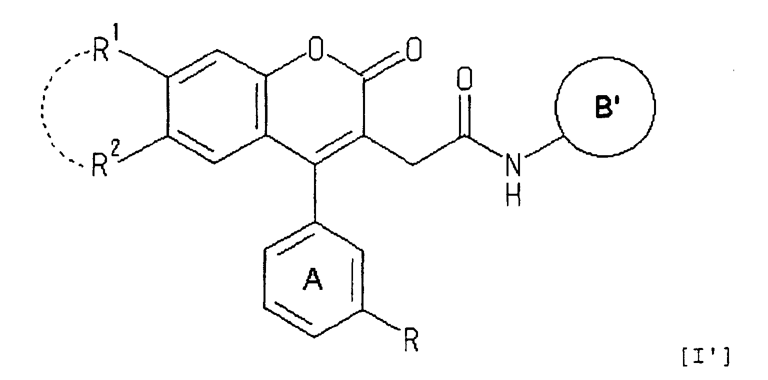 wherein ring B' is an optionally substituted benzene ring or an optionally substituted pyridine ring, R is an optionally esterified carboxyl group, or a linear hydrocarbon group which is substituted with an optionally esterified carboxyl group, and other symbols are as defined in claim 1.

3. The compound according to claim 1, wherein R¹ and R² are each a hydrogen atom, a halogen atom or an optionally substituted linear hydrocarbon group, or R¹ and R² may be taken together with the adjacent carbon atoms to form an optionally substituted cyclic hydrocarbon.

4. The compound according to claim 1, wherein R¹ and R² are each a halogen atom or an optionally substituted C₁₋₇ alkyl group.

5. The compound according to claim 1, wherein R¹ is a halogen atom and R² is a linear hydrocarbon group which is substituted with an optionally substituted amino group.

6. The compound according to claim 1, wherein R¹ is a halogen atom and R² is a linear hydrocarbon group which is substituted with an optionally substituted cyclic amino group.

7. The compound according to claim 1, wherein the cyclic hydrocarbon is C₅₋₇ cyclic hydrocarbon.

8. The compound according to claim 1, wherein ring B is a benzene ring which is substituted with a halogenated alkyl group and/or a halogen atom.

9. The compound according to claim 2, wherein R is a group represented by the formula -(CH₂)ₙ-R' wherein R' is an optionally esterified carboxyl group and n is an integer of 0 to 6.

10. The compound according to claim 2, wherein R is a group represented by the formula -CH=CH-(CH₂)_{n'}-R' wherein R' is an optionally esterified carboxyl group and n' is an integer of 0 to 4.

11. The compound according to claim 2, wherein R is a group represented by the formula -(CH=CH)_{n"}-R' wherein R' is an optionally esterified carboxyl group and n" is an integer of 1 to 3.

12. 3-[3-[7-chloro-3-(2-[[4-fluoro-2-(trifluoromethyl)phenyl]amino]-2-oxoethyl)-6-methyl-2-oxo-2H-chromen-4-yl]phenyl]propionic acid, (2E)-3-[3-[7-chloro-6-methyl-2-oxo-3-(2-oxo-2-[[2-(trifluoromethyl)phenyl]amino]ethyl)-2H-chromen-4-yl]phenyl]-2-propenoic acid, 3-[3-[7-chloro-6-methyl-2-oxo-3-(2-oxo-2-[[2-(trifluoromethyl)phenyl]amino]ethyl)-2H-chromen-4-yl]phenyl]propionic acid, (2E)-3-[3-[6-chloro-3-(2-[[4-fluoro-2-(trifluoromethyl)phenyl]amino]-2-oxoethyl)-7-methyl-2-oxo-2H-chromen-4-yl]phenyl]-2-propenoic acid, 3-[3-[6-chloro-3-(2-[[4-fluoro-2-(trifluoromethyl)phenyl]amino]-2-oxoethyl)-7-methyl-2-oxo-2H-chromen-4-yl]phenyl]propionic acid, (2E)-3-(3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}phenyl)acrylic acid, (2E)-3-(3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}phenyl)acrylic acid, 3-{7-chloro-3-(2-{[4-fluoro-2-(trifluoromethyl)phenyl]amino}-2-oxoethyl)-2-oxo-6-[(4-phenylpiperazin-1-yl)methyl]-2H-chromen-4-yl}benzoic acid, 3-{7-chloro-3-(2-{[4-chloro-2-(trifluoromethyl)phenyl]amino}-2-oxoethyl)-2-oxo-6-[(4-phenylpiperazin-1-yl)methyl]-2H-chromen-4-yl}benzoic acid or a salt thereof.

13. A prodrug of the compound according to claim 1 or a salt thereof.

14. A pharmaceutical composition comprising the compound according to claim 1 or 13 or a salt thereof.

15. The pharmaceutical composition according to claim 14, which is a lipid-rich regressing agent or an ACAT inhibitor.

16. The pharmaceutical composition according to claim 14, which is a prophylactic or therapeutic agent against acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or an agent for regressing, inhibiting progression of or stabilizing an arteriosclerotic lesion.

17. The agent for regressing, inhibiting progression of or stabilizing an arteriosclerotic lesion according to claim 16, which is combined with a HMG-CoA reductase inhibitor.

18. A method for regressing a lipid-rich plaque or inhibiting ACAT in a mammal, which comprises administering an effective amount of the compound according to claim 1 or a salt thereof to the mammal.

19. A method for preventing or treating acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or regressing, inhibiting progression of or stabilizing an arteriosclerotic lesion in a mammal, which comprises administering an effective amount of the compound according to claim 1 or a salt thereof to the mammal.

20. The method for regressing, inhibiting progression of or stabilizing an arteriosclerotic lesion according to claim 19, which comprises administering the compound according to claim 1 or a salt thereof in combination with a HMG-CoA reductase inhibitor.

21. Use of the compound according to claim 1 or a salt thereof for production of a lipid-rich plaque regressing agent or an ACAT inhibitor.

22. Use of the compound according to claim 1 or a salt thereof for production of a prophylactic or therapeutic agent against acute coronary syndrome, acute myocardial infarction, unstable angina, coronary artery restenosis after PTCA or stent placement, peripheral artery occlusion, hyperlipemia, cerebral infarction, cerebral apoplexy, Alzheimer's disease, multiple risk syndrome or metabolic syndrome, or an agent for regressing, inhibiting progression of or stabilizing an arteriosclerotic lesion.

23. The use of the compound according to claim 1 or a salt thereof for production of an agent for regressing, inhibiting progression of or stabilizing an arteriosclerotic lesion according to claim 22, which is combined with a HMG-CoA reductase inhibitor.
